# EUROPEAN PATENT APPLICATION

(11) **EP 2 845 902 A1**
(43) Date of publication of application: **11.03.2015**
(21) Application number: 13306233.1
(22) Date of filing: 09.09.2013
(51) Int. Cl.: C12N 15/82, C07K 14/435

(54) **Method for reducing the plant-parasitic nematode infestation level in a plant**

(71) Applicant: Genoplante-Valor, 75015 Paris (FR)
(72) Inventor: Danchin, Etienne, 06600 Antibes (FR); Abad, Pierre, 06800 Cagnes-sur-Mer (FR); Perfus-Barbeoch, Laetitia, 06600 Antibes (FR)
(74) Representative: Rançon, Xavier Lucien Abel

(57) **Abstract**

The invention relates to a method for reducing the plant-parasitic nematode infestation level in a plant, comprising expressing in said plant a RNA capable of post-transcriptional inhibition of a target gene from said nematode encoding an effector-like protein or a transcription factor.

SEQUENCE LISTING

<110>  GENOPLANTE-VALOR
       DANCHIN , Etienne
       ABAD, Pierre
       PERFUS-BARBEOCH, Laetitia

<120>  METHOD FOR REDUCING THE PLANT-PARASITIC NEMATODE INFESTATION
       LEVEL IN A PLANT

<130>  XRN/cc-F1516/41/EP

<160>  180

<170>  PatentIn version 3.5

<210>  1
<211>  155
<212>  PRT
<213>  Meloidogyne incognita

<220>
<221>  SIGNAL
<222>  (1)..(29)

<220>
<221>  DOMAIN
<222>  (6)..(17)
<223>  MERCI effector-specific motif

<220>
<221>  DOMAIN
<222>  (7)..(21)
<223>  MERCI effector-specific motif

<400>  1

Met Leu Arg Leu Thr Ala Leu Leu Leu Ile Ala Leu Leu Cys Leu Glu
1               5                   10                  15

Met Val Asn Ser Cys Gly Phe Pro Met Pro Ala Pro Ala Pro Thr Met
            20                  25                  30

Cys Cys Cys Gly Gly Gly Gly Gly Gly Gly Cys Gly Arg Arg Lys Arg
        35                  40                  45

Ala Val Gln Pro His Phe Lys Gly Ser Glu Ile Pro Cys Pro Gln Ile
    50                  55                  60

Glu Trp Lys Arg Ile Val Glu Lys Ala Ile Ile Glu Asn Val Gly Lys
65                  70                  75                  80

Glu Met Ala Thr Asn Ala Ile Gln Ser Ala Met Asn Ile Arg Phe Pro
                85                  90                  95

Asp His Lys Phe Ile Val Thr Cys Ala Ser Leu His Asn Ser Asn Val

            100                 105                 110

Ile Asp Gly Lys Ser Ile Ala Ile Arg Gly Val Lys Ile Glu Glu Met
        115                 120                 125

Val Thr Phe Ile Ser Ala Gly Asp Gly Tyr Cys Asn Val Met Asp Lys
    130                 135                 140

Gly Lys Trp Cys Gln Val Val Ala Leu Ser Gly
145                 150                 155

<210> 2
<211> 160
<212> PRT
<213> Meloidogyne incognita

<220>
<221> SIGNAL
<222> (1)..(29)

<220>
<221> DOMAIN
<222> (11)..(25)
<223> MERCI effector-specific motif

<400> 2

Met Ser Ile Ser Phe Asn Asp Gln Pro Lys Leu Val Leu Phe Ile Ala
1               5                   10                  15

Leu Cys Ser Ile Ser Leu Val Glu Ser Ala Tyr Thr Tyr Gln Lys Leu
            20                  25                  30

Asn Glu Leu Met Pro Glu Leu Arg Gln Phe Leu Gly Leu Thr His Leu
        35                  40                  45

Gly Thr Leu Arg Pro Leu Asn Thr Pro Lys Ala Asn Gln Gln Pro Ile
    50                  55                  60

His Leu Ser Ala Phe Ala Ile Pro Ser Phe Ala Asp Gln Gly Gln Phe
65                  70                  75                  80

Gln Ala Ser Lys Arg Thr Pro Asp Tyr Asn Glu Leu Arg Val Arg Lys
                85                  90                  95

Ser Asn Asn Leu Arg Asn Leu Met Arg Ile Gly Lys Arg His Leu Asp
            100                 105                 110

Ser Asn Glu Gln Lys Gly Asn Ser Ala Phe Ala Pro Asn Gly Asp Glu
        115                 120                 125

Gln Thr Gly Gly Asn Ile Tyr Tyr Tyr Ser Pro Ser Phe Pro Ile Asp
    130                 135                 140

Asn Gln Leu Pro Phe Ile Lys Pro Tyr Arg Pro Gln Ser Ile Trp Leu
145                 150                 155                 160

<210>  3
<211>  695
<212>  PRT
<213>  Meloidogyne incognita

<220>
<221>  SIGNAL
<222>  (1)..(29)

<220>
<221>  DOMAIN
<222>  (1)..(15)
<223>  MERCI effector-specific motif

<220>
<221>  misc_feature
<222>  (695)..(695)
<223>  Xaa can be any naturally occurring amino acid

<400>  3

Met Ile Leu Gln Thr Leu Thr Ile Leu Ile Leu Ile Ile Thr Cys Phe
1               5                   10                  15

Lys Gln Cys Arg Gly Glu Leu Arg Glu Met Asp Glu Ala Ser Arg Ser
            20                  25                  30

Lys Arg Gln Tyr Ile Thr Asn Ala Ile Pro Gln Ser Thr Ser Ser Cys
        35                  40                  45

Asn Cys Leu Ser Lys Tyr Thr Pro Val Asn Cys Lys Cys Thr Gly Met
    50                  55                  60

Pro Ser Ile Ser Asp Glu Leu Leu Cys Arg Cys Asp Arg Ala Ser Leu
65                  70                  75                  80

Ala Arg Glu Glu Glu Asn Lys Gln Gln Cys Pro Cys Arg Asn Lys Gly
                85                  90                  95

Ala Gly Asn Gly Trp Gly Glu Ser Glu Gly Gly Tyr Phe Pro Val Tyr
            100                 105                 110

Arg Gly Glu Glu Arg Ser Ser Asp Leu Tyr Pro Arg Ala Leu Ser Pro
        115                 120                 125

Ser Val Ile His Ser Ala Cys His Pro Ile Cys His Gln Ser Cys Leu
    130                 135                 140

Ser Thr Cys Thr Arg Pro Tyr Thr Arg Glu Ile Asp Pro Tyr Arg Glu
145                 150                 155                 160

Arg Glu Arg Tyr Ser Ser Gly Tyr Ala Asn Asp Gln Asp Pro Gln Ser
                165                 170                 175

Glu Asn Ser Leu Leu Ala Ala Asp Tyr Ser Pro Thr Gly Cys Gln Val
            180                 185                 190

Ser His Cys Arg Thr Asn Cys Leu Lys Ala Leu Pro Tyr Gln Glu Thr
        195                 200                 205

Glu Pro Pro Thr Pro Ser His Tyr Glu Val Arg Pro Val Asp Arg Pro
    210                 215                 220

Ile Tyr Val Thr Lys Leu Pro Pro Leu Gln Thr Asp Tyr Gly Ala Ser
225                 230                 235                 240

Ala Leu Ser Ser Cys Gln Cys Ser Pro Asp Ala Ile Cys Glu Cys Pro
                245                 250                 255

Glu Asn Cys Phe Ala Val Cys Phe Asp Ser Cys Glu Ala Asn Cys Asp
            260                 265                 270

Arg Gln Gln Ile Lys Thr Ser Gln Asn Asn Asn Gln Lys Arg Leu Cys
        275                 280                 285

Ala Gln Thr Cys His Phe Asp Cys Gln Arg Asn Cys Ala Glu Lys Gln
    290                 295                 300

Gln Gln Lys Gln Ala Arg Arg Arg Leu Gly Gly Thr Glu Ile Phe Ser
305                 310                 315                 320

Ser Gly Thr Ser Ser Pro Ala Pro Pro Thr Leu Thr Ser Ile Ala Val
                325                 330                 335

Thr Glu Asn Asn Asn Asp Leu Asn Asn Asn Leu Gln Gln Gln Thr Val
            340                 345                 350

Ile Asn Gly Pro Ala Thr Asn Cys Val Ala Arg Cys Pro Lys Ser Cys
        355                 360                 365

Val Val Ser Cys Leu Ser Thr Gln Thr Asn Glu Asp Pro Thr Ile Cys
    370                 375                 380

Val Pro Ala Cys Val Ala Val Cys Ala Gln Ser Cys Ser Arg Gly Leu
385                 390                 395                 400

Gln His Phe Asp Ser Leu Pro Gln Gln Asn Lys Gly Glu Leu Ile Lys
                405                 410                 415

Ser Glu Ser Gln Ile Gln Gln Gln Ile Gln Gln Ile Leu Leu Gln Gly
            420                 425                 430

His Arg Asn Glu Gly Leu Glu Asn Gly Thr Ser Tyr Ser Asn Val Thr
        435                 440                 445

Val Gln Ser Glu Asn Glu Lys Lys Gln Met Leu Val Thr Gln Pro Ser
    450                 455                 460

Met Phe Leu Asn Asp Val Asp Arg Glu Val Ser Pro Gln His Gly Ile
465                 470                 475                 480

Cys Gly Asp Tyr Leu Lys Met Asn Glu Ser Leu Glu Ile Phe Gly Arg
                485                 490                 495

Val Leu Asn Asp Gly Ile Ile Arg Ser Arg Pro Cys Ala Pro Leu Cys
            500                 505                 510

Arg Pro Gln Cys Gln Arg Glu Cys Thr Ser Lys Tyr Val Glu Glu Arg
        515                 520                 525

Gln Ser Pro Phe Ser Thr Phe Ser Gly Arg Lys Arg Glu Phe Asn Lys
    530                 535                 540

Gln Lys Gly Phe Trp Asp Asn Leu Asn Arg Lys Lys Ile Leu Asn Glu
545                 550                 555                 560

Arg Asn Asn Arg Val Phe Pro Pro His Tyr Ile Leu Pro Leu Asn Glu
                565                 570                 575

Gly Leu Asn Glu Glu Lys Asn Lys Glu Ile Asn Lys Glu Glu Asn Lys
            580                 585                 590

Ile Ile Asn Tyr Asp Glu Asn Lys Ser Thr Glu Leu Leu Ser Leu Gln
        595                 600                 605

Lys Glu Gln Asn Gln Thr Glu Asn Ile Gly Asn Lys Gln Leu Leu Asn
    610                 615                 620

Ser Lys Lys Leu Asn Ile Gly Lys Lys Glu Ile Glu Glu Arg Gln Asn
625                 630                 635                 640

Asn Leu Ile Ile Lys Thr Leu Ser Asn Ser Lys Pro Trp Lys Val Lys
                645                 650                 655

Gln Lys Asn Lys Glu Asn Lys Glu Glu Leu Asn Lys Asn Asn Glu Phe
            660                 665                 670

Asn Lys Asp Leu Arg Lys Glu Leu Lys Arg Asn Glu Gln Asn Lys Pro
        675                 680                 685

Lys Trp Phe Phe Leu Phe Xaa
    690                 695

<210>  4
<211>  484
<212>  PRT
<213>  Meloidogyne incognita

<220>
<221>  SIGNAL
<222>  (1)..(29)

<220>
<221>  DOMAIN
<222>  (5)..(19)
<223>  MERCI effector-specific motif

<220>
<221>  DOMAIN
<222>  (9)..(23)
<223>  MERCI effector-specific motif

<400>  4

Met Phe Pro Asn Trp Ala Leu Leu Leu Pro Ile Phe Gly Leu Phe Ala
1               5                   10                  15

Tyr Ser Ser Ala Gln Ser Arg Cys Asn Phe Ile Cys Glu Ala Asp Trp
            20                  25                  30

Gln Thr Arg Val Asp Pro Thr Asp Gly Lys Thr Tyr Gly Tyr Lys Val
        35                  40                  45

Phe Phe Lys Ser Ala Leu Asn Phe Phe Glu Ala Gln Arg Ile Cys Arg
    50                  55                  60

Asp Asn Cys Ala Glu Val Ala Thr Ile His Ser Arg Asp Glu Tyr Asp
65                  70                  75                  80

Phe Val Phe Leu Thr Phe Arg Asp Leu Leu Asp Gln Cys Gln Thr Asn
                85                  90                  95

Ala Ser Val Cys Pro Ile Arg Asp Pro Asn Pro Asp Phe Asn Arg Ile
            100                 105                 110

Leu Asn Ser Phe Trp Leu Gly Met His Arg Ile Gln Gln Tyr Thr Ser
        115                 120                 125

Ile Cys Pro Ala Asp Arg Ala Val Ile Glu Asp Cys Thr Gln Leu Leu
    130                 135                 140

Asp Ser Ala Thr Gly Lys Trp Asn Asp Ile Pro Cys His Tyr Gln Gln
145                 150                 155                 160

Ala Gly Val Val Cys Lys Arg Asp Cys Ala Ser Phe Cys Gln Ala Gln
                165                 170                 175

Val Pro Pro Ile Thr Thr Thr Glu Ala Thr Thr Thr Ile Ser Ser Pro
            180                 185                 190

Gly Gly Gln Pro Gly Gly Pro Gly Gln Pro Gly Pro Asn Gly Pro Asn
        195                 200                 205

Gly Ala Asp Ala Thr Thr Ser Ala Pro Gly Gly Gly Asp Asn Pro Ser
    210                 215                 220

Pro Gly Gly Asn Gly Pro Asn Gly Gln Pro Gly Gln Pro Gly Pro Asn
225                 230                 235                 240

Gly Pro Asn Gly Ala Asp Ala Thr Thr Ser Ala Pro Gly Gly Gly Asp
                245                 250                 255

Asn Pro Ser Pro Gly Gly Asn Gly Pro Asn Gly Gln Pro Gly Gln Pro
            260                 265                 270

Gly Pro Asn Gly Pro Asn Gly Ala Asp Ala Thr Thr Ser Ala Pro Gly
        275                 280                 285

Gly Gly Asp Asn Pro Ser Pro Gly Gly Asn Gly Pro Asn Gly Gln Pro
    290                 295                 300

Gly Gln Pro Gly Pro Asn Gly Pro Asn Gly Ala Asp Ala Thr Thr Ser
305                 310                 315                 320

Ala Pro Gly Gly Gly Asn Asn Pro Ser Pro Gly Gly Asn Gly Pro Asn
                325                 330                 335

Gly Gln Pro Gly Gln Pro Gly Pro Asn Gly Pro Asn Gly Ala Asp Ala
            340                 345                 350

Thr Thr Ser Ala Pro Gly Gly Gly Asn Asn Pro Ser Pro Gly Gly Asn
        355                 360                 365

Gly Pro Asn Gly Gln Pro Gly Gln Pro Gly Pro Asn Gly Pro Asn Gly
    370                 375                 380

Ala Asp Ala Thr Thr Ser Ala Pro Gly Gly Gly Asp Asn Pro Ser Pro
385                 390                 395                 400

Gly Gly Asn Gly Asn Pro Gly Gln Asn Gly Thr Asn Gly Gln Pro Gly
                405                 410                 415

Gln Asn Gly Thr Ser Phe Thr Asn Ser Pro Gly Glu Gly Gly Asn Gly
            420                 425                 430

Asp Leu Thr Thr Thr Pro Glu Pro Thr Thr Ile Glu Pro Val Thr Ile
        435                 440                 445

Thr Ser Thr Met Arg Ala Pro Leu Pro Pro Pro Ala Pro Arg Arg Arg
    450                 455                 460

Ser Ser Arg Ser Pro Arg Ser Thr Gln Glu Thr Thr Thr Ser Asn Pro
465                 470                 475                 480

Asn Ile Val Ser

<210>  5
<211>  348
<212>  PRT
<213>  Meloidogyne incognita

<220>
<221>  SIGNAL
<222>  (1)..(29)

<220>
<221>  DOMAIN
<222>  (1)..(15)
<223>  MERCI effector-specific motif

<220>
<221>  DOMAIN
<222>  (4)..(18)
<223>  MERCI effector-specific motif

<400>  5

Met Phe Ala Ile Gly Gly Leu Cys Leu Leu Leu Trp Leu Ala Val Val
1               5                   10                  15

Asn Ala Ser Asn Ser Ser Glu Val Gln Val Gly Ser Gly Asn Tyr Ala
            20                  25                  30

Leu Lys Asp Gly Lys Val Ala Leu Ser Asp Glu Phe Gly Ile Tyr Asn
        35                  40                  45

Trp Pro Gly Leu Thr Leu Val Pro Asp His Leu Tyr Pro Asn Cys Ser
    50                  55                  60

Thr Ile Arg Ile Asp Glu Asn Phe Ile Ser Leu Trp Tyr Asn Glu Thr
65                  70                  75                  80

Ser Lys Val Val Cys Thr Val Asp Leu Leu Thr Lys Arg Asp Asp Ile
                85                  90                  95

Glu Phe Thr Thr Ser Leu Glu Leu Cys Arg Asp Thr Lys Arg Glu Asp
            100                 105                 110

Leu Lys Asp Pro Thr Val Leu Pro Phe Ala Tyr Ser Leu Asn Asn Lys
        115                 120                 125

Glu Val Asn Glu Ile Lys Asn Gly Pro Leu Tyr Gly Lys Asp Asn Ala
    130                 135                 140

Ile Cys Pro Arg Asp Arg Ser Glu Cys Thr Gly Ser Thr Ser Gly Asn
145                 150                 155                 160

Trp Thr Glu Ile Pro Thr Pro Asp Cys Met Pro Trp Thr Ala Leu Glu
                165                 170                 175

Val Glu Trp Arg Tyr Glu Lys Glu Ser Trp Lys Glu Leu Glu Asp Arg
            180                 185                 190

Ser Cys Thr Leu Tyr Asn Asn Thr Leu Thr Ala Asn Val Ser Phe Ile
        195                 200                 205

Gly Asp Ile Gly Tyr Val Ala His Phe Trp Ile Glu Glu Phe Glu Lys
    210                 215                 220

Lys Ala Gln Phe Ala Val Asn Val Asp Lys Asn Gly Asn Val Lys Ser
225                 230                 235                 240

Thr Asn Leu Trp Lys Asp Asp Gly Trp Thr Asp Lys Glu Ser Asn His
                245                 250                 255

Asn Leu Asp Asp Phe Lys Cys Ile Glu Ala Ser Ile Ile Lys Pro Lys
            260                 265                 270

Val Trp Glu Ile Asp Gly Asn Val Arg Lys Glu Ile Glu Gly Lys Lys
        275                 280                 285

Leu Met Thr Phe His Leu Leu Pro Arg Ser Ala Ser Arg Ala Arg Asp
    290                 295                 300

Glu Leu Gly Ala Arg Leu Thr Gly Lys Leu Pro Asp Leu Thr His Asn
305                 310                 315                 320

Asn Cys Arg Asp Met Tyr Ile Val Phe Lys Arg Pro Asp Tyr Glu Leu
                325                 330                 335

Leu Glu Arg Leu Pro Val Val Tyr Thr Pro Val Leu
            340                 345

<210>  6
<211>  253
<212>  PRT
<213>  Meloidogyne incognita

<220>
<221>  SIGNAL
<222>  (1)..(29)

<220>
<221>  DOMAIN
<222>  (2)..(16)
<223>  MERCI effector-specific motif

<220>
<221>  DOMAIN
<222>  (3)..(17)
<223>  MERCI effector-specific motif

<400>  6

Met Ile Leu Leu Ile Ile Thr Ala Ile Val Cys Gln Ile Asn Ala Gln
1               5                   10                  15

Gln Pro Pro Tyr Ile Pro Pro Gln Asn Ile Asn Gly Thr Phe Gly Leu
            20                  25                  30

Gln Leu Asn Ser Tyr Pro Ser Pro Gln His Leu Phe Gln Tyr Gln Arg
        35                  40                  45

Gln Gln Gln Pro Ser Asp Tyr Arg Gln Gln Gln Gln Tyr Ala Ala Asp
    50                  55                  60

Gly His Tyr Leu Gln Gln Pro Gln Tyr Asn Ser His Tyr Gln Asn Ser
65                  70                  75                  80

Phe Gln Pro Pro Asn Lys Trp Gln Asn Glu Gln Lys Leu Glu Asp Asp
                85                  90                  95

Asp Ser Lys Glu Glu Asn Asn Phe Leu Ser Lys Gln Asn Ser Asn Asn
            100                 105                 110

Arg Gln Tyr Pro Gln Arg Val His Phe Asp Pro Asn Asn Asn Leu Pro
        115                 120                 125

Tyr Leu Tyr Asp Arg Gln Gln Gln Lys Tyr Asn Gln Lys Ser Tyr Gly
    130                 135                 140

Gly Tyr Asn Asn His Leu Asn Gly Gln Asn Asn Pro Ser Ser Phe Asp
145                 150                 155                 160

Phe Glu Asn Glu Ala Asp Ser Tyr Asn Ser Pro Tyr Thr Tyr Gly Asn
                165                 170                 175

Gln Gln Arg Pro Val Asn Pro Asn Met Gln Thr His Gln Tyr Thr Gly
            180                 185                 190

Gln Gln His Ser Tyr Pro Ala Gly Pro Leu Asn Pro His Tyr Arg Ala
        195                 200                 205

Gly Ile Val Tyr Pro Glu Gly Gln Arg Asn Leu Asn Asn His Asn Lys
    210                 215                 220

His Phe Trp Phe Asn Asp Ala Arg Lys Asn Tyr Lys Ile Asn Lys Phe
225                 230                 235                 240

Trp Ile Leu Val Ile Phe Gly Ile Leu Ile Leu Phe Ile
                245                 250

<210>  7
<211>  413
<212>  PRT
<213>  Meloidogyne incognita

<220>
<221>  SIGNAL
<222>  (1)..(29)

<220>
<221>  DOMAIN
<222>  (1)..(15)
<223>  MERCI effector-specific motif

<400>  7

Met Phe Phe Ile Asn Ile Phe Ile Ile Phe Leu Ile Ile Lys Ile Ile

1               5                   10                  15

Thr Gly Gln Phe Glu Asp Glu Tyr Thr Ile Asn Ala Arg Ala Gly Ile
            20                  25                  30

Val Asp Lys Leu Phe Asp Gly Asp Ile Asp Leu Arg Pro Glu Glu Tyr
        35                  40                  45

Tyr Gln Leu Lys Arg Ser Lys Phe Phe Ser Asp Pro Ala Ala Arg Lys
    50                  55                  60

Asn Ala Leu Phe Thr Asn Trp Glu Thr Asp Glu Lys Tyr His Trp Pro
65                  70                  75                  80

His Gln Pro Ser Thr Thr Gln Gly Val Arg Tyr Gly Pro Leu Ile Asn
                85                  90                  95

Tyr Thr Leu Asp Trp Glu Lys Ile Val Glu Pro Pro Ser Phe Val Trp
            100                 105                 110

Leu Lys Asn Ile Leu Asp Ala His Lys Asn Ile Glu Glu Asn Thr Cys
        115                 120                 125

Ile Arg Phe Ala Asn Ser Ser Glu Gly Asp His Ile His Tyr Tyr Trp
    130                 135                 140

Gly Tyr Gly Cys His Ser Leu Val Gly Arg Asn Gly Gly Lys Gln Trp
145                 150                 155                 160

Leu Ser Leu Gly Pro Gly Cys Trp Thr Val Asn Ala Val His Glu Thr
                165                 170                 175

Leu His Ala Leu Gly Ser Asn His Glu Gln Ser Arg Gln Asp Arg Asp
            180                 185                 190

Thr Phe Ile Lys Ile Asn Glu Glu Asn Met Ile Glu Asn Ile Ser Asp
        195                 200                 205

Ala Phe Ala Val Ile Ala Gly Ser Pro Thr Gly Asn Pro Tyr Asp Phe
    210                 215                 220

Gly Ser Cys Met His Tyr Ser Asn Gly Ala Phe Ser Lys Asn Lys Lys
225                 230                 235                 240

Pro Thr Ile Glu Thr Leu Asp Lys Asn Tyr Gln His Thr Ile Gly Tyr
                245                 250                 255

Arg Leu Lys Leu Ser Phe Leu Asp Thr Lys Ala Leu Asn His Arg Tyr
            260                 265                 270

Cys Glu His Ile Cys Asp Asp Leu Thr Trp Ile Thr Pro Asp Cys Arg
        275                 280                 285

Asn Asn Gly Lys Asp Pro Leu Tyr Ala Glu Cys Pro Gly Glu Pro Asn
    290                 295                 300

Ile Leu Leu Thr Pro Asp Thr Pro Glu Met Glu Glu Ile Arg Trp Phe
305                 310                 315                 320

Asn Ala Ser Lys Phe Thr Cys Tyr Trp Arg Ile Gln Ala Pro Ala Asp
                325                 330                 335

His Tyr Ile Gly Val Asn Leu Thr Lys Met Glu Gly Phe Lys Cys Glu
            340                 345                 350

Gln Ser Cys Tyr Ser Phe Val Glu Ile Lys Tyr Gly Asn Met Ala Asn
        355                 360                 365

Ser Gly Pro Arg Phe Cys Cys Asp Leu Pro Thr Glu Thr Leu Tyr Thr
    370                 375                 380

Glu Val Val Gly His Glu Val Ile Ile Ile Thr His Gly Tyr Lys Pro
385                 390                 395                 400

Gly Asn Tyr Ser Val Asn Leu Ser Tyr Ser Ile Thr Lys
                405                 410

<210>  8
<211>  184
<212>  PRT
<213>  Meloidogyne incognita

<220>
<221>  SIGNAL
<222>  (1)..(29)

<220>
<221>  DOMAIN
<222>  (10)..(24)
<223>  MERCI effector-specific motif

<220>
<221>  DOMAIN
<222>  (16)..(27)
<223>  MERCI effector-specific motif

<400>  8

Met Leu Pro Ile Phe Ser Ser Lys Asn Asn Phe Ser Leu Phe Gln Thr

1               5                   10                  15

Thr Leu Ile Leu Leu Leu Leu Ile Ser Gln Ser Gln Ser Val Phe Tyr
            20                  25                  30

Arg Lys Ile Pro Phe Arg Tyr Lys Ser Val Val Glu Lys Pro Trp Thr
        35                  40                  45

Asn Thr Glu Asp Asp Asn Ile Ala Ala Ala Ile Leu Thr Ser Arg Glu
    50                  55                  60

Asp Lys Glu Glu Gly Glu Gln Asn Ile Ala Gln Leu Leu Glu Gln Ile
65                  70                  75                  80

Glu Gln Ser Ser Leu Asp Ser Pro Asp Asn Asp Ser Glu Glu Asp Tyr
                85                  90                  95

Thr Asn Leu Lys Glu Asp Ile Leu Pro Leu Asn Gln Lys Leu Gln Lys
            100                 105                 110

Ile Asn Asn Asn Leu Ile Lys Gln Arg Thr Asp Lys Asn Gly Glu Glu
        115                 120                 125

Val Leu Thr Leu Pro Lys Asn Ile Leu Ile Glu Lys Glu Asp Ile Leu
    130                 135                 140

Pro Lys Tyr Thr His Leu Met Ala Phe Arg Pro Asp Tyr Thr Ile Arg
145                 150                 155                 160

Asn Phe Ala Ser Phe Leu Arg Arg Phe Leu Asp Ser Arg Ile Ile Ala
                165                 170                 175

Ala Thr Val Asp Asp Glu Thr Lys
            180

<210>  9
<211>  274
<212>  PRT
<213>  Meloidogyne incognita

<220>
<221>  SIGNAL
<222>  (1)..(29)

<220>
<221>  DOMAIN
<222>  (4)..(18)
<223>  MERCI effector-specific motif

<220>

<221>  DOMAIN
<222>  (5)..(19)
<223>  MERCI effector-specific motif

<400>  9

Met Lys Val Ile Phe Val Phe Ile Ser Met Ala Ala Leu Leu Gly Val
1               5                   10                  15

Asn Thr Ala Glu Asn Gly Thr Ala Thr Thr Thr Ser Thr Ala Thr Asn
            20                  25                  30

Ile Thr Lys Pro Thr Pro Thr Val Asp Asn Pro Pro Pro Ser Thr Pro
        35                  40                  45

Thr Thr Glu Val Asp Pro Pro Leu Glu Ser His Ser Thr Thr Asn Lys
    50                  55                  60

Met Asp Gly Ala Lys Thr Lys Asn Ile Ser Glu Cys Pro Ile Thr Thr
65                  70                  75                  80

Arg Leu Tyr Asp Asn Ile Gly Glu Lys Gly Asp Gly Asn Asn Lys Lys
                85                  90                  95

Asn Cys Thr Glu Pro Gly Cys Phe Phe Ile Tyr Glu Gln Met Asn Leu
            100                 105                 110

Gly Trp Asp Phe Pro Ser Phe Leu Val Gly Asn Lys Arg Arg Gly Met
        115                 120                 125

Leu Leu Tyr Leu Thr Gly Cys Ile Tyr Asp Ile Pro His Leu Leu Glu
    130                 135                 140

Arg Thr Lys Gly Lys Glu Arg Ile Asn Arg Ala Leu Arg Ala Val Arg
145                 150                 155                 160

Ala Cys Tyr Ile Gly Lys Arg Met Asp Ala Gly Lys Ser Gln His Cys
                165                 170                 175

Gln Pro Glu Tyr Glu Gly Glu Gly Gln Ile Ser Phe Asn Gln Gly Met
            180                 185                 190

Asp Pro Ile Thr Arg Lys Asp Lys His Arg Asp Thr Trp Ala Gly Arg
        195                 200                 205

Phe Leu Pro Ser Thr Gln Asn Arg Cys Phe Val Glu Phe Leu Asn Gly
    210                 215                 220

Ile Phe Tyr Val Asn Asp Asp Gly Lys Ser Ile Ile Val Cys Cys Ser

225                 230                 235                 240

Glu Gly Tyr Thr Ser Glu Lys Glu Val Ala Ser Phe Lys Ser Asp Cys
                245                 250                 255

His Asp Thr Thr Lys Arg Ile Asp Ala Leu Glu Trp Arg Ala Thr Ile
            260                 265                 270

Asp Asn

<210>  10
<211>  361
<212>  PRT
<213>  Meloidogyne incognita

<220>
<221>  SIGNAL
<222>  (1)..(29)

<220>
<221>  DOMAIN
<222>  (5)..(19)
<223>  MERCI effector-specific motif

<400>  10

Met Arg Phe Asp Ile Phe Leu Leu Val Ala Phe Ser Leu Gly Val Ala
1               5                   10                  15

Val Asn Cys Gly Val Val Lys Arg Asn Gln Asn Ser Tyr Gly Asp Glu
            20                  25                  30

Ala Gly Ala Gly Gly Ala Ala Gly Ala Ala Pro Ala Ala Pro Ala Glu
        35                  40                  45

Ala Ala Ala Pro Ser Gly Gly Glu Ala Ala Pro Ala Ala Ala Pro Ala
    50                  55                  60

Pro Glu Ala Ala Pro Ala Ala Ala Pro Ala Pro Glu Gln Ala Ala Pro
65                  70                  75                  80

Ala Val Ala Gln Pro Ala Pro Ala Ala Ala Ala Ser Gln Ala Ser Gly
                85                  90                  95

Tyr Arg Lys Lys Arg Ser Gln Asn Ser Tyr Gly Asp Glu Ala Gly His
            100                 105                 110

Ala Ala Gly Ala Ala Pro Ala Glu Ala Ala Pro Ala Pro Ala Glu Ser
        115                 120                 125

Ala Ala Pro Ser Gly Gly Glu Ala Ala Pro Ala Ala Ala Pro Ala Pro
    130                 135                 140

Glu Ala Ala Pro Ala Pro Glu Ala Ala Pro Ala Ala Ala Pro Ala Pro
145                 150                 155                 160

Glu Gln Ala Ala Pro Ala Val Ala Gln Pro Ala Pro Ala Ala Ala Ala
                165                 170                 175

Ser Gln Ala Ser Gly Tyr Arg Lys Lys Arg Ser Gln Asn Ser Tyr Gly
            180                 185                 190

Asp Glu Ala Gly Ala Gly Gly Ala Ala Gly Ala Ala Pro Ala Glu Ser
        195                 200                 205

Ala Ala Pro Ala Gly Gly Glu Ala Ala Ala Pro Ala Ala Thr Gly Cys
    210                 215                 220

Thr Glu Asn Cys Ala Ala Gly Gly Glu Ser Ala Ala Pro Ala Ala Ala
225                 230                 235                 240

Pro Ala Pro Glu Ala Ala Pro Ala Ala Ala Pro Ala Pro Glu Gln Ala
                245                 250                 255

Ala Pro Ala Val Ala Gln Pro Ala Pro Ala Ala Ala Pro Ser Gln Ala
            260                 265                 270

Ser Gly Tyr Arg Lys Lys Arg Ser Gln Asn Ser Tyr Gly Asp Glu Ala
        275                 280                 285

Gly His Ala Ala Gly Ala Ala Pro Ala Glu Ser Ala Ala Pro Ser Gly
    290                 295                 300

Gly Glu Ala Ala Ala Pro Ser Gly Gly Glu Ala Ala Ala Pro Ala Pro
305                 310                 315                 320

Glu Ala Ala Pro Ala Pro Ala Pro Ala Glu Ala Ala Pro Ala Ala Ala
                325                 330                 335

Pro Ala Pro Glu Gln Ala Ala Pro Ala Val Ala Gln Pro Ala Pro Ala
            340                 345                 350

Ala Ala Pro Ser Gln Ala Ser Gly Tyr
        355                 360

<210>  11
<211>  300

<212>  PRT
<213>  Meloidogyne incognita

<220>
<221>  SIGNAL
<222>  (1)..(29)

<220>
<221>  DOMAIN
<222>  (4)..(18)
<223>  MERCI effector-specific motif

<220>
<221>  DOMAIN
<222>  (6)..(20)
<223>  MERCI effector-specific motif

<220>
<221>  DOMAIN
<222>  (9)..(23)
<223>  MERCI effector-specific motif

<400>  11

Met Leu Val Leu Pro Phe Val Ala Met Leu Ala Phe Leu Leu Pro Ser
1               5                   10                  15

Val Leu Leu Ile Gln Cys Cys Lys Lys Lys Ala Lys Asp Ala Ala Thr
            20                  25                  30

Ile Glu Val Gly Ala Lys Ser Pro Leu Asp Ala Gly Lys Ser Lys Ala
        35                  40                  45

Lys Val Gly Gly Gly Lys Lys Pro Ser Pro Asp Gly Ala Val Lys Lys
    50                  55                  60

Glu Lys Lys Ser Glu Lys Pro Val Gln Val Gln Thr Pro Thr Ala Ala
65                  70                  75                  80

Ala Gly Pro Ser Leu Ala Thr Ala Phe Pro Pro Glu Lys Thr Lys Ser
                85                  90                  95

Lys Pro Asn Ile Lys Gln Gly Ala Ser Thr Lys Thr Ala Ala Val Arg
            100                 105                 110

Thr Lys Ala Gly Ala Val Thr Pro Ala Ala Lys Lys Asn Glu Val Thr
        115                 120                 125

Ala Val Ala Leu Glu Lys Pro Lys Lys Glu Glu Lys Thr Lys Leu Val
    130                 135                 140

Asn Ala Glu Thr Gly Lys Ala Lys Pro Glu Val Asn Pro Met Ser Ala
145                 150                 155                 160

Pro Ser Lys Tyr Asp Asp Gly Thr Pro Thr Val Ile Leu Thr Pro Ala
                165                 170                 175

Asp Met Met Ser Val Ala Ala Met Thr Ala Asn Pro Asn Ser Thr Glu
            180                 185                 190

Ala Pro Asp Asp Lys Ser Lys Val Gly Gln Lys Thr Ser Ala Ser Phe
        195                 200                 205

Tyr Asp Leu Asp Thr Thr Asn Lys Thr Ser Asp Ser Val Tyr Glu Asn
    210                 215                 220

Val Glu Leu Pro Lys Thr Lys Ser Thr Val Glu Met Asp Gly Glu Lys
225                 230                 235                 240

Gly Thr Gly Lys Glu Asp Gly Lys Asp Glu Asp Asp Asp Asp Thr Glu
                245                 250                 255

Lys Ser Ile Met Lys Ser Lys Ile Ala Asp Glu Lys Lys Ala Glu Glu
            260                 265                 270

Gln Lys Lys Gln Pro Met Ala Ala Pro Asn Glu Tyr Gln Thr Leu Ala
        275                 280                 285

Asn Val Ser Thr Asp Lys Val Phe Gln Lys Lys Gly
    290                 295                 300

<210>  12
<211>  387
<212>  PRT
<213>  Meloidogyne incognita

<220>
<221>  SIGNAL
<222>  (1)..(29)

<220>
<221>  DOMAIN
<222>  (7)..(18)
<223>  MERCI effector-specific motif

<400>  12

Met Ile Ser Ile Thr Ser Phe Phe Leu Leu Phe Leu Ser Leu Leu Phe
1               5                   10                  15

Leu Phe Asp Phe Ser Thr Ser Asn Cys Ile Glu Tyr Cys Phe Val Pro
            20                  25                  30

Pro Gln Gln Tyr Ser Gly Cys Asn Ile Cys Gly Gly Tyr Gly Tyr Gly
        35                  40                  45

Ser Gly Tyr Gly Phe Thr Gly Gly Tyr Gly Gly Tyr Val Pro Gln Gln
    50                  55                  60

Thr Gln Gln Leu Tyr Val Pro Gln Gln Gln Gln Tyr Val Pro Gln Gln
65                  70                  75                  80

Val Val Val Gln Pro Ala Gly Gly Gly Val Gly Gly Ser Gly Tyr Pro
                85                  90                  95

Ser Gly Gln Gly Tyr Ile Pro Gln Ser Gly Gly Val Ile Pro Gln Thr
            100                 105                 110

Thr Gly Gly Gln Ser Tyr Val Gly Thr Thr Gly Asn Gly Gly Gly Asn
        115                 120                 125

Val Gly Gly Gly Thr Ser Ser Cys Gly Ser Gly Gly Cys Ser Ala Gly
    130                 135                 140

Gly Thr Gly Gly Thr Thr Gly Gly Ser Asn Tyr Gly Gly Gly Glu Ser
145                 150                 155                 160

Ser Gly Gly Thr Val Ser Gly Ser Gly Leu Gly Gly Ser Thr Gly Gly
                165                 170                 175

Thr Gly Gly Gly Glu Thr Gly Gly Thr Thr Gly Gly Glu Glu Gly Thr
            180                 185                 190

Gly Gly Thr Ser Gly Gly Ser Ser Gly Gly Ser Glu Gly Gly Tyr Gly
        195                 200                 205

Gly Ser Thr Gly Gly Thr Gly Thr Asp Thr Ala Ser Gly Ser Leu Gly
    210                 215                 220

Thr Gly Gly Ser Pro Thr Gly Gly Ser Ser Tyr Gly Ser Gly Gln Ser
225                 230                 235                 240

Gly Thr Gln Thr Ser Gly Tyr Gln Pro Ala Ala Thr Gln Val Ala Thr
                245                 250                 255

Gly Glu Asn Lys Gln Cys Cys Ser Cys Ala Asn Ser Val Gly Cys Tyr
            260                 265                 270

Gln Thr Ser Pro Gln Gln Thr Ala Ala Gly Gly Ser Val Ala Val Gln
        275                 280                 285

Pro Val Ala Val Gln Pro Thr Gly Thr Ala Gln Ala Ser Ser Gln Pro
    290                 295                 300

Gln Lys Gly Tyr Gly Asp Val Arg Arg Lys Lys Phe Ser Ser Arg His
305                 310                 315                 320

Arg Gln Arg Gln Ile Phe Gly Arg Gly Thr Met Val Asn His Met Ala
                325                 330                 335

Met Leu Ser Gln Leu Gln Pro Ile Pro Pro Val Lys Arg Glu Lys Asn
            340                 345                 350

Asn Gly Pro Pro Lys Pro Phe Thr Leu Phe Asn Lys Ser Pro Val Asp
        355                 360                 365

Ala Met Lys Ser Lys Gly Gln Ser Phe Glu Asn Arg Ala Ala Thr Phe
    370                 375                 380

Lys Lys Ile
385

<210>  13
<211>  213
<212>  PRT
<213>  Meloidogyne incognita

<220>
<221>  SIGNAL
<222>  (1)..(29)

<220>
<221>  DOMAIN
<222>  (5)..(19)
<223>  MERCI effector-specific motif

<400>  13

Met Leu Asn Tyr Tyr Phe Ser Ile Leu Ser Ile Phe Ile Leu Val Phe
1               5                   10                  15

Ile Lys Ser Tyr Asp Ala Gln Phe Thr Ile Ser Arg Thr Pro Gly Ser
            20                  25                  30

Lys Pro Leu His Leu Arg Ile Gln Leu Pro Pro Thr Ser Ser Asp Ser
        35                  40                  45

Ile Ser Phe Ile Ala Pro Phe Pro Asn Glu Asn Cys Leu Thr Asn Tyr
    50                  55                  60

Leu Ile Asp Asn Arg Ile Phe Leu Asp Phe Tyr Gly Cys Arg Ile Ser

65                  70                  75                  80

Leu Asp Trp Ser Glu Thr Leu Phe Gly Thr Thr Asn Tyr Phe Lys Ser
                85                  90                  95

Leu Lys Arg Gln Lys Arg Arg Trp Arg Val Gly Pro Val Gln Lys Leu
            100                 105                 110

Lys Met Gly Ser Ala Ile Ala His Tyr Gly Asn Asp Met His Ile Asp
        115                 120                 125

Leu Asp Pro Thr Gly Leu Thr Ala Ser Ser Ile Ala Pro Asn Gly Ile
    130                 135                 140

Gln Val Gly Pro Arg Gln His Lys Lys Phe Lys Cys Leu Leu Asp Ile
145                 150                 155                 160

Ser Ser Ala Lys Ile Leu Asp Phe Asp Ile Phe Met Pro Gln Gly Thr
                165                 170                 175

Ala Lys Cys Gly Ser Leu Ser Ile Gln Phe Asp Pro Asp Tyr Lys Leu
            180                 185                 190

Leu Gly Thr Glu Glu Arg Thr Glu Lys Arg Ile Asn Glu Leu Asp Glu
        195                 200                 205

Thr Ala Ile Ala Asp
    210

<210>  14
<211>  69
<212>  PRT
<213>  Meloidogyne incognita

<220>
<221>  SIGNAL
<222>  (1)..(29)

<220>
<221>  DOMAIN
<222>  (2)..(16)
<223>  MERCI effector-specific motif

<220>
<221>  DOMAIN
<222>  (7)..(21)
<223>  MERCI effector-specific motif

<220>
<221>  DOMAIN
<222>  (12)..(23)
<223>  MERCI effector-specific motif

<400>  14

Met Ala Leu Gln Lys Leu Ile Phe Val Leu Ile Ala Ser Leu Ile Leu
1               5                   10                  15

Ile Asn Leu Ser Ala Asp Ala Gln Asn Thr Pro Ala Phe Tyr Asp Cys
            20                  25                  30

Asn Cys Ala Gln Lys Leu Cys Ser Asn Pro Cys Lys Ser Gly Lys Ser
        35                  40                  45

Ala Cys Asp Asn Cys Lys Ser Ile Asp Ile Pro Lys Gln Cys Ser Ile
    50                  55                  60

Gln Cys Lys Ile Asn
65

<210>  15
<211>  477
<212>  PRT
<213>  Meloidogyne incognita

<220>
<221>  SIGNAL
<222>  (1)..(29)

<220>
<221>  DOMAIN
<222>  (7)..(18)
<223>  MERCI effector-specific motif

<400>  15
   Met Leu Arg Asn Tyr Arg Ile Leu Leu Ala Ile Leu Leu Phe Ala Val
   1               5                   10                  15
   Leu Ile Asp Arg Leu Lys Ala Asp Asp Asp Asp Glu Glu Asn Gly Glu
               20                  25                  30
   Gly Asn Glu Gly Gly Asp Gly Gln Glu Gly Glu Ala Gly Glu Ala Gln
           35                  40                  45
   Glu Asn Glu Glu Gly Ala Ala Asn Glu Glu Lys Glu Ala Glu Asp Gly
       50                  55                  60
   Glu Asn Gln Glu Gly Pro Gly Val Gly Glu Ala Gly Lys Glu Asp Glu
   65                  70                  75                  80
   Glu Gln Thr Asp Lys Thr Asp Glu Asp Val Glu Lys Pro Pro Gly Glu
                   85                  90                  95
   Asp Asp Glu Asn Asn Glu Glu Gly Asp Glu Lys Gly Lys Asp Lys Asn
               100                 105                 110
   Lys Pro Gly Lys Pro Gly Leu Pro Gly Lys Asp Ser Lys Asp Ala Asn
           115                 120                 125
   Lys Lys Asp Asp Lys Gly Gln Gln Gly Gly Ile Asp Gln Gly Asn Lys
       130                 135                 140
   Asp Arg Lys Lys Thr Asn Asn Ala Pro Glu Thr Asp Lys Pro Leu Asn
   145                 150                 155                 160
   Gln Pro Ala Gly Gln Gly Thr Gly Gln Pro Gly Ile Gly Gln Pro Gly
                   165                 170                 175
   Gln Pro Gly Ile Gly Gln Pro Asn Val Asn Pro Asn Asn Asn Ile Pro
               180                 185                 190
   Tyr Ser Pro Thr Val Tyr Pro Asn Gly Leu Pro Gly Gln Ser Gln Gln
           195                 200                 205
   Thr Asn Ile Asn Pro Ser Asn Val Asn Pro Ile Asn Pro Pro Tyr Val
       210                 215                 220
   Asn Pro Ile Pro Asn Pro Val Tyr Pro Gln Gln Thr Asn Val Pro Leu
   225                 230                 235                 240
   Pro Thr Asn Pro Pro Pro Val Tyr Pro Gln Tyr Pro Ser Ala Asn Gln
                   245                 250                 255
   Gln Pro Pro Gln Gln Gln Pro Tyr Ser Pro Pro Ser Gly Phe Gly Thr
               260                 265                 270
   Ser Asn Ser Val Asn Asp Ile Asp Glu Asp Pro Phe Gly Phe Gly Ile
           275                 280                 285
   Thr Leu Gln Arg Gln Gln Gln Gln Gln Tyr Ser Pro Tyr Ser Gln Tyr
       290                 295                 300
   Tyr Ser Gln Pro Asn Gln Asn Ile Pro Pro Gln Tyr Asn Pro Pro Ser
   305                 310                 315                 320
   Asn Asn Leu Tyr Ser Asn Asn Leu Gly Gly Phe Gly Asn Ser Asn Gly
                   325                 330                 335
   Lys Arg Val Pro Gly Gln Thr Asn Phe Pro Val Glu Glu Arg Ser Gln
               340                 345                 350
   Leu Pro Met Ile Val Ser Gly Ser Asn Ser Asn Asn Tyr Asn Asp Gln
           355                 360                 365
   Arg Glu Leu Ser Phe Gly Arg Gly Arg Arg Ser Asp Asp Gln Gln Gln
       370                 375                 380
   Pro Gln Gln Ser Pro Val Gln Pro Thr Val Cys Phe Arg Pro Ser Pro
   385                 390                 395                 400
   Gly Thr Ser Asp Tyr Ala Thr Trp Phe Gln Asn Ile Thr Asn Glu Ile
                   405                 410                 415
   Asn Ser Asn Pro Gln Lys Phe Gln Pro Val Gln Asn Asn Gly Leu Ala
               420                 425                 430
   Asn Leu Leu Gln Gln Gln Ala Leu Asn Gly Gly Ile Pro Glu Ile Cys
           435                 440                 445
   Thr Lys Leu Ala Gln Gln Asn Ile Pro Gln Thr Asn Val Phe Gln Gln
       450                 455                 460
   Gly Ile Leu Ala Arg Lys Lys Arg Ser Ser Gly Arg Glu
   465                 470                 475

<210>  16
<211>  321
<212>  PRT
<213>  Meloidogyne incognita

<220>
<221>  SIGNAL
<222>  (1)..(29)

<400>  16

Met Ala Thr Ile Pro Pro Asn Asn Leu Thr Ile Cys Asn Ile Asp Gly
1               5                   10                  15

Thr Pro Ile Gln Asn Ala Gly Asn Phe Leu Thr Asn Asn Ser Gly Ala
            20                  25                  30

His Ser Trp Gln Thr Glu Gln Thr Ile Asn Leu Glu Thr Ile Asn Leu
        35                  40                  45

Asp Glu Phe Asp Asn Glu His Phe Asn Leu Leu Asn Phe Ile Asn Asp
    50                  55                  60

Gly Ser Ser Pro Leu Glu Glu Ser Gly Pro Arg Leu Glu Asp Asp Glu

65                  70                  75                  80

Ile Gln Lys Gln Lys Ala Glu Ala Leu Tyr Ala Gln Tyr Gln Tyr Asp
                85                  90                  95

Gln Met Met Phe Ala Asn Gln His Leu Gln Gln Arg Asn Asp Phe Gly
            100                 105                 110

Pro Asn Glu Phe Val Thr Ala Pro Val Pro Ser Ser Cys Phe Gly Glu
        115                 120                 125

Val Gly Val Gly Pro Arg Leu Glu Ala Gly Gly Pro Val Arg Asp Glu
    130                 135                 140

Glu Glu Glu Glu Val Arg Arg Arg Leu Leu Glu Pro Pro Asn Thr Ser
145                 150                 155                 160

Ser Leu Val Pro Ser Thr Ser Asn Ala Ser Met Gln Lys Leu Leu Arg
                165                 170                 175

Val Ser Val Val Lys Glu Glu Pro Phe Lys Asp Glu Pro Leu Glu Gln
            180                 185                 190

Asp Asn Val Asn Val Ser Gln Val Phe Gly Ser Ser Ser Ser Arg Ala
        195                 200                 205

Ser Ser Val Ala Ser Ser Val Phe Thr Ser Val Leu Ser Ala Ser His
    210                 215                 220

Ser Gly Thr Pro Arg Lys Tyr Arg Ile Lys Ser Asp Lys Glu Lys Ser
225                 230                 235                 240

Asn Pro Met Tyr Arg Leu Lys Arg Leu Lys Asn Asn Asp Ala Val Arg
                245                 250                 255

Arg Ser Arg Asp Lys Ala Arg Gln Gln Gln Met Ala Lys Glu His Arg
            260                 265                 270

Leu Leu Phe Leu Glu His Glu Thr Arg Glu Gln Ala Val Leu Ile Gln
        275                 280                 285

Glu Leu Lys Gly Gln Asn Ala Glu Leu Lys Asp Glu Phe Gln Lys Ile
    290                 295                 300

Arg Gln Asn Cys Arg Cys Gly Ala Ile Asn Leu Ser Glu Gln Gln Arg
305                 310                 315                 320

His

<210>  17
<211>  129
<212>  PRT
<213>  Meloidogyne hapla

<400>  17

Met Pro Ala Pro Ala Pro Thr Met Cys Cys Cys Gly Gly Gly Gly Gly
1               5                   10                  15

Gly Gly Cys Gly Arg Lys Lys Arg Ala Val Gln Pro His Leu Lys Ser
            20                  25                  30

Phe Asp Val Pro Cys Pro Gln Thr Met Ile Lys Ser Val Lys Lys Gln
        35                  40                  45

Thr Ser Pro Ser Phe Ile Gln Ser Ala Met His Ser Arg Phe Pro Asp
    50                  55                  60

Gln Pro Phe Ile Val Thr Cys Val Thr Leu Gln Ser Leu Ser Ala Tyr
65                  70                  75                  80

Asn Gly Lys Ser Ile Ala Ile Ser Gly His Asp Leu His Lys Thr Glu
                85                  90                  95

Leu Lys Ile Asp Glu Met Val Asn Phe Ser Ser Ser Gly Asp Gly Tyr
            100                 105                 110

Cys Asn Val Ile Ala Asp Glu Gln Trp Cys Gln Val Val Ala Leu Ser
        115                 120                 125

Ala

<210>  18
<211>  154
<212>  PRT
<213>  Meloidogyne hapla

<400>  18

Met Ser Ser Ser Ile Asn Asn Gln His Lys Ile Ile Leu Leu Ile Ala
1               5                   10                  15

Leu Cys Ser Ile Ser Leu Val Glu Ser Ala Tyr Thr Tyr Gln Lys Leu
            20                  25                  30

Asn Glu Leu Met Pro Glu Leu Arg Gln Phe Leu Gly Met Thr His Leu

35                  40                  45

Gly Thr Leu Arg Pro Leu Asn Ser Pro Lys Ala Tyr Gln Gln Pro Ile
    50                  55                  60

His Leu Ser Ala Tyr Ala Ile Pro Ser Phe Ala Asp Glu Gly Gln Phe
65                  70                  75                  80

Gln Leu Arg Val Arg Lys Ser Asn Asn Leu Arg Asn Leu Met Arg Ile
                85                  90                  95

Gly Lys Arg Arg Leu Asp Ser His Glu Phe Lys Gly Asn Ala Ala Phe
            100                 105                 110

Ala Pro Asn Gly Asp Gly Ser Glu Pro Ser Gly Gly Gly Asn Ile Tyr
        115                 120                 125

Tyr Tyr Ser Pro Ser Phe Pro Ile Asp Asn Gln Leu Pro Phe Ile Lys
    130                 135                 140

Pro Tyr Arg Pro Gln Ser Ile Trp Leu Lys
145                 150

<210>  19
<211>  341
<212>  PRT
<213>  Meloidogyne hapla

<400>  19

Met Asp Asp Ile Leu Asn Tyr Phe Tyr Cys Phe Ile Arg Thr Cys Phe
1               5                   10                  15

Pro Val Cys Phe Asp Thr Cys Glu Ala Thr Cys Asp Lys His His Gln
            20                  25                  30

Ile Lys Thr Pro Gln Asn Asn Gln Arg Arg Leu Cys Ala Gln Thr Cys
        35                  40                  45

His Phe Asp Cys Gln Arg Ser Cys Ala Glu Lys Gln Gln Gln Lys Gln
    50                  55                  60

Ala Arg Arg Arg Leu Gly Gly Thr Glu Leu Phe Ser Leu Gly Thr Ser
65                  70                  75                  80

Ser Pro Ser Pro Thr Ile Thr Ser Ala Ala Val Thr Glu Asn Asn Asn
                85                  90                  95

Glu Met Asn Asn Asn Leu Gln Gln Gln Gln Thr Val Ile Asn Gly Pro

            100                 105                 110

Ala Thr Asn Cys Val Ala Arg Cys Pro Lys Ser Cys Val Val Ser Cys
        115                 120                 125

Leu Ser Thr Gln Thr Asn Glu Asp Pro Thr Ile Cys Val Pro Ala Cys
    130                 135                 140

Val Ala Val Cys Ala Gln Ser Cys Ser Arg Gly Leu Gln His Phe Asp
145                 150                 155                 160

Ser Leu Pro Gln Gln Asn Lys Ala Glu Gln Ile Gln Gln Gln Ile Gln
                165                 170                 175

Gln Ile Leu Leu Gln Gly Gln Arg Asn Glu Gly Leu Glu Asn Gly Thr
            180                 185                 190

Ser Tyr Ser Asn Val Thr Val Gln Ser Glu Asn Asn Gly Lys Lys Gln
        195                 200                 205

Met Leu Val Thr Gln Pro Thr Met Phe Leu Asn Asp Val Asp Arg Glu
    210                 215                 220

Leu Asn Asp Gly Leu Ile Arg Ser Arg Pro Cys Ala Pro Leu Cys Arg
225                 230                 235                 240

Pro Gln Cys Gln Arg Glu Cys Thr Leu Lys Tyr Val Glu Glu Arg His
                245                 250                 255

Ser Pro Phe Ser Thr Tyr Ser Gly Arg Lys Arg Gly Phe Asn Asn Gln
            260                 265                 270

Arg Gly Leu Asn Ile Gly Asn Trp Glu Lys Arg Leu Asn Gln Arg Asn
        275                 280                 285

Asn Lys Ile Phe Pro Pro His Tyr Ile Leu Pro Leu Asn Glu Ala Leu
    290                 295                 300

Lys Glu Gly Lys Asn Glu Glu Asn Lys Ile Asn Asp Tyr Asp Glu Lys
305                 310                 315                 320

Ser Thr Glu Ile Ile Ile Gln Gln Gln Asn Gln Thr Glu Asn Asn Asn
                325                 330                 335

Thr Asn Asn Leu Gly
            340

<210>  20
<211>  195
<212>  PRT
<213>  Meloidogyne hapla

<400>  20

Met Phe Pro Asn Trp Ala Leu Leu Leu Pro Leu Phe Gly Leu Val Ala
1               5                   10                  15

Phe Cys Arg Ala Gln Asn Ser Asn Asn Gln Cys Asn Pro Phe Ile Cys
            20                  25                  30

Gly Asn Asp Trp Gln Asn Arg Thr Glu Pro Asn Asp Gly Lys Val Tyr
        35                  40                  45

Gly Tyr Lys Val Phe Leu Lys Ser Ala Leu Asn Phe Phe Glu Ala Gln
    50                  55                  60

Arg Ile Cys Arg Asp Gln Cys Ala Glu Val Ala Ser Val Leu Asn Asp
65                  70                  75                  80

Asp Glu Tyr Asp Phe Val Leu Gln Val Ala Gly Asp Leu Leu Glu Gln
                85                  90                  95

Cys Gln Thr Asn Ala Ser Val Cys Pro Ile Arg Asp Pro Asn Pro Glu
            100                 105                 110

Phe Asn Arg Ile Leu Asn Gly Phe Trp Leu Gly Met His Arg Val Gln
        115                 120                 125

Gln Tyr Thr Pro Tyr Phe Ala Asn Gly Pro Val Asp Ser Glu Ile Val
    130                 135                 140

Cys Met Asn Ser Asp Arg Thr Asn Cys Ser Phe Gly Thr Tyr Asn Gly
145                 150                 155                 160

Thr Ile Gly Glu His Glu Asp Ile Gly Leu Pro Trp Val Thr Ala Thr
                165                 170                 175

Pro Asn Gly Gly Asn Thr Gly Lys Ile Ile Thr Asp Lys Ile Ala Lys
            180                 185                 190

Ile Ile Phe
        195


<210>  21
<211>  792
<212>  PRT
<213>  Meloidogyne hapla

<400>  21

Met Phe Trp Ile Asn Gly Phe Ala Gly Leu Leu Trp Phe Ser Leu Ser
1               5                   10                  15

Ser Ser Ile Val Asn Ser Ser Asn Ser Ser Glu Val Gln Val Gly Phe
            20                  25                  30

Gly Asn His Thr Leu Lys Glu Gly Lys Val Thr Leu Met Asn Gly Val
        35                  40                  45

Lys Ile Val Glu Gly Asn Asp Ile Thr Asn Leu Lys Lys Phe Leu Pro
    50                  55                  60

Ala Cys Val Ala Ser Leu Asp Arg Asp Phe Ile Tyr Val Asp Tyr Asn
65                  70                  75                  80

Glu Gln Ser Glu Glu Val Cys Thr Val Asp Leu Leu Thr Glu Ser Asp
                85                  90                  95

Ser Ile Ile Phe Asn Ala Gln Leu Arg Leu Cys Asp Lys Asn Lys Thr
            100                 105                 110

Lys Asp Leu Asp Gly Tyr Asn Ile Leu Pro Phe Ala Tyr Ser Leu Ser
        115                 120                 125

Asn Glu Glu Ile Asn Met Ile Lys Tyr Gly Pro Leu Phe Gly Lys Ser
    130                 135                 140

Asn Ala Asp Cys Pro Glu Glu Gly Gly Cys Lys Leu Ser Trp Asn Lys
145                 150                 155                 160

Ser Gly Asp Trp Asn Gly Ile Ile Thr Pro Asn Cys Met Pro Trp Thr
                165                 170                 175

Ala Leu Glu Val Glu Trp Leu Cys Lys Asp Ile Ser Val Leu Ala Ala
            180                 185                 190

Thr Val Tyr Phe Ile Gly Glu Ile Gly Phe Val Thr Thr Gly Ser Ile
        195                 200                 205

Gln Leu Ser Asn Leu Thr Thr Gln Val Arg Phe Thr Val Gln Ile Asp
    210                 215                 220

Lys Asn Gly His Val Glu Ser Lys Asp Leu Ala Met Leu Glu Gly Gly
225                 230                 235                 240

Asn Trp Pro Thr Lys Ser Asn Thr Leu His Asn Gly Ile Tyr Phe Lys
                245                 250                 255

Cys Ile Tyr Asn Thr Ser Ile Ile Gln Pro Lys Ala Trp Glu Ile Val
            260                 265                 270

Asn Asn Glu Ala Lys Glu Phe Asn Gly Lys Lys Leu Met Thr Phe His
        275                 280                 285

Leu Leu Pro Lys Ser Ala Ser Arg Ala Arg Asn Gly Thr Glu Leu Thr
    290                 295                 300

Gly Lys Phe Pro Gly Phe Val Ala Gly Lys Tyr Cys Arg Asn Met Ser
305                 310                 315                 320

Ile Val Phe Ser Arg Thr Asp Tyr Lys Leu Leu Met Met Glu Leu Pro
                325                 330                 335

Thr Ile Lys Asn Ile Ser Thr Pro Thr Thr Thr Ala Asp Leu Thr Thr
            340                 345                 350

Gln Pro Thr Thr Ile Gln Glu Ser Glu Asn Ala Gln Thr Glu Thr Leu
        355                 360                 365

Pro Thr Thr Ile Ser Thr Pro Thr Ser Ser Ser Thr Asp Thr Ser Pro
    370                 375                 380

Ser Thr Thr Asp Ile Ser Thr Met Thr Ile Arg Leu Ser Lys Leu Ser
385                 390                 395                 400

Thr Ser Pro Ser Thr Ser Thr Ala Ser Ser Pro Lys Ser Ser Ser Asp
                405                 410                 415

Thr Ser Ser Arg Pro Ala Glu Thr Ile Ser Thr Met Lys Gln Glu Gln
            420                 425                 430

Gln Ser Asp Ser Lys Ser Lys Pro Ser Ser Leu Glu Gln Ala Thr Val
        435                 440                 445

His Thr Thr Ile Gly Gly Ile Ser Leu Thr Glu Glu Asp Gly Ser Leu
    450                 455                 460

Pro Leu Thr Asn Ser Ser Asn Leu Asp Gln Asn Phe Thr Thr Ile Ser
465                 470                 475                 480

Ser Asn Glu Thr Phe Met Asn Val Thr Asn Glu Thr Ile Ala Asp Asn
                485                 490                 495

Ile Thr Ile Thr Tyr Ser Thr Ser Met Pro Thr Ile Ile Leu Ser Pro
        500                 505                 510

Thr Thr Thr Lys Ile Phe Gly Ser Thr Asn Gly Thr Thr Lys Val Ile
    515                 520                 525

Ile Leu Lys Gly Thr Gln Lys Gly Glu Phe Pro Leu His Tyr Tyr Tyr
    530                 535                 540

Ile Ile Phe Gly Gly Ile Val Val Ile Val Leu Leu Leu Phe Val Ile
545                 550                 555                 560

Ile Cys Val Cys Cys Ile Ile Arg Lys Asn Lys Lys Lys Asp Ser Ile
                565                 570                 575

Lys Glu Glu Ser Ser Glu Gly Ser Gly Gly Thr Thr Glu Glu Leu Leu
            580                 585                 590

Ser Ile Glu Ala Lys Lys Glu Gln Ser Lys Trp Met Leu Asp Thr Lys
        595                 600                 605

Met Asp Thr Gln Asp Ser His Ile Ser Glu Lys Lys Thr Glu Lys Lys
    610                 615                 620

Val Ile Val Lys Lys Glu Ile Ile Lys Glu Gly Gly Asn Glu Pro Asn
625                 630                 635                 640

Lys Lys Glu Ile Ser Lys Glu Asn Met Lys Lys Thr Leu Ser Lys Asp
                645                 650                 655

Val Gly Val Val Val Pro Asn Thr Lys Glu Ser Lys Glu Leu Ile Glu
            660                 665                 670

Lys Glu Thr Thr Glu Glu Glu Glu Ile Leu Leu Lys Pro Leu Glu Pro
        675                 680                 685

Lys Ser Lys Ala Thr Thr Pro Asp Pro Gln Ile Ser Thr Phe Ser Ala
    690                 695                 700

Pro Tyr Glu Glu Asp Arg Lys Arg Met Thr Val Pro Leu Lys Asp Asn
705                 710                 715                 720

Ser Val Pro Asn Thr Glu Ser Lys Lys Ser Met Glu Ser Ile Val Leu
                725                 730                 735

Ile Glu Lys Lys Glu Thr Lys Ala Asp Leu Thr Val Ser Asp Glu Ile
            740                 745                 750

Ile Gly Asp Asn Asn Lys Lys Lys Glu Glu Glu Gly Asp Asp Asn Lys
        755                 760                 765

Lys Glu Glu Gly Gly Asp Asp Asn Asn Lys Lys Glu Glu Ser Glu Asp
    770                 775                 780

Asn Lys Glu Val Asp Ala Tyr Glu
785                 790

<210>  22
<211>  252
<212>  PRT
<213>  Meloidogyne hapla

<400>  22

Met Ile Leu Leu Ile Leu Thr Ala Ile Val Trp Gln Ile Asn Ala Gln
1               5                   10                  15

Gln Gln Pro Pro Tyr Phe Pro Pro Gln Asn Ile Asn Gly Thr Phe Gly
            20                  25                  30

Leu Gln Leu Asn Ser Tyr Pro Ser Pro Gln His Leu Leu Gln Tyr Gln
        35                  40                  45

Arg Gln Leu Gln Pro Ser Asp Tyr Arg Gln Gln Gln Tyr Ala Pro Asp
    50                  55                  60

Gly His Tyr Leu His Gln Gln Pro Gln Phe Asn Asn His Tyr Pro Asn
65                  70                  75                  80

Ser Phe Gln Thr Gln Asn Lys Trp Gln Asn Asp Gln Lys Leu Glu Asp
                85                  90                  95

Glu Asp Ser Lys Glu Glu Asn Asn Asn Asn Phe Leu Ser Gln Asn Asn
            100                 105                 110

Asn Asn Arg Gln Tyr Pro Gln Arg Val His Phe Asp Pro Asn Asn Asn
        115                 120                 125

Leu Pro Tyr Leu His Asp Arg Gln Gln Gln Gln Arg Tyr Asn Gln Met
    130                 135                 140

Phe Asn Gly Gly Tyr Asn His Leu Thr Gly Gln Lys Asn Pro Ser Ser
145                 150                 155                 160

Phe Asp Phe Glu Asn Glu Ala Asp Pro Ser Asn Ser Pro Tyr Asn Tyr
                165                 170                 175

Gly Asn Gln Gln Arg Pro Val His Pro Asn Met Gln Thr His Gln Tyr
        180                 185                 190

Asn Gly Gln Gln His Pro Ala Gly Pro Leu Asn Pro His Tyr Arg Thr
        195                 200                 205

Gly Ile Val Tyr Pro Glu Gly Gln Arg Asn Leu Asn Asn His Lys Ser
    210                 215                 220

Phe Trp Phe Asn Asp Ala Lys Thr Asn Asn Lys Ile Asn Asn Phe Trp
225                 230                 235                 240

Leu Leu Thr Ile Phe Gly Ile Leu Ile Leu Tyr Leu
                245                 250


<210>  23
<211>  225
<212>  PRT
<213>  Meloidogyne hapla

<400>  23

Met Pro Gln Asn Met Tyr Lys Ile Ile Asn Leu Phe Phe Pro Ala Leu
1               5                   10                  15

Phe Thr Asn Trp Val Thr Asp Glu Lys Tyr Leu Trp Pro Ala Gln Pro
            20                  25                  30

Ser Thr Thr Pro Gly Val Arg Leu Gly Pro Leu Ile Asn Tyr Thr Leu
        35                  40                  45

Asp Trp Glu Lys Ile Val Glu Pro Pro Ser Phe Val Trp Leu Lys Asn
    50                  55                  60

Ile Leu Asp Ala His Lys Asn Ile Glu Glu Asn Thr Cys Ile Arg Phe
65                  70                  75                  80

Ala Asn Ser Ser Glu Gly Asp His Ile His Tyr Tyr Trp Gly Tyr Gly
                85                  90                  95

Cys His Ser Leu Val Gly Arg Asn Gly Gly Lys Gln Trp Leu Ser Leu
            100                 105                 110

Gly Pro Gly Cys Trp Thr Val Asn Ala Val His Glu Thr Leu His Ala
        115                 120                 125

Leu Gly Ser Asn His Glu Gln Ser Arg Gln Asp Arg Asp Thr Phe Ile
    130                 135                 140

Lys Ile Asn Glu Gly Asn Met Ile Glu Asn Ile Ser Asp Ala Phe Ala
145                 150                 155                 160

Ile Ile Asp Gly Ser Pro Thr Gly Asn Pro Tyr Asp Phe Gly Ser Cys
                165                 170                 175

Met His Tyr Thr Asn Gly Ala Phe Ser Lys Asn Lys Lys Pro Thr Ile
            180                 185                 190

Glu Thr Leu Asp Lys Asn Tyr Gln His Thr Ile Gly Tyr Arg Leu Arg
        195                 200                 205

Leu Ser Phe Leu Asp Thr Lys Ala Leu Asn His Arg Tyr Cys Glu Arg
    210                 215                 220

Arg
225

<210>  24
<211>  163
<212>  PRT
<213>  Meloidogyne hapla

<400>  24

Met Pro Ser Lys Phe Ile Gln Asn Asn Phe Ser Leu Phe Gln Thr Thr
1               5                   10                  15

Leu Ile Leu Ser Leu Leu Ile Ser Pro Ser Gln Ser Val Phe Tyr Arg
            20                  25                  30

Lys Ile Pro Phe Arg Phe Lys Ser Val Val Glu Lys Pro Trp Thr Asn
        35                  40                  45

Thr Glu Asp Asp Asn Ile Ala Ala Ile Ile Ser Ser Arg Glu Asp Lys
    50                  55                  60

Asn Glu Asp Glu Gly Glu Glu Asn Ile Ala Gln Leu Leu Glu Gln Ile
65                  70                  75                  80

Glu Lys Ser Ser Leu Asp Ser Pro Asp Asn Glu Ile Glu Glu Asp Tyr
                85                  90                  95

Gln Tyr Asn Asn Leu Lys Glu Asn Lys Gln Lys Tyr Ile Pro Leu Asp
            100                 105                 110

Gln Thr Leu Gln Lys Ile Asn Asn Asn Leu Ile Lys Gln Arg Ile Asp
        115                 120                 125

Lys Asn Gly Glu Glu Ile Ile Pro Lys Asn Ile Leu Ile Glu Lys Glu
    130                 135                 140

Gly Thr Leu Pro Thr Tyr Thr His Leu Met Ala Phe Arg Pro Asp Tyr
145                 150                 155                 160

Thr Ile Arg

<210>  25
<211>  263
<212>  PRT
<213>  Meloidogyne hapla

<400>  25

Met Ala Ala Leu Leu Gly Val Val Thr Thr Glu Asn Ser Thr Thr Thr
1               5                   10                  15

Ile Ser Pro Val Thr Asn Gly Thr Thr Ile Gly Asn Ile Pro Ser Asn
            20                  25                  30

Ser Pro Pro Pro Pro Asn Pro Glu Gln Thr Thr Ser Thr Lys Ser Val
        35                  40                  45

Gln Glu Thr Lys Val Asp Lys Ser Lys Gln Lys Ser Pro Tyr Glu Cys
    50                  55                  60

Trp Ile Thr Arg Arg Leu Tyr Asp Asn Ser Ile Ala Ala Lys Gly Asp
65                  70                  75                  80

Glu Phe Lys Lys Arg Ile Cys Ala Glu Pro Gly Cys Phe Tyr Ile Tyr
                85                  90                  95

Glu Gln Glu Asn Leu Gly Trp Asp Phe Pro Glu Phe Leu Val Glu Asn
            100                 105                 110

Lys Lys Arg Gly Lys Arg Leu Tyr Leu Thr Gly Cys Arg His Asp Ile
        115                 120                 125

Pro Tyr Leu Leu Glu Arg Ser Lys Gly Lys Glu Arg Ile His Arg Ala
    130                 135                 140

Ile Arg Ala Leu Arg Ala Cys Tyr Val Gly Thr Arg Met Asp Ala Gly
145                 150                 155                 160

Glu Thr Lys Tyr Cys Gln Pro Glu Tyr Glu Gly Glu Gly Gln Ile Ser
                165                 170                 175

Leu Asn Gln Gly Met Asp Lys Val Ile Arg Lys Asp Asn Phe Arg Asp
                180                 185                 190

Thr Trp Ala Gly Arg Phe Ile Pro Ser Thr Gln Asn Arg Cys Phe Ile
            195                 200                 205

Glu Phe Leu Tyr Gly Val Phe Tyr Val Asn Asp Asn Gly Lys Gly Leu
    210                 215                 220

Val Val Cys Cys Ser Asn Gly Thr Ser Thr Thr Thr Glu Thr Asp Leu
225                 230                 235                 240

Phe Lys Ser His Cys Gln Thr Val Thr Val Gly Ile Asp Ser Leu Asp
                245                 250                 255

Trp Arg Lys Asp Val Asp Asn
                260

<210>  26
<211>  378
<212>  PRT
<213>  Meloidogyne hapla

<400>  26

Met Arg Leu Asp Ile Phe Leu Val Ile Ala Phe Ser Leu Gly Val Ala
1               5                   10                  15

Val Asn Cys Gly Val Val Lys Arg Ser Gln Asn Ser Tyr Gly Asp Glu
            20                  25                  30

Ala Gly Ala Gly Gly Ala Ala Gly Ala Ala Pro Ala Ala Ala Ala Ala
        35                  40                  45

Pro Ala Gly Gly Glu Ala Ala Ala Pro Ser Gly Gly Glu Thr Gly Gly
    50                  55                  60

Ala Ala Ala Pro Ala Glu Ala Ala Pro Ala Ala Ala Pro Ala Pro Glu
65                  70                  75                  80

Ala Ala Pro Ala Ala Ala Pro Ala Pro Glu Gln Ala Ala Pro Ala Val
                85                  90                  95

Ala Gln Pro Ala Pro Ala Ala Ala Pro Ser Gln Ala Ser Gly Tyr Arg
            100                 105                 110

Lys Lys Arg Ser Gln Asn Ser Tyr Gly Asp Glu Ala Gly His Ala Ala
        115                 120                 125

Gly Ala Ala Pro Ala Glu Ala Ala Ala Pro Ala Ala Gly Gly Glu Ala
    130                 135                 140

Ala Ala Pro Ser Gly Gly Glu Thr Gly Gly Ala Ala Ala Pro Ala Glu
145                 150                 155                 160

Thr Ala Pro Ala Ala Ala Pro Glu Ala Ala Pro Ala Ala Ala Pro Ala
                165                 170                 175

Pro Glu Gln Ala Ala Pro Ala Val Ala Gln Pro Ala Pro Ala Ala Ala
            180                 185                 190

Pro Ser Gln Ala Ser Gly Tyr Arg Lys Lys Arg Ser Gln Asn Ser Tyr
        195                 200                 205

Gly Asp Glu Ala Gly Ala Gly Gly Ala Ala Gly Ala Ala Pro Ala Ala
    210                 215                 220

Ala Ala Ala Pro Ala Gly Gly Glu Ala Ala Ala Pro Ala Ala Gly Gly
225                 230                 235                 240

Cys Thr Glu Gly Cys Ala Ala Gly Gly Glu Ser Ala Ala Pro Ala Ala
                245                 250                 255

Ala Pro Ala Pro Glu Ala Ala Pro Ala Ala Ala Pro Ala Pro Glu Gln
            260                 265                 270

Ala Ala Pro Ala Val Ala Gln Pro Ala Pro Ala Ala Ala Pro Ser Gln
        275                 280                 285

Ala Ser Gly Tyr Arg Lys Lys Arg Ser Gln Asn Ser Tyr Gly Asp Glu
    290                 295                 300

Ala Gly His Ala Ala Gly Ala Ala Pro Ala Glu Ser Ala Ala Pro Ala
305                 310                 315                 320

Ala Gly Gly Glu Ala Ala Ala Pro Ser Gly Gly Glu Thr Gly Gly Ala
                325                 330                 335

Ala Ala Pro Ala Glu Thr Ala Pro Ala Ala Ala Pro Glu Ala Ala Pro
            340                 345                 350

Ala Ala Ala Pro Glu Gln Ala Ala Pro Ala Val Ala Gln Pro Ala Pro
        355                 360                 365

Ala Ala Ala Pro Ser Gln Ala Ser Gly Tyr

370                 375

<210>  27
<211>  300
<212>  PRT
<213>  Meloidogyne hapla

<400>  27

Met Pro Val Leu Ser Phe Val Ala Leu Leu Ala Phe Leu Leu Pro Ala
1               5                   10                  15

Val Leu Leu Ile Gln Cys Cys Lys Lys Lys Ala Lys Asp Ala Ala Ala
            20                  25                  30

Ala Asp Gly Gly Val Lys Thr Pro Leu Asp Ala Gly Lys Ser Lys Ala
        35                  40                  45

Lys Gly Ser Gly Gly Lys Lys Pro Ile Val Pro Glu Gly Ala Lys Lys
    50                  55                  60

Glu Lys Lys Ser Glu Lys Pro Val Gln Val Pro Thr Ser Thr Ala Ala
65                  70                  75                  80

Gly Pro Ser Leu Ala Thr Ala Phe Ser Pro Glu Lys Thr Lys Ser Lys
                85                  90                  95

Thr Asn Ile Lys Gln Gly Ala Ser Thr Lys Thr Ala Ala Val Lys Thr
            100                 105                 110

Lys Ala Gly Ala Ile Thr Ala Leu Pro Lys Gly Gln Ser Ala Lys Lys
        115                 120                 125

Asn Glu Val Thr Ala Val Ala Leu Glu Lys Thr Lys Asp Glu Lys Thr
    130                 135                 140

Lys Leu Ile Asn Ala Gly Ile Glu Lys Ala Lys Ala Ala Lys Pro Glu
145                 150                 155                 160

Asn Pro Val Ile Glu Pro Ser Lys Phe Asp Asp Gly Thr Pro Thr Val
                165                 170                 175

Ile Phe Thr Pro Ala Asp Met Met Ser Val Ala Ala Met Thr Ala Asn
            180                 185                 190

Pro Asn Ser Thr Glu Thr Ile Asp Asp Lys Ser Lys Val Gly Gln Lys
        195                 200                 205

Thr Ser Ala Ser Phe Tyr Asp Leu Asp Ser Thr Asn Lys Thr Ser Asp

210                 215                 220

Ser Val Tyr Glu Asn Val Asp Leu Pro Lys Thr Lys Ser Thr Val Glu
225                 230                 235                 240

Met Glu Gly Asp Lys Gly Asp Gly Lys Asp Glu Asp Asp Asp Asp Thr
                245                 250                 255

Glu Lys Ser Ile Met Lys Ser Lys Ile Ala Asp Glu Lys Lys Ala Glu
            260                 265                 270

Glu Gln Lys Lys Ser Met Ala Ala Ser Asp Glu Tyr Gln Thr Leu Ala
        275                 280                 285

Asn Met Ser Thr Asp Lys Val Phe Gln Lys Lys Gly
    290                 295                 300

<210>  28
<211>  415
<212>  PRT
<213>  Meloidogyne hapla

<400>  28

Met Ile Pro Ile Thr Ser Phe Phe Phe Phe Phe Leu Ser Leu Leu Phe
1               5                   10                  15

Leu Phe Asp Phe Ser Asn Ser Asn Cys Ile Glu Tyr Cys Phe Val Pro
            20                  25                  30

Pro Gln Gln Tyr Ser Gly Cys Asn Val Cys Gly Gly Tyr Gly Tyr Gly
        35                  40                  45

Gly Gly Tyr Ile Thr Gly Gly Tyr Gly Gly Tyr Val Pro Gln Gln Val
    50                  55                  60

Gln Gln Gln Tyr Val Pro Gln Gln Gln Gln Tyr Ile Pro Gln Gln Gln
65                  70                  75                  80

Gln Tyr Val Pro Gln Gln Gln Val Val Gln Pro Ala Gly Gly Gly Val
                85                  90                  95

Gly Gly Ser Gly Tyr Pro Ser Gly Gln Gly Tyr Ile Pro Gln Ser Gly
            100                 105                 110

Gly Gly Ile Pro Gln Thr Thr Cys Gly Gly His Asn Cys Gly Gly Gly
        115                 120                 125

Ser Gly Val Gly Gly Gly Thr Thr Gly Asn Gly Gly Gly Gly Asn Val

130                 135                 140

Gly Gly Thr Thr Gly Gly Ser Gly Cys Gly Ser Gly Gly Cys Ser Gly
145                 150                 155                 160

Gly Asn Gly Gly Thr Thr Gly Gly Thr Gly Gly Gly Ser Tyr Gly Gly
                165                 170                 175

Gly Glu Asn Thr Gly Gly Thr Ser Gly Ser Gly Leu Gly Gly Ser Ser
            180                 185                 190

Gly Gly Ala Gly Gly Gly Glu Ser Thr Gly Thr Glu Ser Asn Thr Gly
        195                 200                 205

Gly Thr Ser Thr Gly Gly Glu Glu Ser Thr Gly Gly Thr Ser Gly Gly
    210                 215                 220

Ser Asn Gly Gly Ser Glu Gly Gly Tyr Gly Gly Ser Ala Gly Gly Thr
225                 230                 235                 240

Gly Thr Glu Thr Ala Ser Gly Ser Leu Gly Thr Gly Gly Ser Gly Cys
                245                 250                 255

Gly Ser Gly Gly Cys Ser Ser Ser Thr Gly Gln Gly Ser Ser Thr Gly
            260                 265                 270

Gly Ser Gly Tyr Ser Gly Ala Gln Thr Thr Gly Tyr Gln Pro Ala Ala
        275                 280                 285

Thr Ala Thr Gly Gly Ser Thr Gln Val Ala Thr Gly Gly Asn Gln Gln
    290                 295                 300

Cys Cys Thr Thr Gly Ser Thr Gly Thr Val Val Gln Pro Val Gln Pro
305                 310                 315                 320

Gln Ser Ile Ala Thr Gly Gln Ser Gln Lys Gly Tyr Gly Asp Val Arg
                325                 330                 335

Arg Lys Lys Leu Ser Ser Arg His Arg Gln Arg Gln Ile Phe Gly Ile
            340                 345                 350

Ser Pro Arg Gly Thr Met Gly Asn His Met Ala Met Leu Ser Gln Leu
        355                 360                 365

Pro Pro Ile Pro Pro Val Lys Arg Glu Lys Ile Asn Ser Asn Ser Asn
    370                 375                 380

Leu Lys Asn Gly Leu Phe Thr Lys Thr Pro Val Asp Pro Met Lys Ser
385                 390                 395                 400

Lys Ala Gln Ser Phe Glu Asn Arg Ala Ala Lys Leu Lys His Phe
                405                 410                 415

<210>  29
<211>  100
<212>  PRT
<213>  Meloidogyne hapla

<400>  29

Met Gly Ser Ala Ile Ser His Tyr Gly Asn Asp Met His Ile Asp Leu
1               5                   10                  15

Asp Ser Thr Gly Leu Lys Thr Ser Ser Ile Ala Pro Asn Gly Ile Gln
            20                  25                  30

Ile Gly Pro Arg Gln His Lys Lys Ile Lys Cys Ala Leu Gly Ile Thr
        35                  40                  45

Glu Thr Lys Ile Leu Asp Phe Asp Leu Phe Ile Pro His Gly Ala Ser
    50                  55                  60

Lys Cys Ser Asn Leu Ala Val Lys Phe Gly Pro Glu Tyr Lys Leu Leu
65                  70                  75                  80

Gly Ser Glu Glu Arg Glu Glu Lys Lys Ile Thr Asp Leu Asp Gln Glu
                85                  90                  95

Ala Asn Thr Asp
            100

<210>  30
<211>  44
<212>  PRT
<213>  Meloidogyne hapla

<400>  30

Thr Lys Tyr Ile Asp Cys Gln Cys Ala Lys Asn Leu Cys Asn Tyr Pro
1               5                   10                  15

Cys Lys Ser Ser Lys Ser Ala Cys Asp Gly Cys Lys Asn Thr Asp Val
            20                  25                  30

Pro Lys Gln Cys Thr Ile Asn Gln Cys Thr Ile Pro
        35                  40

<210>  31

<211>  391
<212>  PRT
<213>  Meloidogyne hapla

<400>  31

Met Thr Asp Glu Asn Ala Glu Asn Pro Gln Asp Glu Asn Glu Glu Asn
1               5                   10                  15

Asn Gly Val Asp Gly Glu Asp Gln Val Ile Asp Lys Asn Lys Pro Gly
            20                  25                  30

Lys Ser Gly Leu Pro Gly Lys Asp Ala Lys Ser Gly Gln Gln Asp Lys
        35                  40                  45

Asn Lys Lys Asp Glu Lys Val Gln Lys Gly Gly Ile Glu Gln Lys Gly
    50                  55                  60

Thr Lys Thr Gly Thr Asn Asp Gly Leu Asn Thr Glu Lys Asn Thr Asn
65                  70                  75                  80

Gln Pro Gly Gly Gln Gly Thr Gly Gln Ile Gly Gln Gln Gln Gly Gln
                85                  90                  95

Gly Ile Gly Gln Pro Asn Val Asn Pro Asn Ser Asn Ile Pro Tyr Ser
            100                 105                 110

Pro Thr Val Tyr Pro Asn Gly Leu Pro Gly Gln Ser Gln Gln Thr Asn
        115                 120                 125

Ile Asn Pro Thr Asn Leu Asn Pro Phe Asn Pro Ser Pro Val Asn Pro
    130                 135                 140

Ile Pro Asn Pro Val Tyr Pro Gln Gln Thr Asn Asn Pro Tyr Pro Thr
145                 150                 155                 160

Asn Pro Pro Pro Ile Tyr Pro Gln Tyr Pro Pro Ala Asn Gln Gln Gln
                165                 170                 175

Pro Tyr Ser Pro Pro Ser Gly Phe Gly Ala Ser Asn Ser Leu Asn Asp
            180                 185                 190

Ile Asp Asp Asp Pro Phe Gly Phe Gly Ile Thr Leu Gln Arg Gln Gln
        195                 200                 205

Gln Gln Gln Tyr Ser Pro Tyr Ser Gln Tyr Ala Gln Gln Asn Gln Asn
    210                 215                 220

Ile Pro Gln Gln Tyr Asn Pro Pro Asn Asn Phe Tyr Ser Asn Asn Leu

225                 230                 235                 240

Gly Gly Phe Gly Asn Thr Gly Asn Arg Val Ala Gly Gln Thr Asn Val
                245                 250                 255

Pro Pro Glu Gln Arg Ser Gln Leu Pro Met Ile Val Ser Gly Ser Asn
            260                 265                 270

Ser Asn Asn Gly Leu Gln Arg Asp Ala Ser Phe Gly Arg Gly Arg Arg
        275                 280                 285

Ser Asp Asp Tyr Gln Gln Gln Gln Pro Ala Gln Gln Gln Gln Gln Met
    290                 295                 300

Pro Ala Met Pro Thr Val Cys Phe Arg Pro Ser Pro Glu Thr His Asp
305                 310                 315                 320

Ile Ala Ser Trp Phe Gln Asn Ile Thr Asn Glu Ile Asn Ser Asn Pro
                325                 330                 335

Gln Lys Phe Gln Pro Val Gln Asp Asn Gly Leu Ser Asn Ile Leu Gln
            340                 345                 350

Gln Leu Ala Leu Asn Gly Gly Ile Pro Glu Ile Cys Thr Lys Ile Gln
        355                 360                 365

Gln Asn Ile Pro Thr Asn Gly Phe Gln Gln Gly Ile Leu Val Arg Lys
    370                 375                 380

Lys Arg Ser Asn Gly Arg Gly
385                 390

<210>  32
<211>  343
<212>  PRT
<213>  Meloidogyne hapla

<400>  32
   Met Ser His Ser Tyr Ser Asn His Gln Arg Met Ala Thr Arg Pro Pro
   1               5                   10                  15
   Asn Ser Leu Thr Ile Cys Asn Leu Asp Gly Asn Thr Met Gln Asn Val
               20                  25                  30
   Gly Gly Phe Val Asn Asn Asn Gly Ala His Ser Trp Gln Thr Glu Gln
           35                  40                  45
   Thr Ile Asn Leu Glu Thr Ile Asn Leu Asp Glu Phe Asp Asn Glu His
   50                  55                  60
   Phe Asn Leu Leu Asn Phe Ile Asn Asp Gly Ser Ser Pro Leu Glu Glu
   65                  70                  75                  80
   Ser Val Pro Arg Leu Asp Asp Asp Glu Val Gln Lys Gln Lys Ala Glu
               85                  90                  95
   Ala Leu Tyr Ala Gln Tyr Gln Tyr Asp Gln Met Met Phe Gly Asn Pro
               100                 105                 110
   Gln Leu Gln His Arg Asn Gly Leu Gly His Leu Leu Asp Ser Pro Asp
           115                 120                 125
   His Leu Met Asp Ile Tyr Leu Ser Leu Asp Phe Gly Pro Asn Glu Phe
       130                 135                 140
   Met Asn Ser Ser Val Pro Ser Ser Cys Phe Gly Glu Val Glu Gly Gly
   145                 150                 155                 160
   Pro Asn Arg Asp Glu Glu Glu Glu Glu Val Arg Arg Arg Leu Phe Glu
                   165                 170                 175
   Pro Pro Ile Asn Ser Ser Leu Val Pro Ser Thr Ser Asn Ile Ser Met
               180                 185                 190
   Gln Lys Leu Leu Arg Val Pro Val Val Lys Glu Glu Pro Phe Lys Asp
           195                 200                 205
   Glu Pro Leu Glu His Asp Gly Val Asn Val Ser Gln Val Phe Gly Ser
       210                 215                 220
   Ser Ser Ser Arg Ala Ser Ser Val Ala Ser Ser Val Phe Thr Ser Val
   225                 230                 235                 240
   Leu Ser Ala Ser His Ser Gly Thr Pro Arg Lys Tyr Arg Ile Lys Ser
                   245                 250                 255
   Asp Lys Glu Lys Ser Asn Pro Met Tyr Arg Leu Lys Arg Leu Lys Asn
               260                 265                 270
   Asn Asp Ala Val Arg Arg Ser Arg Asp Lys Ala Arg Gln Gln Gln Met
           275                 280                 285
   Ala Lys Glu His Arg Leu Leu Phe Leu Glu His Glu Thr Arg Glu Gln
       290                 295                 300
   Ala Val Leu Ile Gln Glu Leu Lys Gly Gln Asn Ala Glu Leu Lys Asp
   305                 310                 315                 320
   Glu Phe Gln Lys Ile Arg Gln Asn Cys Arg Cys Gly Ala Ala Ala Ser
                   325                 330                 335
   Leu Asn Asp Gln Gln Arg His
               340

<210>  33
<211>  177
<212>  PRT
<213>  Globodera pallida

<400>  33

Met His Phe Cys Leu Leu Ser Gly Gln Leu Leu Leu Cys Cys Thr Leu
1               5                   10                  15

Leu Met Leu Leu Asn Val Glu Pro Thr Leu Glu Cys Gly Phe Pro Met
            20                  25                  30

Pro Gln Pro Thr Val Val Cys Cys Gly Gly Gly Cys Gly Gly Gly Gly
        35                  40                  45

Gly Gly Tyr Gly Gly Gly Gly Gly Gly Cys Cys Gly Arg Arg Lys Arg
    50                  55                  60

Glu Ala Val Gln Pro Leu Tyr Lys Asp Arg Ser Val Pro Cys Pro Gln
65                  70                  75                  80

Thr Glu Trp Arg Glu Ile Ile Lys Gln Ser Ile Leu Ser Phe Gly Ile
                85                  90                  95

Ala Arg Glu Asn Ala Asn Ala Pro Thr Met Ala Ile Gln Thr Ala Leu
            100                 105                 110

Ser Ser Arg Phe Pro Asp Asn Lys Phe Leu Val Ile Cys Ser Met Leu
        115                 120                 125

Asp Glu Lys His Val Glu Glu Lys Gln Ser Gly Ser Asp Val Ala Glu
    130                 135                 140

Gly Gln Met Pro Leu Leu Met His Met Ser Ser Ala Gly Asp Gly Tyr
145                 150                 155                 160

Cys Asn Val Met Glu Ser Arg Leu Trp Cys Gln Ala Val Ala Met Ser
                165                 170                 175

Gly

<210>  34
<211>  123
<212>  PRT
<213>  Globodera pallida

<400>  34

Met Leu Leu Asn Leu Leu Ala Val Tyr Ala Val Ala Leu Met Thr Thr
1               5                   10                  15

Leu Tyr Met Pro Val Gln Ser Ala Tyr Thr Tyr Gln Lys Met Asp Glu
            20                  25                  30

Leu Met Pro Glu Leu Arg Gln Tyr Leu Gly Arg Thr His Leu Gly Thr
        35                  40                  45

Leu Lys Ala Arg Lys Phe Asn Asp Asp Asp Asn Ala Phe Gln Gln Ile
    50                  55                  60

Arg Glu Arg Lys Ser Asn Asn Leu Arg Asn Leu Met Arg Ile Gly Lys
65                  70                  75                  80

Arg Asn Val Pro Ile Ala Asp Asn Phe Ala Arg Glu Asp Glu Gly Gly
                85                  90                  95

Val Gly Ala Leu Ser Pro Val Phe Tyr Ile Pro Thr Asp Phe Leu Gln
            100                 105                 110

Ala Tyr Arg Pro Gln Ser Val Trp Leu Arg Arg
        115                 120

<210>  35
<211>  654
<212>  PRT
<213>  Globodera pallida

<400>  35
   Met Pro Ala Gly Glu Ser Asn Arg Gln Gln Gln Gln Ala Gly Ser Gln
   1               5                   10                  15
   His Gly Phe Gly Thr Tyr Ser Tyr Gly Gly Pro Ala Pro Ile His Ser
               20                  25                  30
   Ala Cys His Pro Ile Cys His Lys Ser Cys Thr Ser Ser Cys Val Ala
           35                  40                  45
   Ser Arg Thr Thr Asp Gln Asn Asn Phe Asn Gly Phe Pro Gln Pro Ala
   50 55 60
   Pro Pro Leu Asn Glu Leu Gln His Pro Gln Gln His Asn Gln Pro Ser
   65 70 75 80
   Thr Met Val Glu Asn Asn Gln Ala Ser Asn Asn Asn Asn Asn Cys Phe
   85 90 95
   Pro Ser Gln Cys Arg Ala Asn Cys Leu Arg Ala Cys Ala Arg Ile Phe
   100 105 110
   Glu Gly Gln Gln Gln Gln Gln Asn Arg Glu Ser Phe Val Ser Thr Asn
   115 120 125
   Gly Ile Asp Asn Asn Asn Val Asp Pro His Tyr Gln Val Glu Thr Gly
   130 135 140
   Gln Asn Lys Gln Asn Pro Asp Tyr Gly Ser Ser Asn Met Met Ser Gly
   145 150 155 160
   Arg Asn Gly Gly Ala Asn Asn Gly Cys Gln Cys Ser Pro Ala Ser Val
   165 170 175
   Cys Glu Cys Gln Glu Thr Cys Tyr Pro Val Cys Ile Asp Thr Cys Glu
   180 185 190
   Ser Asn Cys Leu Gln Gln Gln Ser Gln Gln Thr Pro Ala Ser Gln Arg
   195 200 205
   Ala Arg Cys Ala Gln Phe Cys His Leu Glu Cys Gln His Asp Cys Ala
   210 215 220
   Val Glu Gln Gln Gln Gln Gln Gln Gln Arg Val Pro Thr Arg Lys Ala
   225 230 235 240
   Val Asp Ala Met Glu Thr Ser Cys Ala Arg Gly Ile Gln Phe Asp Ala
   245 250 255
   Ala Gly Leu Thr Gln Gln Met Ala Val Pro Met Pro Thr Met Ala Leu
   260 265 270
   Asp Arg Val Gly Pro Ala Gln Gln Leu Gln Asn Arg Ser Asn Asn Asn
   275 280 285
   Gly Gly Glu Phe Glu Gln His His Gln Gln Met Asn Asp Trp Glu Ile
   290 295 300
   Gly Asp Ser Ala Gln Met Leu Pro Asn Phe Gly Gln Asn Asp Gly Pro
   305                 310                 315                 320
   Asn Met Ala Ala Ile Gly Asn Asp Asp Thr Pro Pro Ser Thr Thr Thr
                   325                 330                 335
   Ala Ala Thr Ile Ile Pro Gln Ala Met Glu Gly Val Arg Gln Glu Gln
               340                 345                 350
   Ala Val Pro Pro Thr Thr Glu Met Lys Pro Cys Ala Pro Leu Cys Gln
           355                 360                 365
   Pro Glu Cys Arg Pro Glu Cys Asn Lys Arg Tyr Met Glu Glu Arg Pro
       370                 375                 380
   Asp Met Glu Arg Ser Gln Gln Gln Gln Gln Gln Gln Gln Tyr Gly Gln
   385                 390                 395                 400
   Met Val Met Ser Asn Asn Gly Ile Ile Ser Arg Gly Glu Asn Val Gly
                   405                 410                 415
   Lys Ser Gly Gly Ala Ala Ser Gly Val Gly Gly Glu Leu Met Arg Leu
               420                 425                 430
   Asp Gln Gln Asn Gln Asn Asp Gln Gln Gln Pro Pro Pro Pro Lys Gln
           435                 440                 445
   Gln Arg Ser Ala Pro Gln Ser Ser Phe Phe Pro Pro His Ile Ile Leu
       450                 455                 460
   Asp Glu Gly Leu Leu Thr Ala Ala Ala Val Ala Pro Pro Ala Asn Asp
   465                 470                 475                 480
   Asp Glu Ser Asn Val Thr Thr Asn Gly Pro Pro Ala Thr Asn Asn Asp
                   485                 490                 495
   Asp Asn Lys Lys Lys Pro Ser Asp Lys Met Ser Trp Leu Arg Pro Gln
               500                 505                 510
   Asn Glu Thr Gly Gly Trp Asp Arg Asn Gly Gly Ala Met Pro Pro Tyr
           515                 520                 525
   Gln Gln Asn Leu Thr Lys Leu Gly Gly Ala Arg Glu Lys Gln Gln Gln
       530                 535                 540
   Gln Gln Ile Ile Lys Asp Gln Gln Gln Glu Gln Gln Arg Gln Lys Val
   545                 550                 555                 560
   Pro Asn Val Lys Gly Gly Gly Lys Gln Arg Val Val His His Arg His
               565                 570                 575
   His Asn Arg Leu Thr Lys Asp Gly Lys Ile Gly Arg Pro Ala Ser Asp
           580                 585                 590
   Phe Phe Arg Arg Arg Arg Ile Leu Phe Asn Gly Gly Glu Pro Lys Val
       595                 600                 605
   Pro Lys Met Ala Lys Lys Val Pro Lys Asn Arg Pro Glu Ala Lys Ile
       610                 615                 620
   Glu Pro Leu Lys Glu Glu Lys Lys Lys Glu Lys Ser Gly Asp Ile Gly
   625                 630                 635                 640
   Thr Lys Gln Gln Lys Ala Asn Asp Lys Lys Met Ala Leu Lys
               645                 650


<210>  36
<211>  328
<212>  PRT
<213>  Globodera pallida

<400>  36

Met Asn Asn Ile Leu Asp Ala His Lys Asn Ile Glu Lys His Thr Cys
1               5                   10                  15

Val Arg Phe Arg Asn Ser Ser Glu Gly Asp His Leu His Tyr Tyr Trp
            20                  25                  30

Gly Tyr Gly Cys Asn Ser Gln Val Gly Arg Ser Gly Gly Lys Gln Trp
        35                  40                  45

Ile Ser Leu Gly Pro Gly Cys Trp Thr Val Asn Gly Val His Glu Thr
    50                  55                  60

Leu His Ala Leu Gly Thr Asn His Glu Gln Ser Arg Leu Asp Arg Asp
65                  70                  75                  80

Ala Phe Ile Lys Ile His Gln Glu Asn Met Ile Glu Asn Ile Ala Asp
                85                  90                  95

Ala Phe Ala Ile Ile Pro Gly Ser Pro Thr Gly Asn Pro Tyr Asp Tyr
            100                 105                 110

Gly Ser Val Met His Tyr Asn Gly Lys Ala Phe Ala Lys Asn Asp Lys
        115                 120                 125

Ala Thr Ile Glu Thr Ile Asp Lys Asn Tyr Gln Gln Thr Met Gly Trp
    130                 135                 140

Arg Leu Gly Leu Ser Phe Leu Asp Ala Lys Ala Ile Asn His Arg Tyr
145                 150                 155                 160

Cys Glu His Val Cys Asp Asp Tyr Pro Trp His Ala Pro Asp Cys Arg
                165                 170                 175

Asn Gly Gly Tyr Ala Asn Pro Asn Asn Cys Thr Glu Cys Leu Cys Pro
            180                 185                 190

Leu Gly Tyr Ala Ser Pro Tyr Cys Asp Ser Val Glu Pro Gly Asn Val
        195                 200                 205

Glu Gly Lys Pro Met Cys Pro Gly Glu Ser Asp Ile Leu Leu Thr Glu
    210                 215                 220

Gln Asn Pro Thr Leu Thr Glu Met Arg Trp Tyr Asn Ala Thr Ser Phe
225                 230                 235                 240

Thr Cys Tyr Trp Arg Leu Arg Ala Pro Ala Asp His Tyr Ile Glu Val
                245                 250                 255

Lys Leu Phe Lys Leu Asp Gly Phe Lys Cys Asp Gln Ser Cys Tyr Asn
            260                 265                 270

Phe Ile Glu Val Lys His Gly Asn Leu Ala Asn Ser Gly Pro Arg Phe
        275                 280                 285

Cys Cys Glu Met Pro Thr Glu Ser Met Phe Thr Asp Val Pro Gly His
    290                 295                 300

Glu Val Ile Ile Phe Ala Ser Ala Val Gln Lys Gly Asn Tyr Ser Ile
305                 310                 315                 320

Gly Leu Thr Tyr Ser Ile Thr Lys
                325

<210>  37
<211>  313
<212>  PRT
<213>  Globodera pallida

<400>  37

Met Met Ala Leu Lys Cys Arg Asn Gly His Arg Ser Val Gln Leu Lys
1               5                   10                  15

Cys Pro Gly Thr Glu Met Met Arg Thr Ala Gln Arg Ala Leu Glu Lys
            20                  25                  30

Asn Glu Met Glu Leu Cys Thr Val Glu Trp Ala Gln Gly Gln Ser Arg
        35                  40                  45

His Lys Asn Pro Tyr Arg Gly Gly Ala Ala Thr Asp Ile Gly Lys Lys
    50                  55                  60

Leu Ser Lys Trp Arg Tyr Phe Glu Asp Phe Asn Leu Leu Asp Tyr Val
65                  70                  75                  80

Gly Glu Asp Thr Ser Pro Leu Asp Glu Val Val Pro Asp Tyr Glu Lys
                85                  90                  95

Leu Asp Glu Asp Glu Ile Glu Lys Gln Lys Glu Asp Ala Met Tyr Ser
            100                 105                 110

Gln Phe Gln Tyr Ser Asp Leu Leu Ile Gly Gly Gln His Pro Ser Asn
        115                 120                 125

Ser Gln His Asp Phe Ser Val Asn Asp Phe Ala Ser Val Pro Thr Asp
    130                 135                 140

Gly Cys Ser Asp Tyr Asp Val Pro Thr Ser Phe Arg Asp Gly Ala His
145                 150                 155                 160

Phe Ser Ala Val Pro Pro Ser Ala Arg His Ile Tyr Ala Gln Asn Leu
                165                 170                 175

Asp Val Thr Pro His Val Pro Ser Thr Ser Lys Ser Ser Ile Pro Leu
            180                 185                 190

Val Lys Gln Glu Pro Ile Ser Glu Gln Glu Gln Ser Asn Leu Lys Phe
        195                 200                 205

Val Val Glu Ala Leu Lys Ser Ser Pro Ser Ala Leu Arg Pro Arg Lys
    210                 215                 220

Tyr Arg Ile Lys Pro Asp Ser Glu Lys Gln Asn Pro Leu Tyr Arg Ile
225                 230                 235                 240

Lys Arg Gln Lys Asn Asn Asp Ala Val Arg Arg Ser Arg Asp Lys Ala
                245                 250                 255

Lys Arg Val Gln Glu Ala Lys Glu Asp Glu Leu Lys Val Leu Gln Asp
            260                 265                 270

Asp Asn Cys Arg Lys Ala Glu Leu Ile Glu Thr Leu Lys Ala Ser Asn
        275                 280                 285

Ala Gln Leu Gln Gly Glu Asn Leu Leu Leu Arg Gln Asn Cys Arg Cys
    290                 295                 300

Gly Ala Val Leu Arg Thr Ala Lys Arg
305                 310

<210>  38
<211>  31
<212>  PRT
<213>  Meloidogyne incognita

<400>  38

Met Leu Arg Leu Thr Ala Leu Leu Leu Ile Ala Leu Leu Cys Leu Glu
1               5                   10                  15

Met Val Asn Ser Cys Gly Phe Pro Met Pro Ala Pro Ala Pro Thr
            20                  25                  30

<210>  39
<211>  31
<212>  PRT
<213>  Meloidogyne incognita

<400>  39

Met Ser Ile Ser Phe Asn Asp Gln Pro Lys Leu Val Leu Phe Ile Ala
1               5                   10                  15

Leu Cys Ser Ile Ser Leu Val Glu Ser Ala Tyr Thr Tyr Gln Lys
            20                  25                  30

<210>  40
<211>  31
<212>  PRT
<213>  Meloidogyne incognita

<400>  40

Met Ile Leu Gln Thr Leu Thr Ile Leu Ile Leu Ile Ile Thr Cys Phe
1               5                   10                  15

Lys Gln Cys Arg Gly Glu Leu Arg Glu Met Asp Glu Ala Ser Arg
            20                  25                  30

<210>  41
<211>  31
<212>  PRT
<213>  Meloidogyne incognita

<400>  41

Met Phe Pro Asn Trp Ala Leu Leu Leu Pro Ile Phe Gly Leu Phe Ala
1               5                   10                  15

Tyr Ser Ser Ala Gln Ser Arg Cys Asn Phe Ile Cys Glu Ala Asp
            20                  25                  30

<210>  42
<211>  31
<212>  PRT
<213>  Meloidogyne incognita

<400>  42

Met Phe Ala Ile Gly Gly Leu Cys Leu Leu Leu Trp Leu Ala Val Val
1               5                   10                  15

Asn Ala Ser Asn Ser Ser Glu Val Gln Val Gly Ser Gly Asn Tyr
            20                  25                  30

<210>  43
<211>  31
<212>  PRT
<213>  Meloidogyne incognita

<400>  43

Met Ile Leu Leu Ile Ile Thr Ala Ile Val Cys Gln Ile Asn Ala Gln
1               5                   10                  15

Gln Pro Pro Tyr Ile Pro Pro Gln Asn Ile Asn Gly Thr Phe Gly
            20                  25                  30

<210>  44
<211>  31
<212>  PRT
<213>  Meloidogyne incognita

<400>  44

Met Phe Phe Ile Asn Ile Phe Ile Ile Phe Leu Ile Ile Lys Ile Ile
1               5                   10                  15

Thr Gly Gln Phe Glu Asp Glu Tyr Thr Ile Asn Ala Arg Ala Gly
            20                  25                  30

<210>  45
<211>  31
<212>  PRT
<213>  Meloidogyne incognita

<400>  45

Met Leu Pro Ile Phe Ser Ser Lys Asn Asn Phe Ser Leu Phe Gln Thr
1               5                   10                  15

Thr Leu Ile Leu Leu Leu Leu Ile Ser Gln Ser Gln Ser Val Phe
            20                  25                  30

<210>  46
<211>  31
<212>  PRT
<213>  Meloidogyne incognita

<400>  46

Met Lys Val Ile Phe Val Phe Ile Ser Met Ala Ala Leu Leu Gly Val
1               5                   10                  15

Asn Thr Ala Glu Asn Gly Thr Ala Thr Thr Thr Ser Thr Ala Thr
            20                  25                  30

<210>  47
<211>  31
<212>  PRT
<213>  Meloidogyne incognita

<400>  47

Met Arg Phe Asp Ile Phe Leu Leu Val Ala Phe Ser Leu Gly Val Ala
1               5                   10                  15

Val Asn Cys Gly Val Val Lys Arg Asn Gln Asn Ser Tyr Gly Asp
            20                  25                  30

<210>  48
<211>  31
<212>  PRT
<213>  Meloidogyne incognita

<400>  48

Met Leu Val Leu Pro Phe Val Ala Met Leu Ala Phe Leu Leu Pro Ser
1               5                   10                  15

Val Leu Leu Ile Gln Cys Cys Lys Lys Lys Ala Lys Asp Ala Ala
            20                  25                  30

<210>  49
<211>  31
<212>  PRT
<213>  Meloidogyne incognita

<400>  49

Met Ile Ser Ile Thr Ser Phe Phe Leu Leu Phe Leu Ser Leu Leu Phe
1               5                   10                  15

Leu Phe Asp Phe Ser Thr Ser Asn Cys Ile Glu Tyr Cys Phe Val
            20                  25                  30

<210>  50
<211>  31
<212>  PRT
<213>  Meloidogyne incognita

<400>  50

Met Leu Asn Tyr Tyr Phe Ser Ile Leu Ser Ile Phe Ile Leu Val Phe
1               5                   10                  15

Ile Lys Ser Tyr Asp Ala Gln Phe Thr Ile Ser Arg Thr Pro Gly
            20                  25                  30

<210>  51
<211>  31
<212>  PRT
<213>  Meloidogyne incognita

<400>  51

Met Ala Leu Gln Lys Leu Ile Phe Val Leu Ile Ala Ser Leu Ile Leu
1               5                   10                  15

Ile Asn Leu Ser Ala Asp Ala Gln Asn Thr Pro Ala Phe Tyr Asp
            20                  25                  30

<210>  52
<211>  31
<212>  PRT
<213>  Meloidogyne incognita

<400>  52

Met Leu Arg Asn Tyr Arg Ile Leu Leu Ala Ile Leu Leu Phe Ala Val
1               5                   10                  15

Leu Ile Asp Arg Leu Lys Ala Asp Asp Asp Asp Glu Glu Asn Gly
            20                  25                  30

<210>  53
<211>  31
<212>  PRT
<213>  Meloidogyne incognita

<400>  53

Met Ala Thr Ile Pro Pro Asn Asn Leu Thr Ile Cys Asn Ile Asp Gly
1               5                   10                  15

Thr Pro Ile Gln Asn Ala Gly Asn Phe Leu Thr Asn Asn Ser Gly
            20                  25                  30

<210>  54
<211>  12
<212>  PRT
<213>  Meloidogyne incognita

<400>  54

Ala Leu Leu Leu Ile Ala Leu Leu Cys Leu Glu Met
1               5                   10


<210>  55
<211>  15
<212>  PRT
<213>  Meloidogyne incognita

<400>  55

Leu Leu Leu Ile Ala Leu Leu Cys Leu Glu Met Val Asn Ser Cys
1               5                   10                  15


<210>  56
<211>  15
<212>  PRT
<213>  Meloidogyne incognita

<400>  56

Leu Val Leu Phe Ile Ala Leu Cys Ser Ile Ser Leu Val Glu Ser
1               5                   10                  15


<210>  57
<211>  15
<212>  PRT
<213>  Meloidogyne incognita

<400>  57

Met Ile Leu Gln Thr Leu Thr Ile Leu Ile Leu Ile Ile Thr Cys
1               5                   10                  15


<210>  58
<211>  15
<212>  PRT
<213>  Meloidogyne incognita

<400>  58

Trp Ala Leu Leu Leu Pro Ile Phe Gly Leu Phe Ala Tyr Ser Ser
1               5                   10                  15


<210>  59
<211>  15
<212>  PRT
<213>  Meloidogyne incognita

<400>  59

Leu Pro Ile Phe Gly Leu Phe Ala Tyr Ser Ser Ala Gln Ser Arg
1               5                   10                  15


<210>  60
<211>  15
<212>  PRT
<213>  Meloidogyne incognita

<400>  60

Met Phe Ala Ile Gly Gly Leu Cys Leu Leu Leu Trp Leu Ala Val
1               5                   10                  15


<210>  61
<211>  15
<212>  PRT
<213>  Meloidogyne incognita

<400>  61

Ile Gly Gly Leu Cys Leu Leu Leu Trp Leu Ala Val Val Asn Ala
1               5                   10                  15


<210>  62
<211>  15
<212>  PRT
<213>  Meloidogyne incognita

<400>  62

Ile Leu Leu Ile Ile Thr Ala Ile Val Cys Gln Ile Asn Ala Gln
1               5                   10                  15


<210>  63
<211>  15
<212>  PRT
<213>  Meloidogyne incognita

<400>  63

Leu Leu Ile Ile Thr Ala Ile Val Cys Gln Ile Asn Ala Gln Gln
1               5                   10                  15


<210>  64
<211>  15
<212>  PRT
<213>  Meloidogyne incognita

<400>  64

Met Phe Phe Ile Asn Ile Phe Ile Ile Phe Leu Ile Ile Lys Ile
1               5                   10                  15


<210>  65
<211>  15
<212>  PRT

<213>  Meloidogyne incognita

<400>  65

Asn Phe Ser Leu Phe Gln Thr Thr Leu Ile Leu Leu Leu Leu Ile
1               5                   10                  15


<210>  66
<211>  12
<212>  PRT
<213>  Meloidogyne incognita

<400>  66

Thr Thr Leu Ile Leu Leu Leu Leu Ile Ser Gln Ser
1               5                   10


<210>  67
<211>  15
<212>  PRT
<213>  Meloidogyne incognita

<400>  67

Ile Phe Val Phe Ile Ser Met Ala Ala Leu Leu Gly Val Asn Thr
1               5                   10                  15


<210>  68
<211>  15
<212>  PRT
<213>  Meloidogyne incognita

<400>  68

Phe Val Phe Ile Ser Met Ala Ala Leu Leu Gly Val Asn Thr Ala
1               5                   10                  15


<210>  69
<211>  15
<212>  PRT
<213>  Meloidogyne incognita

<400>  69

Ile Phe Leu Leu Val Ala Phe Ser Leu Gly Val Ala Val Asn Cys
1               5                   10                  15


<210>  70
<211>  15
<212>  PRT
<213>  Meloidogyne incognita

<400>  70

Leu Pro Phe Val Ala Met Leu Ala Phe Leu Leu Pro Ser Val Leu
1               5                   10                  15

<210>  71
<211>  15
<212>  PRT
<213>  Meloidogyne incognita

<400>  71

Phe Val Ala Met Leu Ala Phe Leu Leu Pro Ser Val Leu Leu Ile
1               5                   10                  15


<210>  72
<211>  15
<212>  PRT
<213>  Meloidogyne incognita

<400>  72

Met Leu Ala Phe Leu Leu Pro Ser Val Leu Leu Ile Gln Cys Cys
1               5                   10                  15


<210>  73
<211>  12
<212>  PRT
<213>  Meloidogyne incognita

<400>  73

Phe Phe Leu Leu Phe Leu Ser Leu Leu Phe Leu Phe
1               5                   10


<210>  74
<211>  15
<212>  PRT
<213>  Meloidogyne incognita

<400>  74

Tyr Phe Ser Ile Leu Ser Ile Phe Ile Leu Val Phe Ile Lys Ser
1               5                   10                  15


<210>  75
<211>  15
<212>  PRT
<213>  Meloidogyne incognita

<400>  75

Ala Leu Gln Lys Leu Ile Phe Val Leu Ile Ala Ser Leu Ile Leu
1               5                   10                  15


<210>  76
<211>  15
<212>  PRT
<213>  Meloidogyne incognita

<400>  76

Ile Phe Val Leu Ile Ala Ser Leu Ile Leu Ile Asn Leu Ser Ala

1               5                   10                  15


<210>  77
<211>  12
<212>  PRT
<213>  Meloidogyne incognita

<400>  77

Ala Ser Leu Ile Leu Ile Asn Leu Ser Ala Asp Ala
1               5                   10


<210>  78
<211>  12
<212>  PRT
<213>  Meloidogyne incognita

<400>  78

Ile Leu Leu Ala Ile Leu Leu Phe Ala Val Leu Ile
1               5                   10


<210>  79
<211>  19
<212>  DNA
<213>  Artificial Sequence

<220>
<223>  Forward

<400>  79
ggttcagaga ttccttgtc                                                    19


<210>  80
<211>  19
<212>  DNA
<213>  Artificial Sequence

<220>
<223>  Reverse

<400>  80
gacaaggaat ctctgaacc                                                    19


<210>  81
<211>  19
<212>  DNA
<213>  Artificial Sequence

<220>
<223>  Forward

<400>  81
ttgacaacca attgccgtt                                                    19


<210>  82
<211>  19

<212>  DNA
<213>  Artificial Sequence

<220>
<223>  Reverse

<400>  82
aacggcaatt ggttgtcaa                                                    19


<210>  83
<211>  19
<212>  DNA
<213>  Artificial Sequence

<220>
<223>  Forward

<400>  83
atgacggcat tatccgttc                                                    19


<210>  84
<211>  19
<212>  DNA
<213>  Artificial Sequence

<220>
<223>  Reverse

<400>  84
gaacggataa tgccgtcat                                                    19


<210>  85
<211>  19
<212>  DNA
<213>  Artificial Sequence

<220>
<223>  Forward

<400>  85
gcaggagttg tatgcaagc                                                    19


<210>  86
<211>  19
<212>  DNA
<213>  Artificial Sequence

<220>
<223>  Reverse

<400>  86
gcttgcatac aactcctgc                                                    19


<210>  87
<211>  19
<212>  DNA
<213>  Artificial Sequence

<220>
<223>  Forward

<400>  87
gagatagaag tgagtgtac                                                    19


<210>  88
<211>  19
<212>  DNA
<213>  Artificial Sequence

<220>
<223>  Reverse

<400>  88
gtacactcac ttctatctc                                                    19


<210>  89
<211>  19
<212>  DNA
<213>  Artificial Sequence

<220>
<223>  Forward

<400>  89
tccacattat cgcgcagga                                                    19


<210>  90
<211>  19
<212>  DNA
<213>  Artificial Sequence

<220>
<223>  Forward

<400>  90
tcctgcgcga taatgtgga                                                    19


<210>  91
<211>  19
<212>  DNA
<213>  Artificial Sequence

<220>
<223>  Forward

<400>  91
cgctccatgc attaggttc                                                    19


<210>  92
<211>  19
<212>  DNA
<213>  Artificial Sequence

<220>
<223>  Reverse

<400>  92
gaacctaatg catggagcg                                                    19


<210>  93
<211>  19
<212>  DNA
<213>  Artificial Sequence

<220>
<223>  Forward

<400>  93
cgacagcgaa gaagactac                                                    19


<210>  94
<211>  19
<212>  DNA
<213>  Artificial Sequence

<220>
<223>  Reverse

<400>  94
gtagtcttct tcgctgtcg                                                    19


<210>  95
<211>  19
<212>  DNA
<213>  Artificial Sequence

<220>
<223>  Forward

<400>  95
agaacggatc aatcgagca                                                    19


<210>  96
<211>  19
<212>  DNA
<213>  Artificial Sequence

<220>
<223>  Reverse

<400>  96
tgctcgattg atccgttct                                                    19


<210>  97
<211>  19
<212>  DNA
<213>  Artificial Sequence

<220>
<223>  Forward

<400>  97
acgttcacag aactcttac                                                    19

<210>  98
<211>  19
<212>  DNA
<213>  Artificial Sequence

<220>
<223>  Reverse

<400>  98
gtaagagttc tgtgaacgt                                                    19


<210>  99
<211>  19
<212>  DNA
<213>  Artificial Sequence

<220>
<223>  Forward

<400>  99
gacgaagctg gtcaatgct                                                    19


<210>  100
<211>  19
<212>  DNA
<213>  Artificial Sequence

<220>
<223>  Reverse

<400>  100
agcattgacc agcttcgtc                                                    19


<210>  101
<211>  19
<212>  DNA
<213>  Artificial Sequence

<220>
<223>  Forward

<400>  101
caagcaatgc tgttcatgc                                                    19


<210>  102
<211>  19
<212>  DNA
<213>  Artificial Sequence

<220>
<223>  Reverse

<400>  102
gcatgaacag cattgcttg                                                    19


<210>  103
<211>  19

<212>  DNA
<213>  Artificial Sequence

<220>
<223>  Forward

<400>  103
tggcattcaa gttggtcct                                                    19


<210>  104
<211>  19
<212>  DNA
<213>  Artificial Sequence

<220>
<223>  Reverse

<400>  104
aggaccaact tgaatgcca                                                    19


<210>  105
<211>  19
<212>  DNA
<213>  Artificial Sequence

<220>
<223>  Forward

<400>  105
atccggcaag tctgcttgt                                                    19


<210>  106
<211>  19
<212>  DNA
<213>  Artificial Sequence

<220>
<223>  Reverse

<400>  106
acaagcagac ttgccggat                                                    19


<210>  107
<211>  19
<212>  DNA
<213>  Artificial Sequence

<220>
<223>  Forward

<400>  107
aggacaacaa ggaggcatt                                                    19


<210>  108
<211>  19
<212>  DNA
<213>  Artificial Sequence

<220>
<223>  Reverse

<400>  108
aatgcctcct tgttgtcct                                                    19


<210>  109
<211>  19
<212>  DNA
<213>  Artificial Sequence

<220>
<223>  Forward

<400>  109
ggatgaaccg ttggaacaa                                                    19


<210>  110
<211>  19
<212>  DNA
<213>  Artificial Sequence

<220>
<223>  Reverse

<400>  110
ttgttccaac ggttcatcc                                                    19


<210>  111
<211>  29
<212>  DNA
<213>  Artificial Sequence

<220>
<223>  Forward

<400>  111
aaacgttcac agaactctta ccctgtctc                                         29


<210>  112
<211>  29
<212>  DNA
<213>  Artificial Sequence

<220>
<223>  Reverse

<400>  112
aagtaagagt tctgtgaacg tcctgtctc                                         29


<210>  113
<211>  29
<212>  DNA
<213>  Artificial Sequence

<220>
<223>  Forward

<400>  113
aaggttcaga gattccttgt ccctgtctc                                          29


<210>  114
<211>  29
<212>  DNA
<213>  Artificial Sequence

<220>
<223>  Reverse

<400>  114
aagacaagga atctctgaac ccctgtctc                                          29


<210>  115
<211>  29
<212>  DNA
<213>  Artificial Sequence

<220>
<223>  Forward

<400>  115
aattgacaac caattgccgt tcctgtctc                                          29


<210>  116
<211>  29
<212>  DNA
<213>  Artificial Sequence

<220>
<223>  Reverse

<400>  116
aaaacggcaa ttggttgtca acctgtctc                                          29


<210>  117
<211>  29
<212>  DNA
<213>  Artificial Sequence

<220>
<223>  Forward

<400>  117
aacgctccat gcattaggtt ccctgtctc                                          29


<210>  118
<211>  29
<212>  DNA
<213>  Artificial Sequence

<220>
<223>  Reverse

<400>  118
aagaacctaa tgcatggagc gcctgtctc                                          29

<210>  119
<211>  29
<212>  DNA
<213>  Artificial Sequence

<220>
<223>  Forward

<400>  119
aagcaggagt tgtatgcaag ccctgtctc                                          29


<210>  120
<211>  29
<212>  DNA
<213>  Artificial Sequence

<220>
<223>  Reverse

<400>  120
aagcttgcat acaactcctg ccctgtctc                                          29


<210>  121
<211>  29
<212>  DNA
<213>  Artificial Sequence

<220>
<223>  Forward

<400>  121
aaatgacggc attatccgtt ccctgtctc                                          29


<210>  122
<211>  29
<212>  DNA
<213>  Artificial Sequence

<220>
<223>  Reverse

<400>  122
aagaacggat aatgccgtca tcctgtctc                                          29


<210>  123
<211>  29
<212>  DNA
<213>  Artificial Sequence

<220>
<223>  Forward

<400>  123
aaagaacgga tcaatcgagc acctgtctc                                          29


<210>  124
<211>  29

<212>  DNA
<213>  Artificial Sequence

<220>
<223>  Reverse

<400>  124
aatgctcgat tgatccgttc tcctgtctc                                         29


<210>  125
<211>  29
<212>  DNA
<213>  Artificial Sequence

<220>
<223>  Forward

<400>  125
aaatccggca agtctgcttg tcctgtctc                                         29


<210>  126
<211>  29
<212>  DNA
<213>  Artificial Sequence

<220>
<223>  Reverse

<400>  126
aaacaagcag acttgccgga tcctgtctc                                         29


<210>  127
<211>  29
<212>  DNA
<213>  Artificial Sequence

<220>
<223>  Forward

<400>  127
aacgacagcg aagaagacta ccctgtctc                                         29


<210>  128
<211>  29
<212>  DNA
<213>  Artificial Sequence

<220>
<223>  Reverse

<400>  128
aagtagtctt cttcgctgtc gcctgtctc                                         29


<210>  129
<211>  29
<212>  DNA
<213>  Artificial Sequence

<220>
<223>  Forward

<400>  129
aagagataga agtgagtgta ccctgtctc                                          29


<210>  130
<211>  29
<212>  DNA
<213>  Artificial Sequence

<220>
<223>  Reverse

<400>  130
aagtacactc acttctatct ccctgtctc                                          29


<210>  131
<211>  29
<212>  DNA
<213>  Artificial Sequence

<220>
<223>  Forward

<400>  131
aatggcattc aagttggtcc tcctgtctc                                          29


<210>  132
<211>  29
<212>  DNA
<213>  Artificial Sequence

<220>
<223>  Reverse

<400>  132
aaaggaccaa cttgaatgcc acctgtctc                                          29


<210>  133
<211>  29
<212>  DNA
<213>  Artificial Sequence

<220>
<223>  Forward

<400>  133
aagacgaagc tggtcaatgc tcctgtctc                                          29


<210>  134
<211>  29
<212>  DNA
<213>  Artificial Sequence

<220>
<223>  Reverse

<400>  134
aaagcattga ccagcttcgt ccctgtctc                                          29


<210>  135
<211>  29
<212>  DNA
<213>  Artificial Sequence

<220>
<223>  Forward

<400>  135
aacaagcaat gctgttcatg ccctgtctc                                          29


<210>  136
<211>  29
<212>  DNA
<213>  Artificial Sequence

<220>
<223>  Reverse

<400>  136
aagcatgaac agcattgctt gcctgtctc                                          29


<210>  137
<211>  29
<212>  DNA
<213>  Artificial Sequence

<220>
<223>  Forward

<400>  137
aatccacatt atcgcgcagg acctgtctc                                          29


<210>  138
<211>  29
<212>  DNA
<213>  Artificial Sequence

<220>
<223>  Reverse

<400>  138
aatcctgcgc gataatgtgg acctgtctc                                          29


<210>  139
<211>  29
<212>  DNA
<213>  Artificial Sequence

<220>
<223>  Forward

<400>  139
aaaggacaac aaggaggcat tcctgtctc                                          29

<210>  140
<211>  29
<212>  DNA
<213>  Artificial Sequence

<220>
<223>  Reverse

<400>  140
aaaatgcctc cttgttgtcc tcctgtctc                                          29


<210>  141
<211>  29
<212>  DNA
<213>  Artificial Sequence

<220>
<223>  Forward

<400>  141
aagtacattt aacgccagcc tcctgtctc                                          29


<210>  142
<211>  29
<212>  DNA
<213>  Artificial Sequence

<220>
<223>  Reverse

<400>  142
aaaggctggc gttaaatgta ccctgtctc                                          29


<210>  143
<211>  24
<212>  DNA
<213>  Artificial Sequence

<220>
<223>  primer

<400>  143
atggctacaa tacctccaaa taat                                               24


<210>  144
<211>  24
<212>  DNA
<213>  Artificial Sequence

<220>
<223>  primer

<400>  144
taattgtctg ttctgtctgc caag                                               24


<210>  145
<211>  22

<212>  DNA
<213>  Artificial Sequence

<220>
<223>  primer

<400>  145
ctatggagat gaggcaggac at                                                22


<210>  146
<211>  21
<212>  DNA
<213>  Artificial Sequence

<220>
<223>  primer

<400>  146
gtgaacgttt cttgcggtat c                                                 21


<210>  147
<211>  25
<212>  DNA
<213>  Artificial Sequence

<220>
<223>  primer

<400>  147
attgctcttt tgtgtttaga aatgg                                             25


<210>  148
<211>  25
<212>  DNA
<213>  Artificial Sequence

<220>
<223>  primer

<400>  148
caaggaatct ctgaaccttt gaaat                                             25


<210>  149
<211>  20
<212>  DNA
<213>  Artificial Sequence

<220>
<223>  primer

<400>  149
cgaaatttaa tgcggattgg                                                   20


<210>  150
<211>  20
<212>  DNA
<213>  Artificial Sequence

<220>
<223>  primer

<400>  150
tgtgggcgat atggcttaat                                                    20


<210>  151
<211>  25
<212>  DNA
<213>  Artificial Sequence

<220>
<223>  primer

<400>  151
tgcccacaaa aatatagaag aaaat                                              25


<210>  152
<211>  24
<212>  DNA
<213>  Artificial Sequence

<220>
<223>  primer

<400>  152
agataaccac tgctttcctc catt                                               24


<210>  153
<211>  20
<212>  DNA
<213>  Artificial Sequence

<220>
<223>  primer

<400>  153
agctgatcgt gctgtcattg                                                    20


<210>  154
<211>  20
<212>  DNA
<213>  Artificial Sequence

<220>
<223>  primer

<400>  154
acagaaagac gcgcaatcac                                                    20


<210>  155
<211>  25
<212>  DNA
<213>  Artificial Sequence

<220>
<223>  primer

<400>  155
tgttactgtc caatcagaaa atgaa                                          25


<210>  156
<211>  25
<212>  DNA
<213>  Artificial Sequence

<220>
<223>  primer

<400>  156
acatatccca tgttgtggac taact                                          25


<210>  157
<211>  24
<212>  DNA
<213>  Artificial Sequence

<220>
<223>  primer

<400>  157
agatccaccc ttagaaagcc atag                                           24


<210>  158
<211>  25
<212>  DNA
<213>  Artificial Sequence

<220>
<223>  primer

<400>  158
ttgtcgtaaa gtcttgtggt gatag                                          25


<210>  159
<211>  24
<212>  DNA
<213>  Artificial Sequence

<220>
<223>  primer

<400>  159
tccagcgttc tatgattgta attg                                           24


<210>  160
<211>  24
<212>  DNA
<213>  Artificial Sequence

<220>
<223>  primer

<400>  160
cactgcttag gaatatcaat gctc                                           24

<210>  161
<211>  26
<212>  DNA
<213>  Artificial Sequence

<220>
<223>  primer

<400>  161
ttattattaa tttcccaaag ccaatc                                          26


<210>  162
<211>  25
<212>  DNA
<213>  Artificial Sequence

<220>
<223>  primer

<400>  162
tatattttgt tccccttcct cctta                                           25


<210>  163
<211>  25
<212>  DNA
<213>  Artificial Sequence

<220>
<223>  primer

<400>  163
agtgagtgta caggatcaac atctg                                           25


<210>  164
<211>  25
<212>  DNA
<213>  Artificial Sequence

<220>
<223>  primer

<400>  164
gctgacattt gctgtcagag tatta                                           25


<210>  165
<211>  21
<212>  DNA
<213>  Artificial Sequence

<220>
<223>  primer

<400>  165
caggtctaac agcctcctca a                                               21


<210>  166
<211>  20

<212>  DNA
<213>  Artificial Sequence

<220>
<223>  primer

<400>  166
cagttccttg tggcatgaat                                                    20


<210>  167
<211>  20
<212>  DNA
<213>  Artificial Sequence

<220>
<223>  primer

<400>  167
tggagctgtt acaccagcag                                                    20


<210>  168
<211>  20
<212>  DNA
<213>  Artificial Sequence

<220>
<223>  primer

<400>  168
aagaatgacc gttggagtgc                                                    20


<210>  169
<211>  20
<212>  DNA
<213>  Artificial Sequence

<220>
<223>  primer

<400>  169
tatcaaccag cagccactca                                                    20


<210>  170
<211>  20
<212>  DNA
<213>  Artificial Sequence

<220>
<223>  primer

<400>  170
cgttgtctgt gccttgaaga                                                    20


<210>  171
<211>  20
<212>  DNA
<213>  Artificial Sequence

<220>
<223>  primer

<400>  171
tacaccggtc agcaacattc                                                   20


<210>  172
<211>  20
<212>  DNA
<213>  Artificial Sequence

<220>
<223>  primer

<400>  172
agattccttt gcccttctgg                                                   20


<210>  173
<211>  20
<212>  DNA
<213>  Artificial Sequence

<220>
<223>  primer

<400>  173
ctccaggtga ggatgacgag                                                   20


<210>  174
<211>  20
<212>  DNA
<213>  Artificial Sequence

<220>
<223>  primer

<400>  174
atgcctggtt gtccagtacc                                                   20


<210>  175
<211>  21
<212>  DNA
<213>  Artificial Sequence

<220>
<223>  primer

<400>  175
ggttatctca gctccgtctg c                                                 21


<210>  176
<211>  20
<212>  DNA
<213>  Artificial Sequence

<220>
<223>  primer

<400>  176
taaccttcgc aagaggagca                                                   20


<210>  177
<211>  20
<212>  DNA
<213>  Artificial Sequence

<220>
<223>  primer

<400>  177
aagacgaagc agctgtagcc                                                   20


<210>  178
<211>  20
<212>  DNA
<213>  Artificial Sequence

<220>
<223>  primer

<400>  178
ggtgttacgc acacagttcc                                                   20


<210>  179
<211>  23
<212>  DNA
<213>  Artificial Sequence

<220>
<223>  primer

<400>  179
atgagtcaaa caataacacc ccc                                               23


<210>  180
<211>  22
<212>  DNA
<213>  Artificial Sequence

<220>
<223>  primer

<400>  180
aatatataag ggccagacaa ag                                                22

## Description

Plant-parasitic nematodes (PPN) are nearly microscopic, worm-shaped animals virtually invisible to the naked eye when in the soil. Although a few nematode species feed on above ground plant parts, such as leaves, stems, flowers and seeds, the majority of these parasites feed on underground parts of plants, including roots, bulbs and tubers. Most PPN feed on root tissue and damage their host mainly by inhibiting root growth resulting in reduced water uptake. Further, these nematodes favour microbial infections through wound sites or serve as vectors for pathogenic viruses. While some species exhibit a hit-and-run strategy, remaining migratory throughout their life cycle into the plant root, an increase in complexity of host-parasite interactions is associated in sedentary parasite species with their enhanced capacity to manipulate host plant genes in their favour. These endoparasitic nematodes settle down after an initial migratory phase and assume a sedentary life style while transforming plant cells into complex feeding structures. Nematodes of this category represent the most damaging species for crops. Some of these nematodes have a relatively specialized host range (*e.g.,* cyst nematodes *Heterodera* and *Globodera* genus) while others are able to successfully infest thousands of unrelated host plant species (*e.g.,* root-knot nematodes *Meloidogyne* spp.).

Because root-knot nematodes represent the most economically-important PPN, they constitute the most explored group of species and can now be considered as one of the most advanced models for understanding mechanisms of plant parasitism in nematodes. As other PPN, sedentary endoparasites have a stylet that is used to pierce and penetrate plant cell walls, to release esophageal secretions into the host tissue and to take up nutrients. During their infective life-cycle, root-knot nematode larvae penetrate plant root tissue and migrate along the vascular cylinder. By injecting secretions into plant cells, they induce the formation of a feeding site indispensable for their development. As a consequence of the formation of these feeding structures, root-knots or galls are observed as symptoms of the infestation. Plant nutrient and water uptake are substantially reduced by the resulting damage to the root system, and infested plants are therefore weak and give low yields. Once the feeding structure is established, female nematodes continue their development and eventually become pear-shaped and produce hundreds to thousands of eggs. These eggs are then extruded as an egg-mass, protected within a gelatinous matrix, at the outer surface of the root.

Plant-parasitic nematodes (PPN) cause significant damage to agriculture throughout the world. A global survey in 1987 evaluated crop losses at $78-125 billion per year (Sasser and Freckman, 1987). More recent direct global estimates are not available, but when the increase in agricultural productivity is taken into account, the extrapolated 2001 loss for crops totalled $118 billion (11% of production) (McCarter, 2009). The current figure is thus probably much higher. Measures such as growing resistant crop varieties and the use of nematicides are extensively employed to control PPN infections. Billions of euros have been spent annually on soil fumigants and other nematicides. Chemical controls against nematodes are not only costly but they are highly toxic and hazardous, and involve application of environmentally unacceptable compounds. Such toxicological problems and environmental damage caused by nematicides have led to banning of the most efficient chemicals that were commonly used so far in Europe (EC directive 2007/619/EC). In the absence of alternative control methods or development of specific and environmentally safe molecules, it is to be feared severe crop losses within major sectors of the agricultural industry. Indeed, nematode problems recently re-emerged in some areas where the use of traditional nematicides has been abandoned for a short while (Djian-Caporalino, 2012; Wesemael *et al.,* 2011).

It appears from the foregoing that there is a need for methods for limiting the damages caused by the plant-parasitic nematodes.

Mining the genomes of root-knot nematodes *Meloidogyne incognita* and *Meloidogyne hapla* through an evolutionary and comparative genomics approach, the inventors have searched genes conserved in various plant parasites while otherwise absent from the genomes of non target species such as those of chordates, plants, annelids, insect pollinators and mollusks. The inventors have identified a set of root-knot nematode genes absent from non-target species but present in several plant parasites. Further bioinformatics pruning of this set of genes yielded 16 candidate genes (15 genes encoding effector like proteins and 1 gene encoding a transcription factor) present both in *M incognita* and *M hapla.* These 16 candidate genes were respectively silenced using siRNA technique in *M incognita.* As much as 12 of the candidate genes turned out to show a significant and reproducible diminution of *M. incognita* infestation on tomato plants when silenced with siRNAs (namely the candidate genes *Minc00801, Minc01632, Minc02483, Minc03313, Minc03866, Minc05001, Minc08013, Minc08335, Minc09526, Minc12224, Minc17713* and *Minc07817).* The remaining 4 candidate genes turned out to show a significant but not reproducible reduction of *M. incognita* infestation when silenced with siRNAs (namely the candidate genes *Minc08014, Minc10706, Minc14652* and *Minc17987*). In particular, during infestation tests on tomato plants, the inventors have shown that:
- siRNA silencing of candidate genes *Minc01632, Minc02483, Minc05001, Minc03866* or *Minc09526* induced a significant and reproducible reduction of both the number of galls and egg masses on tomato (the candidate genes are classified by decreasing order of the amplitude of diminution of the number of egg masses),
- siRNA silencing of candidate genes *Minc17713, Minc03313, Minc07817, Minc08335, Minc08013, Minc00801, Minc12224* induced a significant and reproducible reduction of galls or egg masses on tomato (the candidate genes are classified by decreasing order of the amplitude of diminution of the number of egg masses), and
- siRNA silencing of candidate genes *Minc14652, Minc10706, Minc08014* and *Minc17987* induced a significant but not reproducible reduction of galls or egg masses on tomato (the candidate genes are classified by decreasing order of the effect on the egg mass).

Overall, out of the 34,780 predicted proteins from the *M. incognita* and *M hapla* whole proteomes, the inventors have selected 16 candidate genes.

The inventors have found that the 15 effector-like proteins comprise a signal peptide sequence including a MERCI effector-specific motif, and no transmembrane region.

As used herein, an effector-like protein refers to a protein that presents characteristics similar to those of known effectors. Effectors are proteins secreted by the nematodes and that support the process of plant parasitism. Approximately 100 confirmed effectors are known from root-knot nematodes (Vens *et al.,* 2011). Effectors usually bear a signal peptide for secretion and have no transmembrane region. A MERCI peptide motif is frequently found inside the signal peptide of effectors (Vens *et al.,* 2011).

As used herein, a signal peptide refers to a short polypeptide sequence (typically 5-35 amino acid residues), mainly composed of hydrophobic amino acids that have a tendency to form a single alpha-helix. This signal peptide is found at the N-terminal part of the protein and is responsible for directing the protein towards a secretion pathway. In the effector-like proteins and transcription factors, the signal peptide usually corresponds to the first 31 amino acids of the proteins. Methods for identifying a signal peptide in proteins are well-known to a person skilled in the art. By way of example, signal peptides can be detected in root-knot nematode proteins, by using the softwares SignalP 3.0 (Nielsen *et al.,* 1997) or SignalP 4.0 (Petersen *et al.,* 2011). In SignalP 3.0 softawre, predictions of signal peptides are made according to two approaches, Neural Networks (NN) and Hidden Markov Models (HMM): a signal peptide is considered present when its detection w is significant according to at least one of the two approaches.

As used herein, a MERCI effector-specific motif refers to a short polypeptide sequence identified as frequently present in the signal peptides of known root-knot nematode effector proteins but absent from those of housekeeping proteins. The MERCI effector-specific motifs are identified using the MERCI (Motif - EmeRging and with Classes-Identification) software (Vens *et al.,* 2011). The MERCI software first searches for conserved sequences of amino acids in the proteins of interest. If no exact conservation of the amino acid itself is found, the MERCI software searches for conservation of the physico-chemical properties of an amino acid at a given position. Using the MERCI software, Vens *et al.* (2011) have identified a total of 4 MERCI peptide motifs of 12 or 15 amino acid residues in root-knot nematode effectors. MOTIF 1 consists of 15 amino acid residues classified according to the RasMol's classification (Sayle *et al.,* 1995) from the N-terminal to the C-terminal as follows: large, hydrophobic, neutral, buried, neutral, neutral, buried, buried, neutral, acyclic, acyclic, hydrophobic, neutral, acyclic, acyclic. MOTIF 2 consists of 15 amino acid residues classified according to the RasMol's classification from the N-terminal to the C-terminal as follows: neutral, buried, neutral, large, buried, neutral, neutral, neutral, hydrophobic, hydrophobic, neutral, acyclic, acyclic, acyclic, buried. MOTIF 3 consists of 15 amino acid residues classified according to the RasMol's classification from the N-terminal to the C-terminal as follows: hydrophobic, neutral, buried, acyclic, neutral, neutral, neutral, buried, neutral, large, neutral, acyclic, neutral, polar, acyclic. MOTIF 4 consists of 12 amino acid residues classified according to the RasMol's classification from the N-terminal to the C-terminal as follows: neutral, neutral, L (leucine), buried, hydrophobic, buried, neutral, hydrophobic, neutral, neutral, acyclic, neutral. The amino acid sequence of the 15 effector-like proteins identified by the inventors contains at least one of these four MERCI motifs in their signal peptide sequence.

As used herein, a transmembrane region refers to a portion of a protein, which, given the physico-chemical properties of its constituting amino-acids is thermodynamically stable in a cell membrane. In a protein, transmembrane regions reside in the cell membrane. Secreted proteins are usually devoid of transmembrane region.

Transgenic plants expressing siRNAs, and more generally RNAs (e.g., dsRNAs), capable of driving post-transcriptional inhibition (e.g., post-transcriptional silencing) of one or several of these 16 candidate genes can be produced to obtain plants with a reduced plant-parasitic nematode infestation level.

Accordingly, the present invention provides a method for reducing the plant-parasitic nematode infestation level in a plant, comprising expressing in said plant at least one RNA capable of driving post-transcriptional inhibition of at least one target gene from said nematode,
wherein said gene is selected from the group consisting of a gene encoding an effector-like protein and a gene encoding a transcription factor,
wherein said effector-like protein:
- comprises a signal peptide including 1, 2, 3 or 4 different MERCI effector-specific motifs selected from the group consisting of:
   MOTIF 1: large, hydrophobic, neutral, buried, neutral, neutral, buried, buried, neutral, acyclic, acyclic, hydrophobic, neutral, acyclic, acyclic,
   MOTIF 2: neutral, buried, neutral, large, buried, neutral, neutral, neutral, hydrophobic, hydrophobic, neutral, acyclic, acyclic, acyclic, buried,
   MOTIF 3: hydrophobic, neutral, buried, acyclic, neutral, neutral, neutral, buried, neutral, large, neutral, acyclic, neutral, polar, acyclic, and
   MOTIF 4: neutral, neutral, L, buried, hydrophobic, buried, neutral, hydrophobic, neutral, neutral, acyclic, neutral,
- comprises no transmembrane region, and
- consists of a polypeptide sequence having at least 35% identity, or by order of increasing preference at least 40%, 45%, 50%, 55%, 60%, 65%, 70%, 75%, 80%, 85%, 90%, 95%, 96%, 97%, 98% or 99% identity, and preferably having 100% identity, with a polypeptide selected from the group consisting of SEQ ID NO. 1 (Minc01632), SEQ ID NO. 2 (Minc02483), SEQ ID NO. 3 (Minc05001), SEQ ID NO. 4 (Minc03866), SEQ ID NO. 5 (Minc09526), SEQ ID NO. 6 (Minc17713), SEQ ID NO. 7 (Minc03313), SEQ ID NO. 8 (Minc08335), SEQ ID NO. 9 (Minc08013), SEQ ID NO. 10 (Minc00801), SEQ ID NO. 11 (Minc12224), SEQ ID NO. 12 (Minc14652), SEQ ID NO. 13 (Minc10706), SEQ ID NO. 14 (Minc08014) and SEQ ID NO. 15 (Minc17987), preferably with a polypeptide selected from the group consisting of SEQ ID NO. 1 (Minc01632), SEQ ID NO. 2 (Minc02483), SEQ ID NO. 3 (Minc05001), SEQ ID NO. 4 (Minc03866), SEQ ID NO. 5 (Minc09526), SEQ ID NO. 6 (Minc17713), SEQ ID NO. 7 (Minc03313), SEQ ID NO. 8 (Minc08335), SEQ ID NO. 9 (Minc08013), SEQ ID NO. 10 (Minc00801), SEQ ID NO. 11 (Minc12224), more preferably with a polypeptide selected from the group consisting of SEQ ID NO. 1 (Minc01632), SEQ ID NO. 2 (Minc02483), SEQ ID NO. 3 (Minc05001), SEQ ID NO. 4 (Minc03866), SEQ ID NO. 5 (Minc09526), most preferably with the polypeptide of sequence SEQ ID NO. 1 (Minc01632), and
wherein said transcription factor comprises a signal peptide and consists of a polypeptide sequence having at least 35% identity, or by order of increasing preference at least 40%, 45%, 50%, 55%, 60%, 65%, 70%, 75%, 80%, 85%, 90%, 95%, 96%, 97%, 98% or 99% identity, and preferably having 100% identity, with the polypeptide of sequence SEQ ID NO. 16 (Minc07817).

Unless otherwise specified, the percents of identity between two sequences which are mentioned herein are calculated from an alignment of the two sequences over the whole length of any of the two sequences, preferably over the whole length of the two sequences. By way of example, one can use the *« needle* » program (Needleman *et al.,* 1970).

As used herein, at least one target gene means 1, 2, 3, 4, 5, 6, 7, 8, 9, 10, 11, 12, 13, 14, 15 or 16 target genes, preferably 1, 2, 3, 4, 5 or 6 target genes, more preferably 1, 3 or 6 target genes.

Advantageously, the amino acid sequence of the signal peptide including a MERCI effector-specific motif of an effector-like protein according to the present invention is selected from the group consisting of SEQ ID NO. 38 to 52 (corresponding to the signal peptide present in the amino acid sequence of the proteins Minc01632, Minc02483, Minc05001, Minc03866, Minc09526, Minc17713, Minc03313, Minc08335, Minc08013, Minc00801, Minc12224, Minc14652, Minc10706, Minc08014 and Minc17987 respectively), and the amino acid sequence of the signal peptide of the transcription factor according to the present invention is SEQ ID NO. 53 (corresponding to the signal peptide present in the amino acid sequence of the protein Minc07817).

Advantageously, the amino acid sequence of the MERCI effector-specific motif is selected from the group consisting of SEQ ID NO. 54 (MOTIF 4 present in the amino acid sequence of the protein Minc01632), 55 (MOTIF 1 present in the amino acid sequence of the protein Minc01632), 56 (MOTIF 1 present in the amino acid sequence of the protein Minc02483), 57 (MOTIF 3 present in the amino acid sequence of the protein Minc05001), 58 (MOTIF 3 present in the amino acid sequence of the protein Minc03866), 59 (MOTIF 1 present in the amino acid sequence of the protein Minc03866), 60 (MOTIF 1 present in the amino acid sequence of the protein Minc09526), 61 (MOTIF 1 present in the amino acid sequence of the protein Minc09526), 62 (MOTIF 1 present in the amino acid sequence of the protein Minc17713), 63 (MOTIF 3 present in the amino acid sequence of the protein Minc17713), 64 (MOTIF 3 present in the amino acid sequence of the protein Minc03313), 65 (MOTIF 2 present in the amino acid sequence of the protein Minc08335), 66 (MOTIF 4 present in the amino acid sequence of the protein Minc08335), 67 (MOTIF 1 present in the amino acid sequence of the protein Minc08413), 68 (MOTIF 1 present in the amino acid sequence of the protein Minc08013), 69 (MOTIF 2 present in the amino acid sequence of the protein Minc00801), 70 (MOTIF 1 present in the amino acid sequence of the protein Minc12224), 71 (MOTIF 2 present in the amino acid sequence of the protein Minc12224), 72 (MOTIF 2 present in the amino acid sequence of the protein Minc12224), 73 (MOTIF 4 present in the amino acid sequence of the protein Minc14652), 74 (MOTIF 1 present in the amino acid sequence of the protein Minc10706), 75 (MOTIF 2 present in the amino acid sequence of the protein Minc08014), 76 (MOTIF 2 present in the amino acid sequence of the protein Minc08014), 77 (MOTIF 4 present in the amino acid sequence of the protein Minc08014) and 78 (MOTIF 1 present in the amino acid sequence of the protein Minc17987).

The inventors have determined that the effector-like proteins Minc01632, Minc02483, Minc05001, Minc03866, Minc09526, Minc17713, Minc03313, Minc08335, Minc08013, Minc00801, Minc12224, Minc14652, Minc10706, Minc08014 and Minc17987 of *M incognita* as described above show respectively 58%, 80%, 74%, 60%, 47%, 81%, 95%, 79%, 60%, 87%, 79%, 68%, 68%, 63% and 73% identity with the heterologous effector-like proteins in *M. hapla* (Mhap01632, Mhap02483, Mhap05001, Mhap03866, Mhap09526, Mhap17713, Mhap03313, Mhap08335, Mhap08013, Mhap00801, Mhap12224, Mhap14652, Mhap10706, Mhap08014 and Mhap17987 respectively). Said heterologous effector-like proteins in *M. hapla* are referred to as SEQ ID NO. 17 to 31 respectively.

The inventors have determined that the transcription factor Minc07817 of *M. incognita* shows 81 % identity with the heterologous transcription factor in *M. hapla* (Mhap07817). Said heterologous transcription factor in *M. hapla* of Minc07817 is referred to as SEQ ID NO. 32.

The inventors have determined that the effector-like proteins Minc01632, Minc02483, Minc05001 and Minc03313 of *M. incognita* as described above show respectively 44%, 44%, 35% and 66% identity with the heterologous effector-like proteins in the cyst nematode *Globodera pallida* (Gpa101632, Gpa102483, Gpa105001 and Gpa103313 respectively). Said heterologous proteins in *G. pallida* are referred to as SEQ ID NO. 33 to 36 respectively.

The inventors have determined that the transcription factor Minc07817 of *M. incognita* shows 38% identity with the heterologous transcription factor in *G. pallida* (Gpa107817). Said heterologous transcription factor in *G. pallida* of Minc07817 is referred to as SEQ ID NO. 37.

Advantageously, the RNA capable of driving post-transcriptional inhibition of at least one target gene from a nematode is selected from the group consisting of a sense RNA (for co-suppression), an antisense RNA and a dsRNA.

Preferred RNAs capable of driving post-transcriptional inhibition of at least one target gene are dsRNAs capable of driving the post-transcriptional silencing of the targeted gene with homologous sequences. Post transcriptional gene silencing is induced by means of the cellular mechanism called RNA interference (RNAi). Various methods and DNA constructs for RNAi are available in the art (for review, Watson *et al.,* 2005). Typically, DNA constructs for delivering a dsRNA in a plant include at least a fragment of at least 15 contiguous nucleotides of the cDNA of the target gene, under transcriptional control of a promoter active in said plant. Currently, the more widely used DNA constructs are those encoding a dsRNA that produces - through cleavage by RNases into the plant cell - a small interfering RNA (siRNA), a hairpin RNA (hpRNA) or an artificial microRNA (amiRNA). In these constructs, the fragment of the target gene is inversely repeated, with generally a linker region between the repeats.

Transgenic plants expressing a dsRNA targeting a gene from a plant-parasitic nematode are known in the art (see for review Rosso *et al.,* 2009; Atkinson *et al.,* 2012; Kozub *et al.,* 2012; Schwab *et al.,* 2006). In particular, transgenic plants expressing a dsRNA targeting a gene from a root-knot nematode have been obtained in *Arabidopsis* (Huang *et al.,* 2006), tomato (Charlton *et al.,* 2010), tobacco (Yadav B.C. *et al.,* 2006) or soybean (Ibrahim *et al.,* 2011). Transgenic plants expressing a dsRNA targeting a gene from cyst nematode have been obtained in *Arabidopsis* (Patel *et al.,* 2008 and 2010; Sindhu *et al.,* 2009).

Advantageously, the dsRNA targeting a gene as defined above comprises a first RNA sequence of 18 to 25 nucleotides, preferably of 19 to 24 nucleotides, more preferably of 19, 20 or 21 nucleotides, wherein said first RNA sequence is encoded by a 18 to 25 nucleotide sequence, preferably of 19 to 24 nucleotide sequence, more preferably by a 19, 20 or 21 nucleotide sequence, having at least 90% identity, and by order of increasing preference, at least 95%, 96%, 97%, 98%, or 99 % identity, and preferably 100% identity, with a 18 to 25 contiguous nucleotide fragment, preferably of 19 to 24 contiguous nucleotide fragment, more preferably by a 19, 20 or 21 contiguous nucleotide fragment of said target gene, and a second RNA sequence that is the complementary of said first RNA sequence, said first and second sequences being in opposite orientations, and optionally linked with a spacer sequence (a linker region).

According to another preferred embodiment of the invention the dsRNA targeting a gene as defined above produces (through cleavage by the DICER RNase into a plant cell) a siRNA comprising a sense strand and an antisense strand, wherein the sense strand comprises a 15 to 30 nucleotide sequence having at least 90% identity, or by order of increasing preference at least 95%, 96%, 97%, 98% or 99% identity, or having 100% identity, with a 15 to 30 contiguous nucleotide fragment of said gene, and the antisense strand comprises a 15 to 30 nucleotide sequence that is complementary to the sense strand.

Advantageously, the sense strand of the siRNA comprises a 19 to 24 nucleotide sequence, preferably a 19, 20 or 21 nucleotide sequence, that is complementary to a 19 to 24 nucleotide sequence, preferably a 19, 20 or 21 nucleotide sequence, respectively of the antisense strand of said siRNA.

The siRNA can comprise blunt end(s) or 1 to 10 overhanging nucleotides at the 5' and/or 3' end of each strand.

Advantageously, the siRNA comprises two overhanging thymine nucleotides (TT) at the 3' end of each strand.

The sense strand and the antisense strand of the siRNA can be connected together via a 1 or more (e.g., 2, 3, 4, 5, 6, 7, 8, 9, 10, etc.) nucleotide linker.

According to another preferred embodiment of the invention, the dsRNA targeting a gene as defined above produces (through cleavage by the Dicer-Like1 RNase inside the nucleus plant cell) an amiRNA consisting of a 19 to 24 nucleotide, preferably a 21 nucleotide single stranded RNA having 0 to 5 mismatch, preferably 0 mismatch (*i.e*., 100% identity) with a 19 to 24 contiguous nucleotide fragment, preferably a 21 contiguous nucleotide fragment, of the mRNA encoded by said gene.

In a particular embodiment:
- the dsRNA targeting the gene encoding SEQ ID NO. 1 (Minc01632) comprises a sense strand encoded by the nucleotide sequence SEQ ID NO. 79 (GGTTCAGAGATTCCTTGTC) and an antisense strand encoded by the nucleotide sequence SEQ ID NO. 80 (GACAAGGAATCTCTGAACC),
- the dsRNA targeting the gene encoding SEQ ID NO. 2 (Minc02483) comprises a sense strand encoded by the nucleotide sequence SEQ ID NO. 81 1 (TTGACAACCAATTGCCGTT) and an antisense strand encoded by the nucleotide sequence SEQ ID NO. 82 (AACGGCAATTGGTTGTCAA),
- the dsRNA targeting the gene encoding SEQ ID NO. 3 (Minc05001) comprises a sense strand encoded by the nucleotide sequence SEQ ID NO. 83 (ATGACGGCATTATCCGTTC) and an antisense strand encoded by the nucleotide sequence SEQ ID NO. 84 (GAACGGATAATGCCGTCAT),
- the dsRNA targeting the gene encoding SEQ ID NO. 4 (Minc03866) comprises a sense strand encoded by the nucleotide sequence SEQ ID NO. 85 (GCAGGAGTTGTATGCAAGC) and an antisense strand encoded by the nucleotide sequence SEQ ID NO. 86 (GCTTGCATACAACTCCTGC),
- the dsRNA targeting the gene encoding SEQ ID NO. 5 (Minc09526) comprises a sense strand encoded by the nucleotide sequence SEQ ID NO. 87 (GAGATAGAAGTGAGTGTAC) and an antisense strand encoded by the nucleotide sequence SEQ ID NO. 88 (GTACACTCACTTCTATCTC),
- the dsRNA targeting the gene encoding SEQ ID NO. 6 (Minc17713) comprises a sense strand encoded by the nucleotide sequence SEQ ID NO. 89 (TCCACATTATCGCGCAGGA) and an antisense strand encoded by the nucleotide sequence SEQ ID NO. 90 (TCCTGCGCGATAATGTGGA),
- the dsRNA targeting the gene encoding SEQ ID NO. 7 (Minc03313) comprises a sense strand encoded by the nucleotide sequence SEQ ID NO. 91 (CGCTCCATGCATTAGGTTC) and an antisense strand encoded by the nucleotide sequence SEQ ID NO. 92 (GAACCTAATGCATGGAGCG),
- the dsRNA targeting the gene encoding SEQ ID NO. 8 (Minc08335) comprises a sense strand encoded by the nucleotide sequence SEQ ID NO. 93 (CGACAGCGAAGAAGACTAC) and an antisense strand encoded by the nucleotide sequence SEQ ID NO. 94 (GTAGTCTTCTTCGCTGTCG),
- the dsRNA targeting the gene encoding SEQ ID NO. 9 (Minc08013) comprises a sense strand encoded by the nucleotide sequence SEQ ID NO. 95 (AGAACGGATCAATCGAGCA) and an antisense strand encoded by the nucleotide sequence SEQ ID NO. 96 (TGCTCGATTGATCCGTTCT),
- the dsRNA targeting the gene encoding SEQ ID NO. 10 (Minc00801) comprises a sense strand encoded by the nucleotide sequence SEQ ID NO. 97 (ACGTTCACAGAACTCTTAC) and an antisense strand encoded by the nucleotide sequence SEQ ID NO. 98 (GTAAGAGTTCTGTGAACGT),
- the dsRNA targeting the gene encoding SEQ ID NO. 11 (Minc12224) comprises a sense strand encoded by the nucleotide sequence SEQ ID NO. 99 (GACGAAGCTGGTCAATGCT) and an antisense strand encoded by the nucleotide sequence SEQ ID NO. 100 (AGCATTGACCAGCTTCGTC),
- the dsRNA targeting the gene encoding SEQ ID NO. 12 (Minc14652) comprises a sense strand encoded by the nucleotide sequence SEQ ID NO. 101 (CAAGCAATGCTGTTCATGC) and an antisense strand encoded by the nucleotide sequence SEQ ID NO. 102 (GCATGAACAGCATTGCTTG),
- the dsRNA targeting the gene encoding SEQ ID NO. 13 (Minc10706) comprises a sense strand encoded by the nucleotide sequence SEQ ID NO. 103 (TGGCATTCAAGTTGGTCCT) and an antisense strand encoded by the nucleotide sequence SEQ ID NO. 104 (AGGACCAACTTGAATGCCA),
- the dsRNA targeting the gene encoding SEQ ID NO. 14 (Minc08014) comprises a sense strand encoded by the nucleotide sequence SEQ ID NO. 105 (ATCCGGCAAGTCTGCTTGT) and an antisense strand encoded by the nucleotide sequence SEQ ID NO. 106 (ACAAGCAGACTTGCCGGAT),
- the dsRNA targeting the gene encoding SEQ ID NO. 15 (Minc17987) comprises a sense strand encoded by the nucleotide sequence SEQ ID NO. 107 (AGGACAACAAGGAGGCATT) and an antisense strand encoded by the nucleotide sequence SEQ ID NO. 108 (AATGCCTCCTTGTTGTCCT),
- the dsRNA targeting the gene encoding SEQ ID NO. 16 (Minc07817) comprises a sense strand encoded by the nucleotide sequence SEQ ID NO. 109 (GGATGAACCGTTGGAACAA) and an antisense strand encoded by the nucleotide sequence SEQ ID NO. 110 (TTGTTCCAACGGTTCATCC).

The plant-parasitic nematode is preferably a root-knot nematode, more preferably of the genus *Meloidogyne.*

Advantageously, the nematode is selected from the group consisting of *M javanica, M. arenaria, M. incognita* and *M. hapla,* preferably *M incognita.*

The present invention applies to monocot- or dicotyledon plants of agronomical or ornamental interest, such as alfalfa, banana, barley, beans, cotton, cucurbits, flax, fruit trees, maize, oats, peas, potato, rape, rice, rye, sorghum, soybean, sugar cane, sugarbeet, sunflower, tobacco, tomato, triticale, wheat.

The present invention also provides an isolated RNA for silencing a gene from a nematode encoding an effector-like protein or a transcription factor as defined above, comprising or consisting of a 15 to 30 nucleotide, preferably 18 to 25 nucleotide, more preferably 19 to 21 nucleotide RNA having at least 90% identity, or by order of increasing preference at least 91%, 92%, 93%, 94%, 95%, 96%, 97%, 98% or 99% identity, and preferably having 100% identity, with respectively a 15 to 30 contiguous nucleotide fragment, preferably 18 to 25 contiguous nucleotide fragment, more preferably 19 to 21 contiguous nucleotide fragment of said target gene.

Preferably, said target gene encodes a protein selected from the group consisting of SEQ ID NO. 1 to SEQ ID NO. 16.

In particular, said isolated RNA consists of a dsRNA as described above, preferably an isolated siRNA as described above.

By way of example, said isolated dsRNA targeting a gene from a nematode encoding an effector-like protein or a transcription factor, selected from the group consisting of:
- a dsRNA targeting the gene encoding SEQ ID NO. 1 (Minc01632) comprises a sense strand encoded by the nucleotide sequence SEQ ID NO. 79 (GGTTCAGAGATTCCTTGTC) and an antisense strand encoded by the nucleotide sequence SEQ ID NO. 80 (GACAAGGAATCTCTGAACC),
- a dsRNA targeting the gene encoding SEQ ID NO. 2 (Minc02483) comprises a sense strand encoded by the nucleotide sequence SEQ ID NO. 81 (TTGACAACCAATTGCCGTT) and an antisense strand encoded by the nucleotide sequence SEQ ID NO. 82 (AACGGCAATTGGTTGTCAA),
- a dsRNA targeting the gene encoding SEQ ID NO. 3 (Minc05001) comprises a sense strand encoded by the nucleotide sequence SEQ ID NO. 83 (ATGACGGCATTATCCGTTC) and an antisense strand encoded by the nucleotide sequence SEQ ID NO. 84 (GAACGGATAATGCCGTCAT),
- a dsRNA targeting the gene encoding SEQ ID NO. 4 (Minc03866) comprises a sense strand encoded by the nucleotide sequence SEQ ID NO. 85 (GCAGGAGTTGTATGCAAGC) and an antisense strand encoded by the nucleotide sequence SEQ ID NO. 86 (GCTTGCATACAACTCCTGC),
- a dsRNA targeting the gene encoding SEQ ID NO. 5 (Minc09526) comprises a sense strand encoded by the nucleotide sequence SEQ ID NO. 87 (GAGATAGAAGTGAGTGTAC) and an antisense strand encoded by the nucleotide sequence SEQ ID NO. 88 (GTACACTCACTTCTATCTC),
- a dsRNA targeting the gene encoding SEQ ID NO. 6 (Minc17713) comprises a sense strand encoded by the nucleotide sequence SEQ ID NO. 89 (TCCACATTATCGCGCAGGA) and an antisense strand encoded by the nucleotide sequence SEQ ID NO. 90 (TCCTGCGCGATAATGTGGA),
- a dsRNA targeting the gene encoding SEQ ID NO. 7 (Minc03313) comprises a sense strand encoded by the nucleotide sequence SEQ ID NO. 91 (CGCTCCATGCATTAGGTTC) and an antisense strand encoded by the nucleotide sequence SEQ ID NO. 92 (GAACCTAATGCATGGAGCG),
- a dsRNA targeting the gene encoding SEQ ID NO. 8 (Minc08335) comprises a sense strand encoded by the nucleotide sequence SEQ ID NO. 93 (CGACAGCGAAGAAGACTAC) and an antisense strand encoded by the nucleotide sequence SEQ ID NO. 94 (GTAGTCTTCTTCGCTGTCG),
- a dsRNA targeting the gene encoding SEQ ID NO. 9 (Minc08013) comprises a sense strand encoded by the nucleotide sequence SEQ ID NO. 95 (AGAACGGATCAATCGAGCA) and an antisense strand encoded by the nucleotide sequence SEQ ID NO. 96 (TGCTCGATTGATCCGTTCT),
- a dsRNA targeting the gene encoding SEQ ID NO. 10 (Minc00801) comprises a sense strand encoded by the nucleotide sequence SEQ ID NO. 97 (ACGTTCACAGAACTCTTAC) and an antisense strand encoded by the nucleotide sequence SEQ ID NO. 98 (GTAAGAGTTCTGTGAACGT),
- a dsRNA targeting the gene encoding SEQ ID NO. 11 (Minc12224) comprises a sense strand encoded by the nucleotide sequence SEQ ID NO. 99 (GACGAAGCTGGTCAATGCT) and an antisense strand encoded by the nucleotide sequence SEQ ID NO. 100 (AGCATTGACCAGCTTCGTC),
- a dsRNA targeting the gene encoding SEQ ID NO. 12 (Minc14652) comprises a sense strand encoded by the nucleotide sequence SEQ ID NO. 101 (CAAGCAATGCTGTTCATGC) and an antisense strand encoded by the nucleotide sequence SEQ ID NO. 102 (GCATGAACAGCATTGCTTG),
- a dsRNA targeting the gene encoding SEQ ID NO. 13 (Minc10706) comprises a sense strand encoded by the nucleotide sequence SEQ ID NO. 103 (TGGCATTCAAGTTGGTCCT) and an antisense strand encoded by the nucleotide sequence SEQ ID NO. 104 (AGGACCAACTTGAATGCCA),
- a dsRNA targeting the gene encoding SEQ ID NO. 14 (Minc08014) comprises a sense strand encoded by the nucleotide sequence SEQ ID NO. 105 (ATCCGGCAAGTCTGCTTGT) and an antisense strand encoded by the nucleotide sequence SEQ ID NO. 106 (ACAAGCAGACTTGCCGGAT),
- a dsRNA targeting the gene encoding SEQ ID NO. 15 (Minc17987) comprises a sense strand encoded by the nucleotide sequence SEQ ID NO. 107 (AGGACAACAAGGAGGCATT) and an antisense strand encoded by the nucleotide sequence SEQ ID NO. 108 (AATGCCTCCTTGTTGTCCT),
a dsRNA targeting the gene encoding SEQ ID NO. 16 (Minc07817) comprises a sense strand encoded by the nucleotide sequence SEQ ID NO. 109 (GGATGAACCGTTGGAACAA) and an antisense strand encoded by the nucleotide sequence SEQ ID NO. 110 (TTGTTCCAACGGTTCATCC).

The present invention also provides a composition comprising an isolated RNA for silencing a gene from a nematode encoding an effector-like protein or a transcription factor as defined above and at least one suitable carrier, excipient or diluents.

This composition can be in the form suitable for ingestion by a plant-parasitic nematode, e.g., in solid, liquid or gel form.

This composition can further comprise a pesticide, herbicide or insecticide agent.

Advantageously, this composition is suitable for reducing the plant-parasitic nematode infestation level in a plant.

The present invention also provides the use of an isolated RNA or a composition according to the present invention for reducing the plant-parasitic nematode infestation level in a plant.

The present invention also provides a method for reducing the plant-parasitic nematode infestation level in a plant comprising contacting said plant-parasitic nematode with an effective amount of an isolated RNA or a composition according to the present invention.

The present invention also provides a chimeric DNA construct encoding a dsRNA for silencing a target gene as defined above, comprising:
- a promoter functional in a plant cell;
- a first DNA sequence of 18 to 25 bp, preferably of 19 to 24 bp, more preferably of 19, 20 or 21 bp, consisting of a fragment of a cDNA encoded by said target gene, or having at least 90% identity, and by order of increasing preference, at least 95%, 96%, 97%, 98%, or 99% identity, and preferably 100% identity, with said fragment, said DNA sequence being placed under transcriptional control of said promoter;
- a second DNA sequence that is the complementary of said first DNA, said first and second sequences being in opposite orientations;
- a spacer sequence separating said first and second sequence, such that these first and second DNA sequences are capable, when transcribed, of forming a single double-stranded RNA molecule.

The spacer can be a random fragment of DNA.

A large choice of promoters suitable for expression of heterologous genes in plants is available in the art.

They can be obtained for instance from plants, plant viruses, or bacteria such as those of the *Agrobacterium* lineage. They include constitutive promoters, *i.e.* promoters which are active in most tissues and cells and under most environmental conditions, tissue or cell specific promoters which are active only or mainly in certain tissues (*e.g.* root cells) or certain cell types (*e.g.* giant cells), and inducible promoters that are activated by physical or chemical stimuli, such as those resulting from nematode infection.

One can use constitutive promoters that are commonly used in plant cells such as the cauliflower mosaic virus (CaMV) 35S promoter, the Nos promoter and the rubisco promoter.

However, the most suitable promoters for silencing a target gene as defined above in a plant in order to reduce the plant-parasitic nematode infestation level are promoters that are tissue-specific, having a preferential or exclusive expression in the tissues infected by the nematodes, in particular the giant cells, and/or promoters that are inducible by nematode infection, *i.e.* promoters that drive an increased level of expression in cells infected by nematodes, when compared to non-infected cells. Said promoters may be naturally occurring promoter, may comprise a nematode responsive element isolated from a naturally occurring promoter, or may be a synthetic promoter.

Non-limitative examples of preferred promoters for carrying out the invention include:
- the elongation specific endo-1,4-β-glucanase (cell) promoter of *Arabidopsis thaliana,* which is described in U.S. Patent No. 7,119,254 as being up-regulated in root-knot nematode feeding cells;
- the promoter of the POX gene ofA. *thaliana* (Vercauteren *et al.,* 2001);
- the promoter of the Trypsin inhibitor gene of *A. thaliana* (Vercauteren *et al.,* 2001);
- the promoter of the endomembrane protein gene of *A. thaliana* (Vercauteren *et al.,* 2001);
- the promoter of the DAP decarboxylase gene of *A. thaliana* (Vercauteren *et al.,* 2001);
- the promoter of the WRKY23 (Att0001) gene of *A. thaliana* (Barthels *et al.,* 1997);
- the promoter of the *AtFH6* gene (Favery *et al.,* 2004), also disclosed in International Application WO 2005/063989 (SEQ ID NO: 1 of WO 2005/063989).

The DNA constructs of the invention generally also include a transcriptional terminator (for instance the 35S transcriptional terminator, or the nopaline synthase (Nos) transcriptional terminator).

These DNA constructs can be obtained and introduced in a host cell or organism by the well-known techniques of recombinant DNA and genetic engineering. The choice of the recombinant vector depends on the intended host and on the intended method of transformation of said host. A variety of methods for genetic transformation of plant cells or plants are available in the art for many plant species, dicotyledons or monocotyledons. By way of non-limitative examples, one can mention virus mediated transformation, transformation by microinjection, by electroporation, microprojectile mediated transformation, *Agrobacterium* mediated transformation, and the like.

The invention also comprises a plant cell or a plant genetically modified by a construct of the invention. The polynucleotide may be transiently expressed; it can also be incorporated in a stable extrachromosomal replicon, or integrated in the chromosome.

In particular, the invention relates to a transgenic plant containing a transgene comprising a chimeric DNA construct of the invention. The expression of said chimeric DNA construct, resulting in decreased level of expression of a target gene as defined above, provides to said transgenic plant a reduction of the plant-parasitic nematode infestation level when compared with a plant devoid of said transgene.

The invention also encompasses isolated organs or tissues of said plants (such as seeds, leafs, flowers, roots).

The present invention also provides a method for screening a candidate compound that can be used for reducing the plant-parasitic nematode infestation level in a plant comprising the steps of:
- contacting a candidate compound with a plant-parasitic nematode expressing an effector-like protein or a transcription factor as defined above;
- determining whether the candidate compound modulates the expression of the gene encoding said effector-like protein or said transcription factor;
wherein said candidate compound that decreases the expression of the gene encoding said effector-like protein or said transcription factor is identified as a candidate compound capable of reducing the plant-parasitic nematode infestation level in a plant.

The step of contacting a candidate compound with a plant-parasitic nematode can be carried out by incubating the nematode in a solution containing candidate compound using the soaking method.

The plant-parasitic nematode is preferably a root-knot nematode, more preferably of the genus *Meloidogyne.* Advantageously, it is selected from the group consisting of *M. javanica, M. arenaria, M. incognita* and *M. hapla,* preferably *M. incognita.*

Methods for determining the expression of a gene in a nematode are well known to a person skilled in the art. For instance, expression of a gene coding for an effector-like protein or a transcription factor as defined above can be determined by quantifying the effector-like protein or the transcription factor as defined above in a cell lysate, by means of antibodies specific for said effector-like protein or transcription factor, chromatography techniques, mass spectrometry techniques. Expression of a gene encoding an effector-like protein or a transcription factor as defined above can also be determined by quantifying mRNA encoded by said effector-like gene or transcription factor gene, including quantitative RT-PCR using primers specifically hybridizing to said mRNA.

The candidate compound can be a dsRNA, a siRNA, a hpRNA, an amiRNA or organic chemicals.

Foregoing and other objects and advantages of the invention will become more apparent from the following detailed description and accompanying drawings. It is to be understood however that this foregoing detailed description is exemplary only and is not restrictive of the invention.

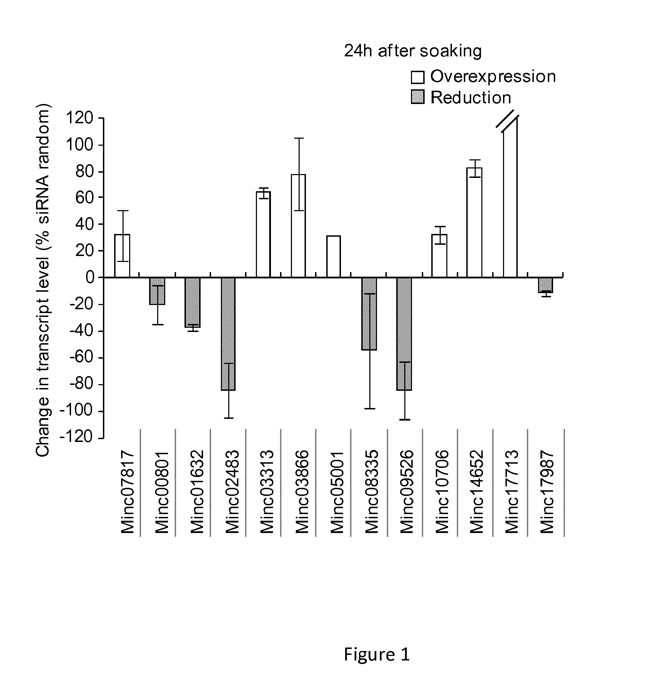

**Figure 1****:** Transcript abundance percentage change in *M incognita* infective juveniles soaked in a siRNA solution relative to control. siRNAs induced significant change in the targeted transcripts expression level. Transcript level for each of the targeted gene was measured by qPCR 24h after soaking treatment and compared to their transcript level in control sample treated with siRNA targeting no sequence in the *M incognita* genome.

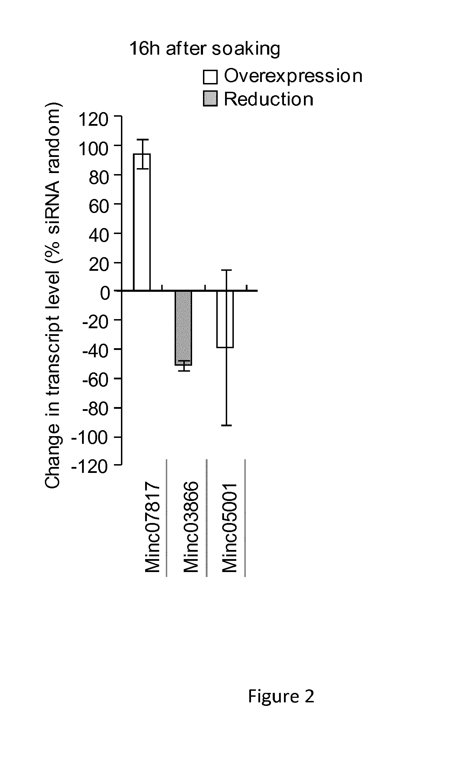

**Figure 2****:** Transcript level measured 16h after soaking to test bounce effect. qPCR analysis was performed 16h after soaking on Minc07817, Minc03866 and Minc05001 treated with siRNA.

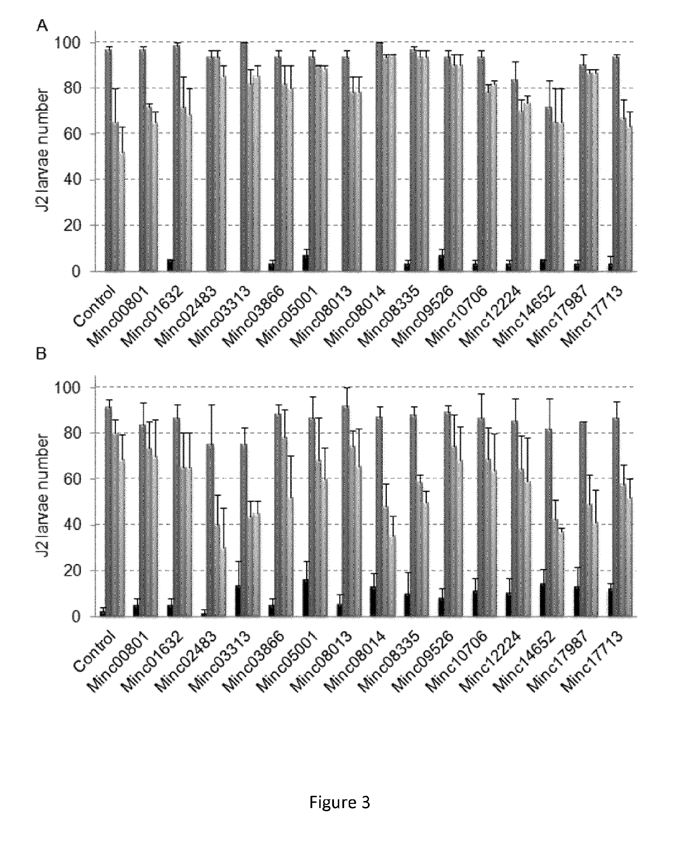

**Figure 3****:** Effect of siRNA on nematode mobility / viability. Controls are J2 larvae soaked with siRNA targeting no sequence in the *M incognita* genome, accession numbers indicate *M incognita* genes targeted by siRNAs. Viability was assessed 1h **(A)** and 16h **(B)** after soaking by counting the number of dead nematodes (1^{st} bars = far left bars), the number of individuals with movements restricted to extremities (2^{nd} bars), movements on the whole length of the body (3^{rd} bars) and fast undulations (4^{th} bars = far right bars) under microscope observation. Three independent replicates were analyzed. Error bars represent standard error of the mean.

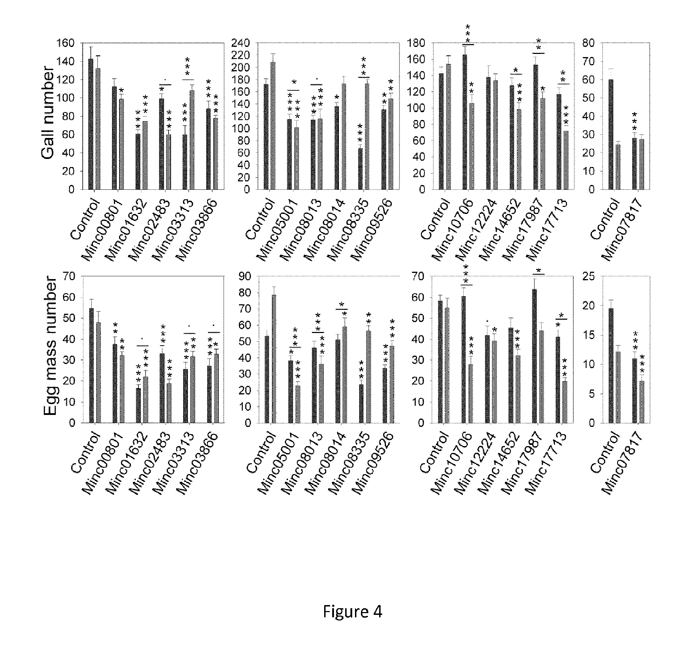

**Figure 4****:** Effect of siRNA on nematode infestation. Controls are J2 larvae soaked with siRNA targeting no sequence in the *M incognita* genome, accession numbers indicate *M incognita* genes targeted by siRNAs. Infection tests were performed in duplicate represented as left bars for the first replicate and right bars for the second replicate. Variations of number of galls and of numbers of egg masses, measured six weeks after inoculation, are represented in the top panels and in the bottom panel, respectively. Error bars were calculated by standard error of the mean (SEM). P-Value above each bar indicates measures statistically different from controls. A significant change between the two duplicates is represented by p-values above a line spanning duplicates. P-value signification codes are as follows: 0.0001 '***', 0.001 '**', 0.01 '*', 0.05 '.'.

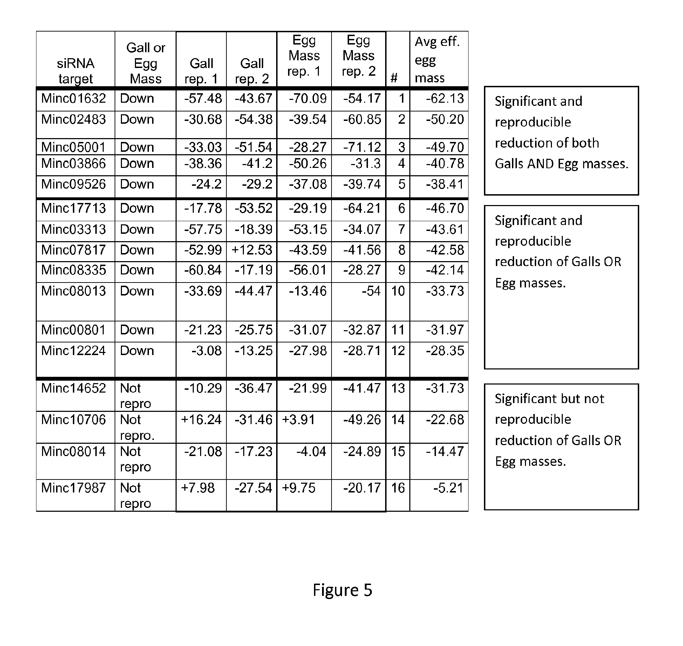

**Figure 5****:** Table showing the percent reduction in number of egg masses and galls after siRNA treatment for the 16 target genes.

### EXAMPLE: EXPERIMENTAL VALIDATION OF THE 16 TARGET GENES BY M. INCOGNITA INFESTATION ON TOMATO PLANTS AFTER GENE SILENCING

### Materials and Methods

### siRNA design and siRNA treatment for viability tests and infection assays

siRNAs (see Table 1 below) were prepared using the Silencer® siRNA Construction Kit (AM1620, Ambion®, Austin, TX) and siRNA yields were determined using a NanoDrop 2000 spectrophotometer (NanoDrop® Products, Wilmington, DE).

**Table 1. Small interfering RNAs used to silence M. incognita target genes**

| siRNA target | sequence 5'-> 3' | SEQ ID NO. |
|---|---|---|
| Minc00801 | F: AAACGTTCACAGAACTCTTACCCTGTCTC | 111 |
| | R: AAGTAAGAGTTCTGTGAACGTCCTGTCTC | 112 |
| Minc01632 | F:AAGGTTCAGAGATTCCTTGTCCCTGTCTC | 113 |
| | R:AAGACAAGGAATCTCTGAACCCCTGTCTC | 114 |
| Minc02483 | F:AATTGACAACCAATTGCCGTTCCTGTCTC | 115 |
| | R:AAAACGGCAATTGGTTGTCAACCTGTCTC | 116 |
| Minc03313 | F:AACGCTCCATGCATTAGGTTCCCTGTCTC | 117 |
| | R:AAGAACCTAATGCATGGAGCGCCTGTCTC | 118 |
| Minc03866 | F:AAGCAGGAGTTGTATGCAAGCCCTGTCTC | 119 |
| | R:AAGCTTGCATACAACTCCTGCCCTGTCTC | 120 |
| Minc05001 | F:AAATGACGGCATTATCCGTTCCCTGTCTC | 121 |
| | R:AAGAACGGATAATGCCGTCATCCTGTCTC | 122 |
| Minc08013 | F:AAAGAACGGATCAATCGAGCACCTGTCTC | 123 |
| | R:AATGCTCGATTGATCCGTTCTCCTGTCTC | 124 |
| Minc08014 | F:AAATCCGGCAAGTCTGCTTGTCCTGTCTC | 125 |
| | R:AAACAAGCAGACTTGCCGGATCCTGTCTC | 126 |
| Minc08335 | F:AACGACAGCGAAGAAGACTACCCTGTCTC | 127 |
| | R:AAGTAGTCTTCTTCGCTGTCGCCTGTCTC | 128 |
| Minc09526 | F:AAGAGATAGAAGTGAGTGTACCCTGTCTC | 129 |
| | R:AAGTACACTCACTTCTATCTCCCTGTCTC | 130 |
| Minc10706 | F:AATGGCATTCAAGTTGGTCCTCCTGTCTC | 131 |
| | R:AAAGGACCAACTTGAATGCCACCTGTCTC | 132 |
| Minc12224 | F:AAGACGAAGCTGGTCAATGCTCCTGTCTC | 133 |
| | R:AAAGCATTGACCAGCTTCGTCCCTGTCTC | 134 |
| Minc14652 | F:AACAAGCAATGCTGTTCATGCCCTGTCTC | 135 |
| | R:AAGCATGAACAGCATTGCTTGCCTGTCTC | 136 |
| Minc17713 | F:AATCCACATTATCGCGCAGGACCTGTCTC | 137 |
| | R:AATCCTGCGCGATAATGTGGACCTGTCTC | 138 |
| Minc17987 | F:AAAGGACAACAAGGAGGCATTCCTGTCTC | 139 |
| | R:AAAATGCCTCCTTGTTGTCCTCCTGTCTC | 140 |
| Control siRNA | F: AAGTACATTTAACGCCAGCCTCCTGTCTC | 141 |
| | R: AAAGGCTGGCGTTAAATGTACCCTGTCTC | 142 |

| | | |
|---|---|---|
| F = Forward oligomer, R= Reverse oligomer. | | |

For control it was used a siRNA designed to have no sequence similarity in the *M incognita* genome (Dalzell *et al.,* 2010a) as confirmed by blastn searches. siRNAs were conserved at -80°C in 2 µg aliquots until use. Eggs of *M incognita* were collected from tomato plants (*Solanum esculentum* cv. St Pierre) cultured in greenhouse. Eggs were collected as described by Rosso *et al.* (1999) and J2s were hatched in water. About 10,000 J2s were soaked in 40 µl final volume of spring water in the presence of 0.05 mg/ml siRNA for 1 hour. Worms were washed twice with water by centrifugation at 10,000 g for 1 min and suspended in 100 µl of water. For viability assay, the number of dead J2s was counted under microscope observation on 100 individuals. It was also reported movement quality and rapidity for 25 individuals. For movement quality it was observed extremity and whole length movement and the quality of movement was noticed by rapidity of undulation or absence of undulation. For infection assays, roots of 24 tomato plants aged of four weeks were each inoculated with 250 *M. incognita* infective J2 larvae, previously incubated over night in a water solution containing siRNAs as described by Arguel *et al.* (2012). Two replicates of the infection assays were performed at three weeks intervals. Galls and egg masses were counted six weeks after inoculation. Statistical analyses were performed by an ANOVA test using the R software.

### Quantitative-PCR

RNA was isolated from approximately 500 J2s soaked for 1hour in 0.05 mg/ml siRNA and incubated for 16h or 24h in water, using TRIzol® Reagent (Invitrogen, Carlsbad, CA, USA). Purity and concentration of the RNA was determined on a NanoDrop_2000 spectrophotometer (NanoDrop Products, Wilmington, DE). Reverse transcription was carried out using the iScript cDNA Synthesis Kit (Bio-Rad laboratories, Marnes la Coquette, France). The primers for qPCR were determined using primer3 software (Rozen *et al.,* 2000) and synthesised by Eurogentec (Seraing, Belgium) (see Table 2 below).

**Table 2. Sequences of oligonucleotides used for qPCRs for M. incognita target and control genes**

| target gene | primer name | primer sequence (5' → 3') | SEQ ID NO. | amplicon length (bp) |
|---|---|---|---|---|
| *Minc07817* | N48b | ATGGCTACAATACCTCCAAATAAT | 143 | 124 |
| | N49b | TAATTGTCTGTTCTGTCTGCCAAG | 144 | |
| *Minc00801* | NF64 | CTATGGAGATGAGGCAGGACAT | 145 | 248 |
| | NR65 | GTGAACGTTTCTTGCGGTATC | 146 | |
| *Minc01632* | NF66 | ATTGCTCTTTTGTGTTTAGAAATGG | 147 | 155 |
| | NR67 | CAAGGAATCTCTGAACCTTTGAAAT | 148 | |
| *Minc02483* | F68 | CGAAATTTAATGCGGATTGG | 149 | 167 |
| | R69 | TGTGGGCGATATGGCTTAAT | 150 | |
| *Minc03313* | NF70 | TGCCCACAAAAATATAGAAGAAAAT | 151 | 133 |
| | NR71 | AGATAACCACTGCTTTCCTCCATT | 152 | |
| *Minc03866* | F72 | AGCTGATCGTGCTGTCATTG | 153 | 127 |
| | R73 | ACAGAAAGACGCGCAATCAC | 154 | |
| *Minc05001* | NF74 | TGTTACTGTCCAATCAGAAAATGAA | 155 | 106 |
| | NR75 | ACATATCCCATGTTGTGGACTAACT | 156 | |
| *Minc08013* | F76b | AGATCCACCCTTAGAAAGCCATAG | 157 | 99 |
| | R77b | TTGTCGTAAAGTCTTGTGGTGATAG | 158 | |
| *Minc08014* | F78 | TCCAGCGTTCTATGATTGTAATTG | 159 | 108 |
| | R79 | CACTGCTTAGGAATATCAATGCTC | 160 | |
| *Minc08335* | NF80 | TTATTATTAATTTCCCAAAGCCAATC | 161 | 159 |
| | FR81 | TATATTTTGTTCCCCTTCCTCCTTA | 162 | |
| *Minc09526* | NF82 | AGTGAGTGTACAGGATCAACATCTG | 163 | 168 |
| | NR83 | GCTGACATTTGCTGTCAGAGTATTA | 164 | |
| *Minc10706* | F84 | CAGGTCTAACAGCCTCCTCAA | 165 | 135 |
| | R85 | CAGTTCCTTGTGGCATGAAT | 166 | |
| *Minc12224* | F86 | TGGAGCTGTTACACCAGCAG | 167 | 225 |
| | R87 | AAGAATGACCGTTGGAGTGC | 168 | |
| *Minc14652* | F88 | TATCAACCAGCAGCCACTCA | 169 | 230 |
| | R89 | CGTTGTCTGTGCCTTGAAGA | 170 | |
| *Minc17713* | F92 | TACACCGGTCAGCAACATTC | 171 | 89 |
| | R93 | AGATTCCTTTGCCCTTCTGG | 172 | |
| *Minc17987* | F90 | CTCCAGGTGAGGATGACGAG | 173 | 238 |
| | R91 | ATGCCTGGTTGTCCAGTACC | 174 | |
| *GAPDH** | GAP17 | GGTTATCTCAGCTCCGTCTGC | 175 | 138 |
| | GAP18 | TAACCTTCGCAAGAGGAGCA | 176 | |
| *ACT** | ACT23 | AAGACGAAGCAGCTGTAGCC | 177 | 230 |
| | ACT24 | GGTGTTACGCACACAGTTCC | 178 | |
| *Mi-pg-1** | PolyF4 | ATGAGTCAAACAATAACACCCCC | 179 | 244 |
| | PolyR2 | AATATATAAGGGCCAGACAAAG | 180 | |

| | | | | |
|---|---|---|---|---|
| *Accession numbers of control genes are as follow, *ACT:* actin (Minc06773), *GAPDH:* glyceraldehyde 3-phosphate dehydrogenase (Minc10963) *Mi-pg-1:* polygalacturonase (Minc18543). T°C: annealing temperature used in PCR and quantitative-PCR run. Amplicon size in base pairs (bp) attempted while amplification with specific primer couple. | | | | |

The cDNA was diluted 10 times, and 5µL was used per PCR reaction. Seven and a half microliters of 2X SYBR® Green Master Mix (Eurogentec, Liege, Belgium), 0.2 µL each of 100 µM of forward and reverse primer, and 4.6 µL of water were added to the cDNA. Thermocycling was carried out with one cycle at 95°C for 15 min, followed by 40 cycles of 95°C for 15 sec and 56°C for 1 min and 72°C for 30 sec. The dissociation curve of the final products was checked to ascertain the presence of a single amplification product. qPCR was performed on triplicate samples of each cDNA. Among the three tested candidate reference genes, *i.e., M. incognita* polygalacturonase *(Mi-pg-1,* Minc18543), Glyceraldehyde 3-phosphate dehydrogenase (GAPDH, Minc10963) and Actin α (Minc06773), GAPDH was determined as the most stable reference using Genorm algorithm (Vandesompele J. *et al.,* 2002) and was selected as reference gene. For normalization, the threshold cycle values of GAPDH amplifications (CT_{GAPDH}) were subtracted from the threshold cycle values of the analyzed genes (CTexp). Transcript levels in arbitrary units (AU) were calculated with the formula: AU= 100 x 2 ^{(CTexp - CTGAPDH)}. Figure 1 shows results from two independent replicates.

### Transcript analyses by in situ hybridizations

Sense and antisense probes were synthesized from each target gene with specific primers designed with Primer3 software (Rozen *et al.,* 2000) and synthesised by Eurogentec (Seraing, Belgium) (see Table 1 above). As controls, it was used the polygalacturonase *Mi-pg-1* (Minc18543) gene for esophageal gland-specific labeling and the *GAPDH* Minc10963 gene for ubiquitous labeling. *In situ* hybridizations were conducted as described previously (Jaubert *et al.,* 2002). 10,000 J2s were hybridized with DIG-labeled specific probes at 40°C over night for each target transcript.

### Results

### Effect of siRNA on the 16 target genes

To test whether the designed siRNA interfere with the expression of each of the 16 target genes (15 effector-like proteins, namely Minc00801, Minc01632, Minc02483, Minc03313, Minc03866, Minc05001, Minc08013, Minc08014, Minc08335, Minc09526, Minc10706, Minc12224, Minc14652, Minc17713 and Minc17987, and one transcription factor, namely Minc07817), it was performed Real-Time quantitative PCR (qPCR) experiments on soaked J2s.

Six siRNAs induced significant reduction in the corresponding targeted transcripts (Minc00801, Minc01632, Minc02483, Minc08335, Minc09526, Minc17987) 24h after soaking treatment, compared to their expression level in control sample (Figure 1). On the other hand, Minc08413, Minc08014 and Minc12224 siRNAs induced a silencing that was not reproducible between independent qPCR replicate experiments. Surprisingly, 7 siRNAs (Minc03313, Minc03866, Minc05001, Minc10706, Minc14652, Minc17713, Minc07817) induced significant and reproducible increase in transcript abundance when qPCR was performed 24h after soaking. A similar "bounce" effect, that could be due to a response of the nematode RNAi machinery to increased siRNA quantities within the cells, had already been reported in PPN after gene silencing (Dubreuil *et al.,* 2007; Bakhetia *et al.,* 2008; Dalzell *et al.,* 2010b). Therefore, it was hypothesized that the transcripts may be present in higher quantity after an initial early silencing. To test this "bounce" hypothesis, it was performed qPCR analysis 16h after soaking on Minc07817, Minc03866 and Minc05001. Confirming siRNA knockdown and bounce effect, Minc03866 was significantly silenced 16h after soaking. However, Minc05001 expression was reduced but not significantly and Minc07817 expression was still higher than control (Figure 2). Overall, the qPCR results indicate that the designed siRNAs, were efficient to knockdown 7 out of the 16 target genes at the tested time points.

### Effect of siRNA soaking on nematode viability and mobility

It was tested whether treatment with siRNAs had effect on nematode viability or mobility, by counting the number of dead nematodes and the number of individuals with altered movements 1 hour and 16 hours after soaking with anti-effector siRNAs. These measures were compared to those observed for control nematodes soaked with a siRNA having no similarity in the genome of *M. incognita.*

In terms of viability, 1 hour after soaking, it was started to observe a few dead nematodes (up to 10%) in 10 out of 16 tested genes, while no dead nematodes were observed in controls (Figure 3). 16 hours after soaking, the number of dead nematodes was substantially higher than in controls for a total of 9 targeted genes (Minc03313, Minc05001, Minc08014, Minc08335, Minc10706, Minc12224, Minc14652, Minc17987, Minc17713).

It was observed no substantial change in terms of nematode mobility, 1 hour after soaking with siRNA compared to control nematodes. However, 16 hours after siRNA soaking, an effect on mobility was observed for some targeted genes. The number of nematodes with movement on the whole body length was reduced by 40% or more for 5 out of the 15 targeted genes (Minc02483, Minc03313, Minc08014, Minc14652, Minc17987). Similarly, it was observed reductions of 40% or more in the number of individuals presenting fast undulations for 4 out of the 15 siRNA-targeted genes (Minc02483, Minc08014, Minc14652 and Minc17987) (Figure 3).

Overall, it was noticed no systematic common effect of siRNA treatment on nematode viability and mobility.

### Effect of siRNA soaking on nematode infestation

For each of the 16 identified targets, it was tested whether nematodes soaked with matching siRNAs showed a significant reduction in the numbers of galls and/or egg masses on infected plants as compared to control nematodes. Reduction in the number of galls implies that fewer nematodes have managed to induce a feeding structure. A reduction in the number of egg masses signifies that fewer female nematodes have managed to complete their development until the production of egg masses, a necessary step to propagate the infection at the next generation.

Overall, 12 out of the 16 siRNA-treated samples (Minc00801, Minc01632, Minc02483, Minc03313, Minc03866, Minc05001, Minc08013, Minc08335, Minc09526, Minc12224, Minc17713 and Minc07817) showed a significant and reproducible reduction in the number of galls or egg masses after nematode infestation compared to control nematodes (Figure 4).

Among all silencing experiments and replicates, significant reductions in terms of gall numbers ranged from 21.08 % (Minc08014, replicate 1) to 60.84 % (Minc08335, replicate 1), (see Figure 5). Significant reductions in terms of number of egg masses ranged from 24.89% (Minc08014, replicate 2) to 71.12% (Minc05001, replicate 2). Considering observations that were reproducible and significant in both replicates, genes that presented the highest reproducible reduction in number of galls were Minc01632 (57.48% and 43.67% reduction in replicates 1 and 2, respectively), Minc03866 (38.36% and 41.20% reduction in replicates 1 and 2, respectively), Minc05001 (33.03% and 51.54% reductions in replicates 1 and 2, respectively) and Minc08013 (33.03% and 51.54% reductions in replicates 1 and 2, respectively). In terms of egg mass numbers, genes presenting the most important reproducible effects were Minc01632 (70.09% and 54.17% reduction in replicates 1 and 2, respectively), Minc02483 (39.54% and 60.85% in replicates 1 and 2, respectively), and Minc09526 (37.08% and 39.74% in replicates 1 and 2, respectively).

Interestingly, siRNAs targeted against gene Minc01632 are those responsible for both the most important reproducible diminution of the number of galls and of the number of egg masses.

### In situ hybridizations of the 16 novel candidate targets

Among the 16 siRNA experiments (15 on effector-like genes and one on the transcription factor), 12 yielded significant and reproducible reduction of the number of galls or egg masses. Because all these genes are novel candidates and nothing is known about their possible functions, it was investigated whether information could be gained from their expression localization using *in-situ* hybridization (methods). Results of *in-situ* hybridizations could be grouped in 5 different categories. (i) Genes with ubiquitous expression detected (Minc01632, Minc08335, Minc12224). (ii) Genes with expression detected specifically in secretory subventral gland cells (Minc03866). (iii) Genes with expression detected specifically in the intestinal tract (Minc00801, Minc17713). (iv) Genes with expression detected specifically in the nerve ring, a structure surrounding the metacorpus (Minc02483). (v) Genes that returned no detectable signal (Minc03313, Minc05001, Minc08013, Minc09526, Minc07817). No further indication could be deduced from genes expressed ubiquitously or from those that returned no detectable signal. However, the gene expressed in the subventral gland cell (Minc03866) could well encode an effector protein eventually secreted in plant tissue during infestation. Minc02483, expressed in the nerve ring is also interesting in itself. Indeed, the nerve ring is a structure surrounding the metacorpus that acts as a pump to inject secretion or take-up nutrients from the plant. These two genes with interesting expression localizations are part of a group of 5 genes (Minc01632, Minc02483, Minc03866, Minc05001 and Minc09526) that yielded significant and reproducible reductions of both the number of egg masses and galls in siRNA assays and thus are among the most promising novel targets. Besides Minc03866 (subventral gland cell) and Minc02483 (nerve ring), Minc01632 showed an ubiquitous expression and the two remaining genes (Minc05001 and Minc09526) returned no detectable signal.

### REFERENCES

Arguel M.J. et al., 2012, Genes, 3:391-408
Atkinson H.J. et al., 2012, Curr Opin Biotechnol., 23:251-256
Bakhetia M. et al., 2008, Int J Parasitol., 38:1589-1597
Barthels N. et al., 1997, Plant Cell, 9:2119-2134
Charlton W.C. et al., 2010, Int J Parasitol., 40:855-864
Dalzell J.J. et al., 2010a, Int J Parasitol., 40:91-100
Dalzell J.J., et al., 2010b, Int J Parasitol., 40: 1303-1310.
Djian-Caporalino C., 2012, EPPO Bulletin, 42: 127-137
Dubreuil G. et al., 2007, New Phytol., 176:426-436
Favery B. et al., 1998, Embo J. 17:6799-6811
Huang G. et al., 2006, Proc Natl Acad Sci U S A.,103:14302-14306
Ibrahim H.M.M. et al., 2011, Exp Parasitol., 127:90-99
Jaubert S. et al., 2002, FEBS Letters, 522:109-112
Klink V.P. et al., 2009, Planta, 230:53-71
Kozub N.O. et al., 2012, Cytology and Genetics, 46:251-262
McCarter J.P., 2009, Molecular Approaches Toward Resistance to Plant-Parasitic Nematodes. In: Berg RH, Taylor C, editors. Cell Biology of Plant Nematode Parasitism: Springer Berlin Heidelberg. pp. 239-267
Needleman S.B. and Wunsch C.D., 1970, J. Mol. Biol., 48:443-453
Nielsen H. et al., 1997, Protein Engineering, 10:1-6
Patel N. et al., 2008, J Nematol., 40:299-310
Patel N. et al., 2010, J Exp Bot., 61:235-248
Petersen T.N. et al., 2011, Nature Methods, 8:785-786
Rosso M.N. et al., 1999, Mol Plant Microbe Interact., 12:585-591
Rosso M.N. et al., 2009, Annu Rev Phytopathol., 47:207-232
Rozen S. et al., 2000, Methods Mol Biol., 132: 365-386
Sasser J.N. and Freckman D.W., 1987, A world perspective on nematology; the role of society. In: Veech JA, Dickerson DW, editors. Vistas on nematology. Hyattsville, USA: Society of Nematologists. pp. 7-14
Sayle, R. and Milner-White, E., 1995, Trends Biochem. Sci., 20:374
Schwab R. et al., 2006, Plant Cell, 18:1121-1133
Sindhu A.S. et al., 2009, J Exp Bot., 60:315-324
Vandesompele J. et al., 2002, Genome Biol. 3:RESEARCH0034
Vens C. et al., 2011, Bioinformatics, 27:1231-1238
Vercauteren I. et al., 2001, Mol Plant Microbe Interact., 14:288-299
Watson J.M. et al., 2005, FEBS Letters, 579:5982-5987
Wesemael W.M.L. et al., 2011, Nematology, 13: 3-16
Yadav B.C. et al., 2006, Mol Biochem Parasitol., 148:219-222

## Claims

1. A method for reducing the plant-parasitic nematode infestation level in a plant, comprising expressing in said plant at least one RNA capable of post-transcriptional inhibition of at least one target gene from said nematode,
wherein said gene is selected from the group consisting of a gene encoding an effector-like protein and a gene encoding a transcription factor,
wherein said effector-like protein:
- comprises a signal peptide including 1, 2, 3 or 4 different MERCI effector-specific motifs selected from the group consisting of:
MOTIF 1: large, hydrophobic, neutral, buried, neutral, neutral, buried, buried, neutral, acyclic, acyclic, hydrophobic, neutral, acyclic, acyclic,
MOTIF 2: neutral, buried, neutral, large, buried, neutral, neutral, neutral, hydrophobic, hydrophobic, neutral, acyclic, acyclic, acyclic, buried,
MOTIF 3: hydrophobic, neutral, buried, acyclic, neutral, neutral, neutral, buried, neutral, large, neutral, acyclic, neutral, polar, acyclic, and
MOTIF 4: neutral, neutral, L, buried, hydrophobic, buried, neutral, hydrophobic, neutral, neutral, acyclic, neutral,
- comprises no transmembrane region, and
- consists of a polypeptide sequence having at least 35% identity, or by order of increasing preference at least 40%, 45%, 50%, 55%, 60%, 65%, 70%, 75%, 80%, 85%, 90%, 95%, 96%, 97%, 98% or 99% identity, and preferably having 100% identity, with a polypeptide selected from the group consisting of SEQ ID NO. 1 (Minc01632), SEQ ID NO. 2 (Minc02483), SEQ ID NO. 3 (Minc05001), SEQ ID NO. 4 (Minc03866), SEQ ID NO. 5 (Minc09526), SEQ ID NO. 6 (Minc17713), SEQ ID NO. 7 (Minc03313), SEQ ID NO. 8 (Minc08335), SEQ ID NO. 9 (Minc08013), SEQ ID NO. 10 (Minc00801), SEQ ID NO. 11 (Minc12224), SEQ ID NO. 12 (Minc14652), SEQ ID NO. 13 (Minc10706), SEQ ID NO. 14 (Minc08014) and SEQ ID NO. 15 (Minc17987), and
wherein said transcription factor comprises a signal peptide, and consists of a polypeptide sequence having at least 35% identity, or by order of increasing preference at least 40%, 45%, 50%, 55%, 60%, 65%, 70%, 75%, 80%, 85%, 90%, 95%, 96%, 97%, 98% or 99% identity, and preferably having 100% identity, with the polypeptide of sequence SEQ ID NO. 16 (Minc07817).

2. The method according to claim 1, **characterized in that** the amino acid sequence of the MERCI effector-specific motif is selected from the group consisting of SEQ ID NO. 54 to 74.

3. The method according to claim 1 or claim 2, **characterized in that** the amino acid sequence of the signal peptide including a MERCI effector-specific motif of the effector-like protein is selected from the group consisting of SEQ ID NO: 38 to 52.

4. The method according to claim 1, **characterized in that** the effector-like protein consists of a polypeptide sequence selected from the group consisting of SEQ ID NO. 1 to 15, SEQ ID NO. 17 to 31 and SEQ ID NO. 33 to 36

5. The method according to claim 1, **characterized in that** the amino acid sequence of the signal peptide of the transcription factor consists of SEQ ID NO. 53.

6. The method according to claim 1, **characterized in that** the transcription factor consists of a polypeptide sequence selected from the group consisting of SEQ ID NO. 16, 32 and 37.

7. The method according to any of claims 1 to 6, **characterized in that** said RNA capable of post-transcriptional inhibition of at least one target gene is a dsRNA.

8. The method according to claim 7, **characterized in that** said dsRNA comprises a first RNA sequence of 18 to 25 nucleotides encoded by a 18 to 25 nucleotide sequence having at least 90% identity, and by order of increasing preference, at least 95%, 96%, 97%, 98%, or 99 % identity, and preferably 100% identity, with a 18 to 25 contiguous nucleotide fragment of said gene, and a second RNA sequence that is the complementary of said first RNA sequence, said first and second sequences being in opposite orientations.

9. The method according to claim 8, **characterized in that**
- the dsRNA targeting the gene encoding SEQ ID NO. 1 (Minc01632) comprises a sense strand encoded by the nucleotide sequence SEQ ID NO. 79 (GGTTCAGAGATTCCTTGTC) and an antisense strand encoded by the nucleotide sequence SEQ ID NO. 80 (GACAAGGAATCTCTGAACC),
- the dsRNA targeting the gene encoding SEQ ID NO. 2 (Minc02483) comprises a sense strand encoded by the nucleotide sequence SEQ ID NO. 81 (TTGACAACCAATTGCCGTT) and an antisense strand encoded by the nucleotide sequence SEQ ID NO. 82 (AACGGCAATTGGTTGTCAA),
- the dsRNA targeting the gene encoding SEQ ID NO. 3 (Minc05001) comprises a sense strand encoded by the nucleotide sequence SEQ ID NO. 83 (ATGACGGCATTATCCGTTC) and an antisense strand encoded by the nucleotide sequence SEQ ID NO. 84 (GAACGGATAATGCCGTCAT),
- the dsRNA targeting the gene encoding SEQ ID NO. 4 (Minc03866) comprises a sense strand encoded by the nucleotide sequence SEQ ID NO. 85 (GCAGGAGTTGTATGCAAGC) and an antisense strand encoded by the nucleotide sequence SEQ ID NO. 86 (GCTTGCATACAACTCCTGC),
- the dsRNA targeting the gene encoding SEQ ID NO. 5 (Minc09526) comprises a sense strand encoded by the nucleotide sequence SEQ ID NO. 87 (GAGATAGAAGTGAGTGTAC) and an antisense strand encoded by the nucleotide sequence SEQ ID NO. 88 (GTACACTCACTTCTATCTC),
- the dsRNA targeting the gene encoding SEQ ID NO. 6 (Minc17713) comprises a sense strand encoded by the nucleotide sequence SEQ ID NO. 89 (TCCACATTATCGCGCAGGA) and an antisense strand encoded by the nucleotide sequence SEQ ID NO. 90 (TCCTGCGCGATAATGTGGA),
- the dsRNA targeting the gene encoding SEQ ID NO. 7 (Minc03313) comprises a sense strand encoded by the nucleotide sequence SEQ ID NO. 91 (CGCTCCATGCATTAGGTTC) and an antisense strand encoded by the nucleotide sequence SEQ ID NO. 92 (GAACCTAATGCATGGAGCG),
- the dsRNA targeting the gene encoding SEQ ID NO. 8 (Minc08335) comprises a sense strand encoded by the nucleotide sequence SEQ ID NO. 93 (CGACAGCGAAGAAGACTAC) and an antisense strand encoded by the nucleotide sequence SEQ ID NO. 94 (GTAGTCTTCTTCGCTGTCG),
- the dsRNA targeting the gene encoding SEQ ID NO. 9 (Minc08013) comprises a sense strand encoded by the nucleotide sequence SEQ ID NO. 95 (AGAACGGATCAATCGAGCA) and an antisense strand encoded by the nucleotide sequence SEQ ID NO. 96 (TGCTCGATTGATCCGTTCT),
- the dsRNA targeting the gene encoding SEQ ID NO. 10 (Minc00801) comprises a sense strand encoded by the nucleotide sequence SEQ ID NO. 97 (ACGTTCACAGAACTCTTAC) and an antisense strand encoded by the nucleotide sequence SEQ ID NO. 98 (GTAAGAGTTCTGTGAACGT),
- the dsRNA targeting the gene encoding SEQ ID NO. 11 (Minc12224) comprises a sense strand encoded by the nucleotide sequence SEQ ID NO. 99 (GACGAAGCTGGTCAATGCT) and an antisense strand encoded by the nucleotide sequence SEQ ID NO. 100 (AGCATTGACCAGCTTCGTC),
- the dsRNA targeting the gene encoding SEQ ID NO. 12 (Minc14652) comprises a sense strand encoded by the nucleotide sequence SEQ ID NO. 101 (CAAGCAATGCTGTTCATGC) and an antisense strand encoded by the nucleotide sequence SEQ ID NO. 102 (GCATGAACAGCATTGCTTG),
- the dsRNA targeting the gene encoding SEQ ID NO. 13 (Minc10706) comprises a sense strand encoded by the nucleotide sequence SEQ ID NO. 103 (TGGCATTCAAGTTGGTCCT) and an antisense strand encoded by the nucleotide sequence SEQ ID NO. 104 (AGGACCAACTTGAATGCCA),
- the dsRNA targeting the gene encoding SEQ ID NO. 14 (Minc08014) comprises a sense strand encoded by the nucleotide sequence SEQ ID NO. 105 (ATCCGGCAAGTCTGCTTGT) and an antisense strand encoded by the nucleotide sequence SEQ ID NO. 106 (ACAAGCAGACTTGCCGGAT),
- the dsRNA targeting the gene encoding SEQ ID NO. 15 (Minc17987) comprises a sense strand encoded by the nucleotide sequence SEQ ID NO. 107 (AGGACAACAAGGAGGCATT) and an antisense strand encoded by the nucleotide sequence SEQ ID NO. 108 (AATGCCTCCTTGTTGTCCT).
- the dsRNA targeting the gene encoding SEQ ID NO. 16 (Minc07817) comprises a sense strand encoded by the nucleotide sequence SEQ ID NO. 109 (GGATGAACCGTTGGAACAA) and an antisense strand encoded by the nucleotide sequence SEQ ID NO. 110 (TTGTTCCAACGGTTCATCC).

10. The method according to any of claims 1 to 9, **characterized in that** said dsRNA produces through cleavage by RNases into the plant cell a small interfering RNA (siRNA), a hairpin RNA (hpRNA) or an artificial microRNA (amiRNA).

11. The method according to any of claims 1 to 10, **characterized in that** said plant expresses at least two RNAs capable of post-transcriptional inhibition of at least two different target genes as defined in any of claims 1 to 6.

12. The method according to any of claims 1 to 11, **characterized in that** the nematode is a root-knot nematode of the genus *Meloidogyne.*

13. The method according to claim 12, **characterized in that** the nematode is selected from the group consisting of *M. javanica, M. arenaria, M. incognita* and *M. hapla.*

14. The method according to any of claim 1 to 13, **characterized in that** the plant is a tomato plant.

15. A chimeric DNA construct encoding a dsRNA for silencing a target gene as defined in any of claims 1 to 6, comprising:
- a promoter functional in a plant cell;
- a first DNA sequence of 18 to 25 bp, preferably of 19 to 24 bp, more preferably of 19, 20 or 21 bp, consisting of a fragment of a cDNA encoded by said target gene, or having at least 90% identity, and by order of increasing preference, at least 95%, 96%, 97%, 98%, or 99% identity, and preferably 100% identity, with said fragment, said DNA sequence being placed under transcriptional control of said promoter;
- a second DNA sequence that is the complementary of said first DNA, said first and second sequences being in opposite orientations;
- a spacer sequence separating said first and second sequence, such that these first and second DNA sequences are capable, when transcribed, of forming a single double-stranded RNA molecule.

16. A plant cell or a plant genetically modified by a chimeric DNA construct as defined in claim 15.

17. An isolated RNA for silencing a gene from a nematode encoding an effector-like protein or a transcription factor, comprising or consisting of a 15 to 30 nucleotide RNA having at least 90% identity with a 15 to 30 contiguous nucleotide fragment of the gene encoding a protein selected from the group consisting of SEQ ID NO. 1 to SEQ ID NO. 16.

18. The isolated RNA according to claim 17, **characterized in that** it is selected from the group consisting of:
- a dsRNA targeting the gene encoding SEQ ID NO. 1 (Minc01632) comprising a sense strand encoded by the nucleotide sequence SEQ ID NO. 79 (GGTTCAGAGATTCCTTGTC) and an antisense strand encoded by the nucleotide sequence SEQ ID NO. 80 (GACAAGGAATCTCTGAACC),
- a dsRNA targeting the gene encoding SEQ ID NO. 2 (Minc02483) comprising a sense strand encoded by the nucleotide sequence SEQ ID NO. 81 (TTGACAACCAATTGCCGTT) and an antisense strand encoded by the nucleotide sequence SEQ ID NO. 82 (AACGGCAATTGGTTGTCAA),
- a dsRNA targeting the gene encoding SEQ ID NO. 3 (Minc05001) comprising a sense strand encoded by the nucleotide sequence SEQ ID NO. 83 (ATGACGGCATTATCCGTTC) and an antisense strand encoded by the nucleotide sequence SEQ ID NO. 84 (GAACGGATAATGCCGTCAT),
- a dsRNA targeting the gene encoding SEQ ID NO. 4 (Minc03866) comprising a sense strand encoded by the nucleotide sequence SEQ ID NO. 85 (GCAGGAGTTGTATGCAAGC) and an antisense strand encoded by the nucleotide sequence SEQ ID NO. 86 (GCTTGCATACAACTCCTGC),
- a dsRNA targeting the gene encoding SEQ ID NO. 5 (Minc09526) comprising a sense strand encoded by the nucleotide sequence SEQ ID NO. 87 (GAGATAGAAGTGAGTGTAC) and an antisense strand encoded by the nucleotide sequence SEQ ID NO. 88 (GTACACTCACTTCTATCTC),
- a dsRNA targeting the gene encoding SEQ ID NO. 6 (Minc17713) comprising a sense strand encoded by the nucleotide sequence SEQ ID NO. 89 (TCCACATTATCGCGCAGGA) and an antisense strand encoded by the nucleotide sequence SEQ ID NO. 90 (TCCTGCGCGATAATGTGGA),
- a dsRNA targeting the gene encoding SEQ ID NO. 7 (Minc03313) comprising a sense strand encoded by the nucleotide sequence SEQ ID NO. 91 (CGCTCCATGCATTAGGTTC) and an antisense strand encoded by the nucleotide sequence SEQ ID NO. 92 (GAACCTAATGCATGGAGCG),
- a dsRNA targeting the gene encoding SEQ ID NO. 8 (Minc08335) comprising a sense strand encoded by the nucleotide sequence SEQ ID NO. 93 (CGACAGCGAAGAAGACTAC) and an antisense strand encoded by the nucleotide sequence SEQ ID NO. 94 (GTAGTCTTCTTCGCTGTCG),
- a dsRNA targeting the gene encoding SEQ ID NO. 9 (Minc08013) comprising a sense strand encoded by the nucleotide sequence SEQ ID NO. 95 (AGAACGGATCAATCGAGCA) and an antisense strand encoded by the nucleotide sequence SEQ ID NO. 96 (TGCTCGATTGATCCGTTCT),
- a dsRNA targeting the gene encoding SEQ ID NO. 10 (Minc00801) comprising a sense strand encoded by the nucleotide sequence SEQ ID NO. 97 (ACGTTCACAGAACTCTTAC) and an antisense strand encoded by the nucleotide sequence SEQ ID NO. 98 (GTAAGAGTTCTGTGAACGT),
- a dsRNA targeting the gene encoding SEQ ID NO. 11 (Minc12224) comprising a sense strand encoded by the nucleotide sequence SEQ ID NO. 99 (GACGAAGCTGGTCAATGCT) and an antisense strand encoded by the nucleotide sequence SEQ ID NO. 100 (AGCATTGACCAGCTTCGTC),
- a dsRNA targeting the gene encoding SEQ ID NO. 12 (Minc14652) comprising a sense strand encoded by the nucleotide sequence SEQ ID NO. 101 (CAAGCAATGCTGTTCATGC) and an antisense strand encoded by the nucleotide sequence SEQ ID NO. 102 (GCATGAACAGCATTGCTTG),
- a dsRNA targeting the gene encoding SEQ ID NO. 13 (Minc10706) comprising a sense strand encoded by the nucleotide sequence SEQ ID NO. 103 (TGGCATTCAAGTTGGTCCT) and an antisense strand encoded by the nucleotide sequence SEQ ID NO. 104 (AGGACCAACTTGAATGCCA),
- a dsRNA targeting the gene encoding SEQ ID NO. 14 (Minc08014) comprising a sense strand encoded by the nucleotide sequence SEQ ID NO. 105 (ATCCGGCAAGTCTGCTTGT) and an antisense strand encoded by the nucleotide sequence SEQ ID NO. 106 (ACAAGCAGACTTGCCGGAT),
- a dsRNA targeting the gene encoding SEQ ID NO. 15 (Minc17987) comprising a sense strand encoded by the nucleotide sequence SEQ ID NO. 107 (AGGACAACAAGGAGGCATT) and an antisense strand encoded by the nucleotide sequence SEQ ID NO. 108 (AATGCCTCCTTGTTGTCCT),
- a dsRNA targeting the gene encoding SEQ ID NO. 16 (Minc07817) comprising a sense strand encoded by the nucleotide sequence SEQ ID NO. 109 (GGATGAACCGTTGGAACAA) and an antisense strand encoded by the nucleotide sequence SEQ ID NO. 110 (TTGTTCCAACGGTTCATCC).

19. A method for reducing the plant-parasitic nematode infestation level in a plant comprising contacting said plant-parasitic nematode with an effective amount of an isolated RNA as defined in claims 17 and 18.

20. A method for screening a candidate compound that can be used for reducing the plant-parasitic nematode infestation level in a plant comprising the steps of:
- contacting a candidate compound with a plant-parasitic nematode expressing an effector-like protein or a transcription factor as defined in any of claims 1 to 6;
- determining whether the candidate compound modulates the expression of the gene encoding said effector-like protein or said transcription factor;
wherein said candidate compound that decreases the expression of the gene encoding said effector-like protein or said transcription factor is identified as a candidate compound capable of reducing the plant-parasitic nematode infestation level in a plant.
